(19) Europäisches Patentamt
European Patent Office
Office européen des brevets

(11) EP 2 202 230 A1

(12) **EUROPEAN PATENT APPLICATION**

(43) Date of publication:
**30.06.2010 Bulletin 2010/26**

(21) Application number: **08382085.2**

(22) Date of filing: **23.12.2008**

(51) Int Cl.:
*C07D 333/54* (2006.01) *C07D 333/56* (2006.01)
*C07C 62/26* (2006.01) *C07C 62/38* (2006.01)
*A61K 31/381* (2006.01) *A61K 31/19* (2006.01)
*A61P 31/00* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **UNIVERSIDADE DE SANTIAGO DE COMPOSTELA**
**15782 Santiago de Compostela (ES)**

(72) Inventors:
- **Gonzalez Bello, Concepción**
**E-15782, Santiago de Compostela (ES)**
- **Viera Prazeres, Verónica Filipa**
**E-15782, Santiago de Compostela (ES)**
- **Paz Gomez, Sonia**
**E-15782, Santiago de Compostela (ES)**
- **Sanchez Sixto, Cristina**
**E-15782, Santiago de Compostela (ES)**
- **Tizon Valverde, Lorena**
**E-15782, Santiago de Compostela (ES)**

(74) Representative: **ABG Patentes, S.L.**
**Avenida de Burgos 16D**
**Edificio Euromor**
**28036 Madrid (ES)**

(54) **Competitive inhibitors of type ii dehydroquinase enzyme**

(57) The present invention is directed to a compound of formula I, its diastcrcoisomcrs, its enantiomers or its pharmaceutically acceptable salts or solvates,

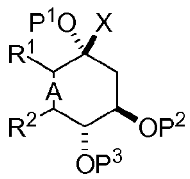

**I**

to procedures of obtaining the same, to intermediates thereof, and use as competitive inhibitors of the third enzyme of the shikimic acid pathway, the type 11 dehydroquinase.

EP 2 202 230 A1

## Description

### FIELD OF THE INVENTION

The present invention relates to compounds of general formula I, to procedures of obtaining the same, to intermediates thereof, and use as competitive inhibitors of the third enzyme of the shikimic acid pathway, the type II dehydroquinase.

### STATE OF THE ART

Although nowadays enormous effective chemotherapeutic agents have been developed, the number of deaths among hospitalized patients infected with resistant bacterial strains has increased dramatically. This fact is especially remarkable for important diseases such as tuberculosis, where the current therapies become less efficient. Their effects are particularly strong in people with a compromised immune system such as HIV patients. The synergy between the AIDS epidemic and increasing surge of multidrug-resistant isolates to antibiotics leads to the alarming conclusion that antibiotics are loosing their effectiveness. It is therefore necessary to discover new, safe, selective and more efficient antibiotics to face this problem.

For example, selectivity can sometimes be achieved by using compounds that inhibit one of the biosynthetic pathways present in bacteria. Thus, there are antibiotics that interfere in the protein, lipids or catetenoids biosynthesis, etc. In bacteria, there is a metabolic route, known as the shikimic acid pathway (Haslam, E. The shikimate pathway. New York: Wiley; 1974), through which chorismic acid is biosynthesized. The later compound is the precursor in the synthesis of aromatic compounds such as the aromatic amino acids, folates, ubiquinones and certain vitamins (Abell, C. Enzymology and molecular biology of the shikimate pathway. In: Sankawa U, editor. Comprehensive Natural Products Chemistry. Oxford: Pergamon, Elsevier Science Ltd.; 1998. p 573). The shikimic acid pathway is present in bacteria, fungi, higher plants and has recently been discovered in apicomplexan parasites, such as *Cryptosporidium parvum* (Roberts, F.; et all Nature 1998, 393, 801; Roberts, C. W. et all J. Infect. Dis. 2002, 185 (suppl 1), S25; McConkey, G. A.; Pinney, J. W.; Westhead, D. R.; Plueckhahn, K.; Fitzpatrick, T. B.; Macheroux, P.; Kappes, B. Trends in Parasitology 2004, 20, 60).

The enzyme dehydroquinase (3-dehydroquinate dehydratase, EC 4.2.1.10) catalyzes the reversible dehydration of 3-dehydroquinic acid to form 3-dehydroshikimic acid (Scheme 1). There are two different dehydroquinases, known as type I and type II, which possess different biochemical and biophysical properties and do not show sequence similarity (Hawkins, A. R. Curr. Genet. 1987, 11, 491). These two enzymes catalyse the same reaction, but they utilize completely different mechanisms and opposite stereochemistry (Kleanthous, C.; Davis, K.; Kelly, S. M.; Cooper, A.; Harding, S. E.; Price, N. C.; Hawkins, A. R.; Coggins, J. R. Biochem. J. 1992, 282, 687).

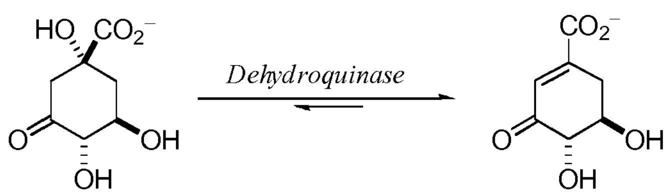


3-dehydroquinic acid    3-dehydroshikimic acid

**Scheme 1**

The type II enzyme (Gourley, D. G.; Coggins, J. R.; Isaacs, N. W.; Moore, J. D.; Charles, I. G.; Hawkins, A. R. J. Mol. Biol. 1994, 241, 488; Krell, T.; Pitt, A. R.; Coggins, J. R. FEBS Lett. 1995, 360, 93), may come from different sources (*Mycobacterium tuberculosis*, *Streptomyces coelicolor*, *Helicobacter pylori, Aspergillus nidulans*), and catalyzes the anti elimination of water.

A number of compounds with antibiotic properties have been tested in recent years, some of which are believed to inhibit the dehydroquinase of the shikimic acid pathway. For example, González-Bello, C. et al Org. Biomol. Chem., 1, 2003, p. 2075-2083 or González-Bello, C. et al Medicinal Research Reviews, Vol. 27(2), 2007, p. 177-208 discloses derivative of formula

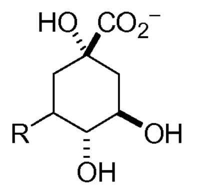

having a $K_i$ of 180 to more than 20,000 micro molar with *S. coelicolor* Type II Dehydroquinase.

González-Bello, C. et al Org. Biomol. Chem., 1, 2003, p. 2075-2083 discloses 3-substituted derivatives of 1,4,5-Trihydroxycyclohexanecarboxylic acid, having the formula

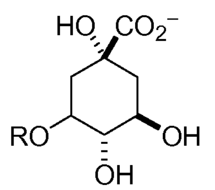

which have a $K_i$ of 180 to more than 20,000 micro molar against *S. coelicolor* Type II Dehydroquinase.

González-Bello, C. et al ChemMedChem, 2008, 3, 756-770 discloses derivative of formula

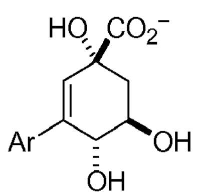

having a $K_i$ of 0.54 to more than 400 micro molar with *H. pylori* Type II Dehydroquinase. Compounds having the above general formula and further compounds where tested in González-Bello, C. et al ChemMedChem, 2007, 2, p. 194-207 against *S. coelicolor* Type II Dehydroquinase, showing a $K_i$ between 0.13 and 33.5 micro molar.

Patent application WO 2005/009330 discloses derivatives of formula

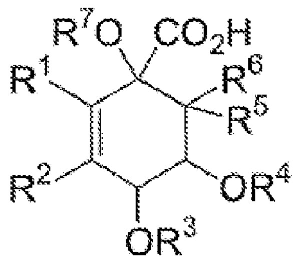

Mainly those wherein $R^3$ and/or $R^7$ are substituted benzyl groups.

Thus, there is a need to provide further compounds with antibiotic and/or antimicrobial activity.

## SUMMARY OF THE INVENTION

The present invention relates to compounds with antibiotic and/or antimicrobial activity activity, whose action is based on the effective and selective inhibition of the essential amino acids biosynthesis, particularly, by inhibition of the dehydroquinase, the third enzyme of the shikimic acid pathway.

The present invention provides compounds based on the quinic acid structure, which are effective competitive

inhibitors of type II dehydroquinase, the third enzyme of the shikimic acid pathway. These compounds are significantly more potent than the described compounds of similar structure (in some cases with a Ki more than a thousand times higher). The present invention also provides procedures of obtaining of these compounds, as well as their use as antibiotics and/or antimicrobials.

Accordingly, a first aspect of the present invention is directed to a compound of formula **I,** its diastereoisomers, its enantiomers or its pharmaceutically acceptable salts or solvates

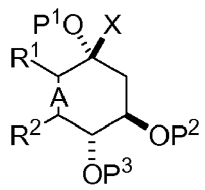

**I**

wherein,

A represents a single or double bond;
X is selected from the group consisting of -(C=O)OR$^a$ and -(C=O)NR$^b$R$^c$, wherein each of R$^a$, R$^b$ and R$^c$ is independently selected from the group consisting of hydrogen, substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl; or R$^b$ and R$^c$ together form a 5 or 6 membered heterocyclyc ring together with the nitrogen atom to which they are attached;
each of P$^1$, P$^2$ and P$^3$ is independently selected from the group consisting of hydrogen, substituted or unsubstituted alkyl, substituted or unsubstituted alkenyl, substituted or unsubstituted alkynyl, substituted or unsubstituted silyl, substituted or unsubstituted arylalkyl and -(C=O)R$^a$, wherein R$^a$ is as defined above; and wherein
if A is a double bond,
then R$^2$ is selected from the group consisting of -OR$^a$, -SR$^a$ and - NR$^b$R$^c$, wherein R$^a$, R$^b$ and R$^c$ are as defined above; and R$^1$ is hydrogen or R$^{1a}$, wherein R$^{1a}$ is selected from the group consisting of substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl, or R$^1$ and R$^2$ together form a 5-membered ring; and
if A is a single bond,
then R$^2$ is =O, =S or =NR$^b$, wherein R$^b$ is as defined above; and R$^1$ is R$^{1a}$, wherein R$^{1a}$ is a defined above.

Further aspects of the present invention are methods for the synthesis of said compounds of formula **I**, and intermediates thereof.

A further aspect of the present invention is a pharmaceutical composition comprising said compound of formula **I** and a pharmaceutically acceptable carrier.

A further aspect of the present invention is a compound of formula **I** as defined above, for use as a medicament.

A further aspect of the present invention is a compound of formula **I** as defined above, for use as an antibiotic and/or antimicrobial.

## DETAILED DESCRIPTION OF THE INVENTION

### Definitions

**"Alkyl"** refers to a straight or branched, cyclic or acyclic hydrocarbon radical consisting of carbon and hydrogen atoms, containing no unsaturation, having 1-12, preferably one to eight, more preferably one to four carbon atoms, and which is attached to the rest of the molecule by a single bond, optionally substituted by one or more substituents selected from the group consisting of an halogen atom, an alkoxy group, a cyano group, a nitro group, a thioalkoxy group, an heterocyclylalkyl group, an heterocyclyl group or $CF_3$, for example, methyl, ethyl, n-propyl, i-propyl, n-butyl, t-butyl, n-pentyl, cyclopropyl, etc.

**"Alkenyl"** refers to a straight or branched, cyclic or acyclic hydrocarbon radical consisting of carbon and hydrogen atoms, containing at least one unsaturation, conjugated or not, having 2 to 12, preferably two to eight, more

preferably two to four carbon atoms, and which is attached to the rest of the molecule by a single bond. Alkenyl radicals may be optionally substituted by one or more substituents such as a halogen atom, an alkoxy group, a cyano group, a nitro group, a thioalkoxy group, an heterocyclylalkyl group, an heterocyclyl group or $CF_3$, such as vinyl, allyl, butenyl (e.g. 1-butenyl, 2-butenyl, 3-butenyl), or pentenyl (e.g. 1-pentenyl, 2-pentenyl, 3-pentenyl, 4-pentenyl).

**"alkynyl"** refers to a straight or branched, cyclic or acyclic hydrocarbon radical consisting of carbon and hydrogen atoms, containing at least one carbon-carbon triple bond, conjugated of not, having two to twelve, preferably two to eight, more preferably two to four carbon atoms, and which is attached to the rest of the molecule by a single bond, such as -CCH, $-CH_2CCH$, $-CCCH_3$, $-CH_2CCCH_3$. Alkynyl radicals may be optionally substituted by one or more substituents such as a halogen atom, an alkoxy group, a cyano group, a nitro group, a thioalkoxy group, an heterocyclylalkyl group, an heterocyclyl group or $CF_3$.

**"Aryl"** refers to an aromatic hydrocarbon with 6 to 10 carbon atoms, such as phenyl or naphtyl, optionally substituted by one or more substituents selected from the group consisting of a halogen atom, an alkoxy group, a cyano group, a nitro group, an thioalkoxy group, an alkyl group or $CF_3$.

**"Silyl"** refers to trialkylsilyl species which are commonly used in organic chemistry as protecting groups, such as those disclosed in Greene, T. W.; Wuts, P. G. M. "Protective Groups in Organic Synthesis", 3˚ Ed., Wiley-Interscience, New York, 1999. According to a particular embodiment, a radical of formula $-SiR^dR^eR^f$ wherein $R^d$, $R^e$ and $R^f$ are independently selected from a substituted or unsubstituted alkyl group or a substituted or unsubstituted aryl group, for example, methyl, ethyl, t-butyl, i-propyl, phenyl, etc.

**arylalkyl** refers to a one or various aryl groups bonded to the rest of the molecule by an alkyl radical, for example, benzyl, 3-(phenyl)-propyl, etc.

**"Heterocyclyl"** refers to a stable 3 to 15 membered-ring constituted by carbon atoms and 1 to 5 heteroatoms selected from nitrogen, oxygen and sulphur, preferably a 4 to 8 membered-ring constituted by one or more heteroatoms, and more preferably a 5 to 6 membered-ring with one or more heretoatoms. For the purposes of this invention, heterocyclyl groups can be a monocyclic, bicyclic or tricyclic systems, that can include fused rings; and the nitrogen or sulphur atom in the heterocyclic ring can be optionally oxidized; the nitrogen atom can be optionally quatemarized; and the heterocyclyl radical can be partially or totally saturated or can be aromatic. The heterocyclic ring can be substituted by one or more substituents selected from the group consisting of a halogen atom, an alkoxy group, an alkyl group, a thioalkoxy group, a cyano group, a nitro group or $CF_3$. Examples of such heterocycles include, for example, furan, thiophene, pyrrole, imidazole, triazole, isothiazole, benzothiophene, benzofurane, indol, benzoimidazole, tetrahydrofuran.

**"Heteroaryl"** refers to a heterocyclyl group wherein at least one of the rings is aromatic.

**"Alkoxy"** refers to a radical of formula -O-alkyl, for example, methoxy, ethoxy, propoxy, etc.

**"Thioalkoxy"** refers to a radical of formula -S-alkyl, for example, thiomethoxy, thioethoxy, thiopropoxy, etc.

**"Amino"** refers to a radical of formula $-NR^bR^c$ wherein $k^b$ and $R^c$ are as previously defined.

**"Alkoxycarbonyl"** refers to a radical of formula -C(=O)-O-alkyl.

**"Aminocarbonyl"** refers to a radical of formula $-C(=O)-NR^bR^c$, wherein $k^b$ and $R^c$ are as defined above.

**"Alkylcarbonyl"** refers to a radical of formula -C(=O)-alkyl.

**"Heterocyclylalkyl"** refers to a one or various heterocyclyl groups bonded to the rest of the molecule by an alkyl radical, for example, 2-(thienyl)ethyl, benzothiophenylmethyl, etc.

**"Quinic acid"** refers to (1*S*,3*R*,4*S*,5*R*)-1,3,4,5-tetrahydroxycyclohexanecarboxylic acid. Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a $^{13}C$- or $^{14}C$-enriched carbon or $^{15}N$-enriched nitrogen are within the scope of this invention.

Further, the term **"pharmaceutically acceptable"** refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. According to a particular embodiment ethyl ether, ethyl acetate, ethanol, isopropanol or acetonitrile are used as solvents. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic amino acids salts.

The compounds of the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of the present invention may include diastereoisomers and/or enantiomers depending on the presence of chiral centres or isomers depending on the presence of multiple bonds (e.g. Z, E). The single isomers, diastereoisomers, enantiomers and mixtures thereof fall within the scope of the present invention.

## Compounds of formula I

According to a particular embodiment, the compound of formula **I** is a compound of formula **Ia**, its diastereoisomers, its enantiomers or its pharmaceutically acceptable salts or solvates

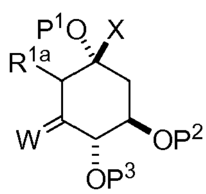

**Ia**

wherein X, $P^1$, $P^2$, $P^3$ and $R^{1a}$ are as defined above, and W is =O, =S or $=NR^b$.

According to a particular embodiment, the compound of formula **I** is a compound of formula **Ib**, its diastereoisomers, its enantiomers or its pharmaceutically acceptable salts or solvates

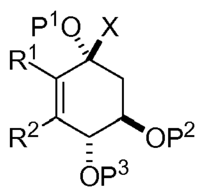

**Ib**

wherein,

X, $P^1$, $P^2$, $P^3$ and $R^1$ are as defined above; and
$R^2$ is selected from the group consisting of $-OR^a$, $-SR^a$ and $-NR^bR^c$, wherein $R^a$, $R^b$ and $R^c$ are as defined above.

According to a particular embodiment, the compound of formula **I** is a compound of formula **Ic**, its diastereoisomers, its enantiomers or its pharmaceutically acceptable salts or solvates

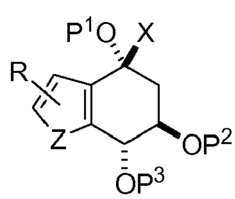

**Ic**

wherein,

X, $P^1$, $P^2$ and $P^3$ are as defined above;
Z is selected from the group consisting of O, S, $NR^b$ and $\oplus NR^bR^c$, wherein $k^b$ and $R^c$ are as defined above; and
R is selected from the group consisting of a hydrogen, halogen, substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl.

According to particular embodiment X in a compound of formula **I, Ia**, **Ib** or **Ic** is - $CO_2H$ or -$CO_2M$, wherein M is a metal cation, preferably a metal cation of Group I of the Periodic Table, more preferably, of sodium.
According to a particular embodiment, $R^1$ in a compound of formula **I, Ia** or **Ib** is an alkyl or an alkenyl group, preferably a $C_{1-4}$ alkyl group or a $C_{1-4}$ alkenyl group.
According to a particular embodiment, $R^1$ in a compound of formula **I, Ia** or **Ib** is an alkyl group, preferably a $C_{1-4}$ alkyl group, substituted with an aryl or heteroaryl group.
According to a particular embodiment, $R^1$ in a compound of formula **I, Ia** or **Ib** is a radical of formula **VIII**

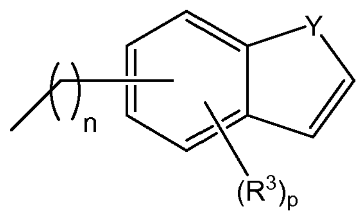

**VIII**

wherein

n is 1, 2, 3 or 4, preferably 1;
p is 1, 2, or 3, preferably 1;
$R^3$ is selected from the group consisting of hydrogen, substituted or unsubstituted alkyl, substituted or unsubstituted alkenyl, substituted or unsubstituted alkynyl, substituted or unsubstituted alkoxy, substituted or unsubstituted thioalkoxy, substituted or unsubstituted aryl, substituted or unsubstituted heterocyclyl, substituted or unsubstituted arylalkyl and substituted or unsubstituted heterocyclylalkyl; and
Y is selected from the group consisting of O, S, $NR^b$ and $\oplus NR^bR^c$, wherein $R^b$ and $R^c$ are as defined above,

wherein the -$(CH_2)_n$- moiety and $R^3$ may be in any of the free positions.
According to a particular embodiment, $R^1$ in a compound of formula **I, Ia** or **Ib** is a radical of formula **IX**

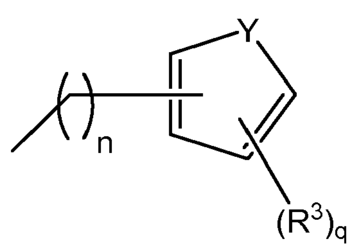

**IX**

wherein

n, Y and $R^3$ are as defined above; and
q is 1, 2, or 3, preferably 1.

According to a particular embodiment, $R^3$ is hydrogen or alkyl, preferably $C_{1-4}$ alkyl.
According to a particular embodiment, Y is S or O, preferably S.
According to a particular embodiment, $R^1$ in a compound of formula **I, Ia** or **Ib** is substituted or unsubstituted

benzyl group, preferably a radical of formula **X**

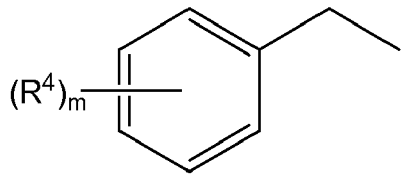

**X**

wherein

m is 0, 1, 2, 3, 4 or 5, preferably 1; and
$R^4$ is selected from the group consisting of halogen, substituted or unsubstituted alkyl, substituted or unsubstituted alkenyl, substituted or unsubstituted alkynyl, substituted or unsubstituted alkoxy, substituted or unsubstituted thioalkoxy, substituted or unsubstituted aryl, substituted or unsubstituted heterocyclyl, substituted or unsubstituted arylalkyl and substituted or unsubstituted heterocyclylalkyl.

According to a particular embodiment, $R^4$ is selected from the group consisting of halogen, alkyl and alkoxy. According to a further particular embodiment m is 5 and $R^4$ is fluor. According to a further particular embodiment, m is 1 or 2, and $R^4$ is a $C_{1-4}$ alkyl group or a $C_{1-4}$ alkoxy group.

According to a particular embodiment, $R^1$ is H in a compound of formula **Ib.**

According to a particular embodiment, $R^1$ is alkenyl in a compound of formula **Ib,** preferably, $C_{1-4}$ alkenyl, more preferably allyl.

According to a particular embodiment, $R^2$ in a compound of formula **Ib** is selected from the group consisting of $-OR^a$, $-SR^a$ and $-NR^bR^c$, wherein each of $R^a$, $R^b$ and $R^c$ is independently selected from the group consisting of substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl; or $R^b$ and $R^c$ together form a 5 or 6 membered heterocyclyc ring together with the nitrogen atom to which they are attached.

According to a particular embodiment, $R^2$ in a compound of formula **Ib** is $-OR^a$ or $- SR^a$, wherein $R^a$ is preferably a heterocyclylalkyl group, preferably a heteroarylalkyl, more preferably a radical of formula **VIII** or **IX** as defined above, preferably a radical of formula **VIII** or **IX** wherein $R^3$ is selected from the group consisting of alkyl, preferably $C_{1-4}$ alkyl and/or wherein Y is S or O, preferably S.

According to a further embodiment, $R^1$ and $R^2$ together form a 5-membered heteroaryl ring.

According to a particular embodiment, in a compound of formula **I, Ia, Ib** or **Ic,** at least one of $P^1$, $P^2$ and $P^3$ is hydrogen, preferably $P^1$, $P^2$ and $P^3$ are all hydrogen.

According to a particular embodiment, R in a compound of formula **Ic** is selected from the group consisting of substituted or unsubstituted alkyl, substituted or unsubstituted alkenyl, substituted or unsubstituted alkynyl and substituted or unsubstituted aryl.

According to a further particular embodiment, R in a compound of formula **Ic** is selected from the group consisting of substituted or unsubstituted alkyl, substituted or unsubstituted, preferably branched, alkenyl and unsubstituted aryl According to a particular embodiment, R is selected from the group consisting of substituted alkyl, substituted alkenyl, substituted alkynyl and substituted aryl, preferably a substituted alkyl or substituted alkenyl, wherein the substituent is a $C_{3-6}$ cycloalkyl group, preferably cyclopropyl. According to a particular embodiment, said group R is in position two of the ring.

According to a particular embodiment, Z is S.

According to a particular embodiment, W is =S or =O, preferably =O.

In another more particular embodiment, the present invention relates to compounds of formula **I** preferably selected from:

[1] (2*R*)-2-allyl-3-dehydroquinic acid,
[2] (2*S*)-2-allyl-3-dehydroquinic acid,
[3] (2*R*)-2-propyl-3-dehydroquinic acid,
[4] (2*R*)-2-benzyl-3-dehydroquinic acid,
[5] (2*S*)-2-benzyl-3-dehydroquinic acid,

[6] (2*R*)-2-(4-methyl)benzyl-3-dehydroquinic acid,
[7] (2*S*)-2-(4-methyl)benzyl-3-dehydroquinic acid,
[8] (2*R*)-2-(4-methoxy)benzyl-3-dehydroquinic acid,
[9] (2*S*)-2-(4-methoxy)benzyl-3-dehydroquinic acid,
[10] (2*R*)-2-perfluorobenzyl-3-dehydroquinic acid,
[11] (2*S*)-2-perfluorobenzyl-3-dehydroquinic acid,
[12] (2*R*)-2-(benzo[*b*]thiophen-5-yl)methyl-3-dehydroquinic acid,
[13] (2*S*)- 2-(benzo[*b*]thiophen-5-yl)methyl-3-dehydroquinic acid,
[14] Sodium (1*R*, 4*S*, 5*R*)-3-(benzo[*b*]thiophen-2-yl)methoxy-1,4,5-trihydroxycyclohex-2-en-1-carboxylate,
[15] Sodium (1*R*, 4*S*, 5*R*)-3-(benzo[*b*]thiophen-2-yl)methoxy-2-(benzo[*b*]thiophen-2-yl)methyl-1,4,5-trihydroxycyclohex-2-en-1-carboxylate,
[16] Sodium (1*R*, 4*S*, 5*R*)-1,4,5-trihydroxy-3-(5-methylbenzo[*b*]thiophen-2-yl)methoxycyclohex-2-en-1-carboxylate,
[17] Sodium (1*R*, 4*S*, 5*R*)-1,4,5-trihydroxy-3-(5-methylbenzo[*b*]thiophen-2-yl)methoxy-2-(5-methylbenzo[*b*]thiophen-2-yl)methylcyclohex-2-en-1-carboxylate,
[18] Sodium (1*R*, 4*S*, 5*R*)-1,4,5-trihydroxy-3-(5-methylbenzo[*b*]thiophen-2-yl)methoxy-2-(5-methylbenzo[*b*]thiophen-2-yl)methylcyclohex-2-en-1-carboxylate,
[19] Sodium (1*R*, 4*S*, 5*R*)-2-allyl-3-(benzo[*b*]thiophen-2-yl)methoxy-1,4,5-trihydroxycylohex-2-en-1-carboxylate,
[20] (4*R*, 6*R*, 7*S*)-4,6,7-trihydroxy-4,5,6,7-tetrahydrobenzo[*b*]thiophen-4-carboxylic acid,
[21] (4*R*, 6*R*, 7*S*)-4,6,7-trihydroxy-2-methyl-4,5,6,7-tetrahydrobenzo[*b*]thiophen-4-carboxylic acid,
[22] (4*R*, 6*R*, 7*S*)-4,6,7-trihydroxy-2-vinyl-4,5,6,7-tetrahydrobenzo[*b*]thiophen-4- carboxylic acid,
[23] (4*R*, 6*R*, 7*S*)-4,6,7-trihydroxy-2-[(*E*)-prop-1-enyl]-4,5,6,7-tetrahydrobenzo[*b*]thiophen-4-carboxylic acid,
[24] (4*R*, 6*R*, 7*S*)-4,6,7-trihydroxy-2-(1-methyl)vinyl-4,5,6,7-tetrahydrobenzo[*b*]thiophen-4-carboxylic acid,
[25] (4*R*, 6*R*, 7*S*)-2-[(*E*)-2-cyclopropyl]vinyl-4,6,7-trihydroxy-4,5,6,7-tetrahydrobenzo[*b*]thiophen-4-carboxylic acid,
[26] (4*R*, 6*R*, 7*S*)-4,6,7-trihydroxy-2-phenyl-4,5,6,7-tetrahydrobenzo[*b*]thiophen-4- carboxylic acid,
[27] (4*R*, 6*R*, 7*S*)-2-(2-cyclopropyl)ethyl-4,6,7-trihydroxy-4,5,6,7-tetrahydrobenzo[*b*]thiophen-4-carboxylic acid,
[28] (4*R*, 6*R*, 7*S*)-4,6,7-trihydroxy-2-isopropyl-4,5,6,7-tetrahydrobenzo[b]thiophen-4-carboxylic acid,
[29] (4*R*, 6*R*, 7*S*)-2-ethyl-4,6,7-trihydroxy-4,5,6,7-tetrahydrobenzo[*b*]thiophen-4-carboxylic acid,
[30] Ethyl (1*R*, 4*S*, 5*R*)-2-allyl-3-(benzo[*b*]thiophen-2-yl)methoxy-1,4,5-trihydroxycyclohex-2-en-1-carboxylate;

or its enantiomers or its pharmaceutically acceptable salts or solvates.

## Synthesis of compounds of formula I

### Synthesis of compounds of formula Ia

Another aspect of the invention relates to a procedure of obtaining compounds of formula **Ia**, that comprises the ring opening of lactones of formula **III** in acidic medium,

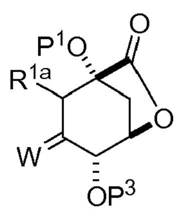

**III**

wherein, W, $P^1$, $P^3$ and $R^{1a}$ are as defined above.

The new compounds of formula **III** are useful intermediates for the synthesis of compounds of formula **Ia.** Thus, the compounds of formula **III** are also a further aspect of the invention. Accordingly, a further aspect of the invention relates to the preparation of compounds of formula **III,** comprising the *C*-alkylation of a compound of formula **II,**

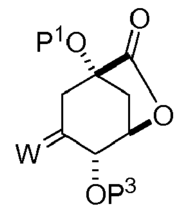

**II**

wherein W, $P^1$ and $P^3$ are as defined above.

The skilled person can choose different alkylation conditions. For example, in March, J. "Advanced Organic Chemistry; Reactions Mechanism and Structure", Wiley-Interscience, fourth ed. p. 464-473 different alkylation conditions are described. According to a particular embodiment, the alkylation is carried out in the presence of a base and of a compound $R^1L$, wherein L is a leaving group. The base is preferably selected from an anion of a dialkyl amine (amidure), such as lithium diisopropylamide, lithium hexamethyldisilazide, sodium hexamethyldisilazide or potassium hexamethyldisilazide. According to a particular embodiment, the leaving group is selected from iodide, bromide, chloride, tosylate, triflate or mesylate.

Compounds of formula **II** wherein W is =O can be obtained by a procedure described in the article: Sánchez-Sixto, C.; Prazeres, V. F. V.; Castedo, L.; Lamb, H.; Hawkins, A. R.; González-Bello, C. J. Med. Chem. 2005, 48, 4871; and Hanessian, S.; Pan, J.; Carnell, A.; Bouchard, H.; Lesage, L. Total Synthesis of (-)-Reserpine Using the Chiron Approach J. Org. Chem. 1997, 62, 465, which are hereby entirely included by reference.

Either of the compounds of formula **II**, of formula **III,** or of formula **Ia**, obtained by the process described above, and wherein W is =O, can be transformed into further compounds of formula **II**, of formula **III,** or of formula **Ia**, respectively, wherein W is =S or $=NR^b$ by methods known in the art. For example, a compound wherein W is =O can be transformed into a compound wherein W is =S, by reaction with Lawesson's reagent (other commonly known methods are described in books, such as in March, J. "Advanced Organic Chemistry; Reactions Mechanism and Structure", Wiley-Interscience, fourth ed. p. 893-895). A compound wherein W is =O can be transformed into a compound wherein W is $=NR^b$, for example, by reaction with an amine of formula $H_2NR^b$ and removal of water (other commonly known methods are described in books, such as in March, J. "Advanced Organic Chemistry; Reactions Mechanism and Structure", Wiley-Interscience, fourth ed. p. 896-898).

According to a particular embodiment, $R^1$ is an allyl chain. According to a further particular embodiment, $R^1$ is an allyl chain in a compound of formula **III,** which is preferably obtained by bromation of a compound of formula **II**, followed by an allylation reaction with a commercially available allyl tin derivative, in the presence of a catalytic amount of a radical initiator. Examples of conditions for these reactions can be found in "Bridgehead radicals in organic chemistry. An efficient construction of the ABDE ring system of the lycoctonine alkaloids", Kraus, G. A.; Andersh, B.; Su, Q.; Shi, J. Tetrahedron Letters (1993), 34(11), 1741-4; or in "Stereoselective Reactions of a (-)-Quinic Acid-Derived Enone: Application to the Synthesis of the Core of Scyphostatin", Murray, L. M.; O'Brien, P.; Taylor, R. J. K Organic Letters (2003), 5(11), 1943-1946. According to this embodiment, bromation is preferably carried out with a brominating agent selected from the group consisting of bromine, *N*-bromosuccinimide, trimethylphenylammonium tribromide, pyridinium bromide-perbromide, pyrrolidone hydrotribromide and bromine-dioxane complex. The radical initiator is preferably selected from the group consisting of *tert*-butyl hydroperoxide, *tert*-butyl perbenzoate, di(*tert*-butyl)peroxide, perbenzoic acid, peroxyacetic acid, 9-BBN, $ZnCl_2$, $SmI_2$, $Et_3B$, 2,2'-azobisisobutyronitrile or 2,2'-azobis(2-methylpropionamidine) dihydrochloride. According to a particular embodiment, the allyl tin derivative is selected from the group consisting of allyltributylstannane, tributyl(2-methylallyl)stannane, tributyl(2-phenylallyl)stannane, and tributyl(2-butylallyl)stannane.

The scope of the present invention also includes other transformations, usually functional group transformations, which transform a compound of a given formula into a different compound of the same formula. All such transformations are within the scope of the present invention.

For example, a compound of formula **III** wherein $R^{1a}$ is an alkenyl group, may be transformed into compound of formula **I** wherein $R^1$ is an alkyl group, by first opening a lactone of formula **III** in acidic medium and then performing a catalytic hydrogenolysis over the resulting compound of formula **I**. Alternatively, the order of the reactions can be reversed by first performing the catalytic hydrogenolysis over a compound of formula **III** wherein $R^{1a}$ is alkenyl, and then opening the lactone ring, to obtain a compound of formula **I** wherein $R^1$ is an alkyl group.

Catalytic hydrogenolysis can be carried out following known procedures (March, J. "Advanced Organic Chemistry; Reactions Mechanism and Structure", Wiley-Interscience, fourth ed. p. 750 and 771-780), e.g. in the presence of catalyst such as palladium on carbon, palladium hydroxide, Raney Nickel, platinum, ruthenium, platinium oxide or zinc oxide.

Also, in a compound of formula **Ia** or **III** the stereochemistry of the carbon atom supporting the $R^1$ or $R^{1a}$, respectively (position 2), can be inverted by treatment with a base (for example, see Carey, F. A.; Sundberg, R. J. "Advanced Organic Chemistry. Part B: Reaction and Synthesis", second ed. P. 1-41). For example, when $R^1$ or $R^{1a}$ is allyl, *R* configuration at position 2 is the mayor product when allylating a compound of formula **II.** The present invention also provides an epimerization reaction to obtain compounds with S configuration at position 2. This epimerization reaction can be preferably carried out by treatment of compounds of formula **III** with a base, preferably selected from the group consisting of lithium diisopropylamide, lithium hexamethyldisilazide, sodium hexamethyldisilazide and potassium hexamethyldisilazide.

Synthesis of compounds of formula Ib

A further aspect of the invention relates to a procedure of obtaining compounds of formula **Ib,** that comprises the ring opening of lactones of formula **IV** in acidic or basic medium,

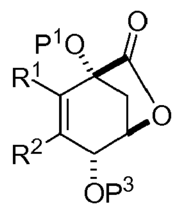

**IV**

wherein, $P^1$, $P^3$, $R^1$ and $R^2$ are as defined above.

The new compounds of formula **IV** are useful intermediates for the synthesis of compounds of formula **Ib.** Thus, the compounds of formula **IV** are also a further aspect of the invention. Accordingly, a further aspect of the invention relates to the preparation of compounds of formula **IV,** comprising: a) an *O*-, *S*- or *N*-alkylation reaction of compounds of formula **III** or of formula **II**; or b) a dialkylation reaction of compounds of formula **II.**

According to a particular embodiment, the *O*-, *S*- or *N*-alkylation reaction can be carried out by generation of the enol-type intermediate of a ketone, thioketone or imine of a compound of formula **III** in the presence of a base followed by treatment with compounds of formula $R^2L$ wherein L is a leaving group. The base is preferably selected from lithium diisopropylamide, lithium hexamethyldisilazide, sodium hexamethyldisilazide or potassium hexamethyldisilazide and the leaving group is preferably selected from iodide, bromide, chloride, tosylate, triflate or mesylate.

According to a particular embodiment, the dialkylation reaction can be carried out by generation of the enol intermediate of a ketone, thioketone or imine of a compound of formula **III** in the presence of a base followed by treatment with compounds of formula $R^1L$ wherein L is a leaving group. The base is an amine preferably selected from lithium diisopropylamide, lithium hexamethyldisilazide, sodium hexamethyldisilazide or potassium hexamethyldisilazide and the leaving group is preferably selected from iodide, bromide, chloride, tosylate, triflate or mesylate.

The *O*-, *S*- or *N*-alkylation reaction is favored by carring out the reaction in polar aprotic solvents such as DMF, HMPA, etc. In addition, higher *O*-, *S*- or *N*-alkylation ratio is obtained by using alkylating agents containing hard leaving groups, particularly oxygen-containing leaving groups, such as tosylate, mesylate or triflate or soft halides such as chloride or bromide.

On the contrary, the *C*-alkylation ratio is favored by using apolar or protic solvents such as $Et_2O$, THF, dioxane, t-BuOH, and by employing alkylating agents containing soft leaving groups such as iodide. The *O*-, *S*- or *N*-alkylation reaction is favored by using bromide as leaving group and N,N-dimethylformamide as reaction solvent. The *C*-alkylation reaction is favored by using iodide as leaving group, tetrahydrofuran as reaction solvent and in the presence of a suitable crown ether.

The skilled person can choose between *O*-, *S*- or *N*-alkylation and *C*-alkylation as mayor products through routine experimentation by introducing variations in the above mentioned factors (see pages 365-368 and 464-465 of March, J. "Advanced Organic Chemistry; Reactions Mechanism and Structure", Wiley-Interscience, fourth ed.). Also, the reaction may provide a mixture of the *O*-, *S*- or *N*-alkylated product and the dialkylated product, which can be separated using standard purification techniques.

Synthesis of compounds of formula **Ic**

A further aspect of the invention relates to a procedure of obtaining compounds of formula **Ic**, that comprises the ring opening of lactones of formula **V** in acidic or basic medium,

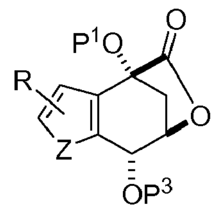

**V**

wherein, $P^1$, $P^3$, R and Z are as defined above.

The new compounds of formula **V** are useful intermediates for the synthesis of compounds of formula **Ic.** Thus, the compounds of formula **V** are also a further aspect of the invention. Accordingly, a further aspect of the invention relates to a process for the preparation of compounds of formula **V,** comprising the ozonolysis of a compound of formula **III,** wherein $R^1$ is a substituted or unsubstituted allyl group, and intramolecular cyclization, in the presence of amines, phosphites or Lawesson reagent. Conditions under which these type of reactions can be performed, may be found, for example in "COMPREHENSIVE HETEROCYCLIC CHEMISTRY II, A review of the literature 1982-1995, The Structure, Reactions, Synthesis, and Uses of Heterocyclic Compounds", Editors-in-chief Alan R. Katritzky, FRS Charles W. Rees, CBE, FRS F. V. Scriven, Volume Editor Clive W. Bird, Volume 2, "Five-membered Rings with One Heteroatom and Fused Carbocyclic Derivatives", PERGAMON. For example, Chapter 2.03 - Pyrroles and their Benzo Derivatives: Synthesis, R.J. Sundberg, University of Virginia, Charlotlesville, VA, USA, pp. 119-206, describes conditions for the synthesis of compounds of formula **V** wherein Z is $NR^b$ or $^{\oplus}NR^bR^c$. For example, Chapter 2.07 - Furans and their Benzo Derivates: Synthesis, W. Friedrichsen, Universität Kiel, Germany. pp. 351-394, describes conditions for the synthesis of compounds of formula **V** wherein Z is O. For example, Chapter 2.11 - Thiophenes and their Benzo Derivates: Synthesis, J. Nakayama, Saitama University, Japan. pp. 607-678, describes conditions for the synthesis of compounds of formula **V** wherein Z is S. According to a particular embodiment, the process comprises the ozonolysis of a compound of formula **IIIa**

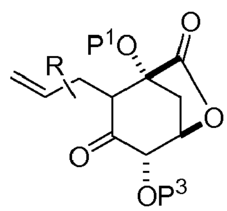

**IIIa**

wherein, $P^1$, $P^3$ and R are as defined above.

Prior to or once the cycle is formed, the resulting compound of formula **Ic** may undergo further transformations. Thus, if the group R is hydrogen in the compound of formula **IIIa**, the resulting compound of formula **Ic** can be further functionalized. Also, if R is different from hydrogen, said R group may be transformed.

For example, the compound of formula **Ic** may be optionally halogenated, and then submitted to a carbon-carbon cross-coupling reaction in order to introduce the R group. This cross-coupling reaction is typically performed in the presence of a metal catalyst, a cross-coupling reagent and a base. Preferably, the metal catalyst is selected from the group consisting of $Pd(PPh_3)_4$, $Pd(PPh_3)_2Cl_2$, $Pd(OAc)_2$, $Pd(dppf)_2Cl_2{\bullet}CH_2Cl_2$, $Pd_2(dba)_3$, $PdCl_2$, $Ni(PPh_3)_4$ and $Pd_2(dba)_3{\bullet}CHCl_3$. According to a particular embodiment, the base is selected from the group consisting of $KO^tBu$, NaOAc, $NaO^tBu$, $Ba(OH)_2$, *N*-methylmorpholine, piperidine, $(^iPr)_2EtN$, $Na_2CO_3$, $K_2CO_3$, $Cs_2CO_3$, $K_3PO_4$, $Et_3N$ and mixtures thereof. According to a particular embodiment, said cross-coupling reagent is a boronic acid or an stannane.

Preferably, the carbon-carbon cross-coupling reaction is selected from the group consisting of Suzuki-type reaction, a Heck-type reaction, a Sonogashira-type reaction, a Negishi-type reaction or a Stille-type reaction.

For the purposes of the present invention it is understood that a Suzuki-type reaction is the cross-coupling reaction catalyzed by a palladium(O) complex between a halide or a triflate and an boronic acid or its corresponding ester, or a potassium trifluoroborate. The palladium catalysts usually employed are $Pd(PPh_3)_4$, $Pd(PPh_3)_2Cl_2$, $Pd(OAc)_2$, $Pd_2(dba)_3$ o $Pd_2(dba)_3{\bullet}CHCl_3$, optionally in the presence of phosphines or arsines, typically selected from $PPh_3$, $P^tBu_3$, $PCy_3$ or $AsPh_3$. The base is usually selected from $KO^tBu$, $NaO^tBu$, NaOAc, $Ba(OH)_2$, $Na_2CO_3$, $K_2CO_3$, $Cs_2CO_3$, $K_3PO_4$, $Et_3N$, *N*-methylmorpholine or mixtures of them. This type of reactions are known to the skilled person, who can choose between a wide range of conditions (e.g., "Metal-catalyzed cross-coupling reactions", 2nd Ed., Armin de Meijere

&François Diederich, Wiley-VCH, pp 1-31 and pp 41-109)

For the purposes of the present invention it is understood that a Heck-type reaction is the reaction between an halide or a triflate with an alkene catalyzed by palladium(O). The palladium catalysts usually employed are $Pd(OAc)_2$, $PdCl_2$, $Pd(PPh_3)_4$ o $Pd_2(dba)_3$ optionally in the presence of phosphines or arsines, typically selected between $PPh_3$, $P^tBu_3$, $PCy_3$ or $AsPh_3$. The base is usually selected from $Na_2CO_3$, $K_2CO_3$, NaOAc, *N*-methylmorpholine, $K_3PO_4$, $Et_3N$ or mixtures of them. Examples of suitable Heck-type reaction conditions may be found, for example, in "Metal-catalyzed cross-coupling reactions", 2nd Ed., Armin de Meijere &François Diederich, Wiley-VCH, pp 1-31 and pp 217-296).

For the purposes of the present invention it is understood that a Stille-type reaction is the cross-coupling reaction catalyzed by a palladium(0) complex between an halide or a triflate and an organotin compound. The palladium catalysts usually employed are $Pd(dppf)_2Cl_2$•$CH_2Cl_2$, $PdCl_2$, $Pd(OAc)_2$, $Pd_2(dba)_3$ or $Pd(PPh_3)_4$, optionally in the presence of phosphines or arsines, typically selected between $PPh_3$, $P^tB_{U3}$, $PCy_3$ or $AsPh_3$ and in the presence of salts, such as LiCl, CsF, CuCl or CuI. Examples of suitable Heck-type reaction conditions may be found, for example, in "Metal-catalyzed cross-coupling reactions", 2nd Ed., Armin de Meijere &François Diederich, Wiley-VCH, pp 1-31 and pp 125-155.

For the purposes of the present invention it is understood that a Sonogashira-type reaction is the cross-coupling reaction catalyzed by a palladium(O) complex and cocatalyzed by Cu(I) between an halide or a triflate and a terminal alkyne. The typical catalysts are $Pd(PPh_3)_4$, $PdCl_2$, $Pd(OAc)_2$ or $Pd(PPh_3)_2Cl_2$ optionally in the presence of phosphines or arsines, typically selected between $PPh_3$, $P^tBu_3$, $PCy_3$ or $AsPh_3$. The base is usually selected from $Na_2CO_3$, $K_2CO_3$, $Cs_2CO_3$, $Et_3N$, $(^iPr)_2EtN$ or mixtures of them. Examples of suitable Heck-type reaction conditions may be found, for example, in "Metal-catalyzed cross-coupling reactions", 2nd Ed., Armin de Meijere &François Diederich, Wiley-VCH, pp 1-31 and pp 317-386.

For the purposes of the present invention it is understood that a Negishi-type reaction is the cross-coupling catalyzed by a palladium(O) or nickel(O) complex between an halide or a triflate and an organozinc compound. The typical catalysts are $Ni(PPh_3)_4$, $Pd(dppf)_2Cl_2$•$CH_2Cl_2$, $Pd(PPh_3)_2Cl_2$ or $Pd_2(dba)_3$ optionally in the presence of phosphines or arsines, typically selected between $PPh_3$, $P^tBu_3$ or $PCy_3$. Examples of suitable Heck-type reaction conditions may be found, for example, in "Metal-catalyzed cross-coupling reactions", 2nd Ed., Armin de Meijere &François Diederich, Wiley-VCH, pp 1-31 and pp 815-882.

According to particular embodiment, the halogenation comprises a iodation with *N*-Iodosuccinimide, followed by Suzuki-typecoupling between a boronic acid or its corresponding ester, for example, phenylboronic acid, (*E*)-prop-1-enylboronic acid, (*E*)-2-(cyclopropyl)vinyl-4,4,5,5-tetramethyl-1,3,2-dioxaborolane, in the presence of a palladium catalysts, for example, $Pd(PPh_3)_4$, $Pd_2(dba)_3$•$CHCl_3$ or $Pd_2(dba)_3$.

In summary, compounds of formula **I** can be obtained by opening of lactones of formula **III, IV** or **V.** This opening reaction can be carried out either in acidic medium, for example, in the presence of an organic acid, such as trifluoroacetic acid, *p*-toluensulfonic acid, camphorsulfonic acid, acetic acid, acidic ion-exchange resin; a Lewis acid or mixtures thereof. The reaction can also be carried our in basic medium, for example, in the presence of an inorganic base, such as $K_2CO_3$, $Na_2CO_3$, LiOH, NaOH, or KOH; an organic base, such as a primary amine, a secondary amine, MeONa or EtONa.

Also, any of the compounds of formula **I, Ia**, **Ib, Ic, III, IIIa**, **IV** or **V** may undergo protection-deprotection reactions using well-known procedures (Greene, T. W.; Wuts, P. G. M. "Protective Groups in Organic Synthesis", 3˚ Ed., Wiley-Interscience, New York, 1999). For example, if $P^1$, $P^2$ and/or $P^3$ is/are a TBS group, the deprotection will be preferably carried out by treatment with tetrabutylamonium fluoride. If $P^1$, $P^2$ and/or $P^3$ is/are a benzyl group, the deprotection will be preferably performed by catalytic hydrogenolysis. If $P^1$, $P^2$ and/or $P^3$ is/are an acetyl group, the deprotection will be preferably carried out by treatment with $K_2CO_3$ in combination with methanol. If $P^1$, $P^2$ and/or $P^3$ is/are methoxyethoxymethyl ether (MEM), the deprotection will be preferably preformed by treatment with trifluoroacetic acid.

## Biological Activity

The compounds of formula **I** are potent competitive inhibitors of type II dehydroquinases from various bacterial sources. This enzyme acts in an essential biosynthetic route in bacteria, the shikimic acid pathway. These compounds have, in many cases, inhibition constants in the low nanomolar range and to even picomolar, which makes them, the most potent known inhibitors against any dehydroquinase.

Thus a further aspect of the invention is a pharmaceutical composition comprising a compound of formula **I** as defined above and a pharmaceutically acceptable carrier.

A further aspect of the invention is a compound of formula **I** as defined above for use as a medicament.

A further aspect of the invention is the use of a compound of formula **I** for the preparation of a medicament to treat tuberculosis, stomach cancer, gastritis, stomach ulcers, duodenal ulcers, or heartburn. That is, a compound of formula **I** for use in the treatment or prophylaxis of a disease selected from the group consisting of tuberculosis, stomach cancer, gastritis, stomach ulcers, and duodenal ulcers heartburn.

Examples of pharmaceutical compositions include any solid (tablets, pills, capsules, granules etc.) or liquid (solutions, suspensions or emulsions) compositions. Typical administration routes are oral, topical or parenteral administration. In a particular embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

Suitable pharmaceutical carriers are described in "Remington's Pharmaceutical Sciences" by E.W. Martin

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of many of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

## EXAMPLES

The Examples, which are detailed next, will have to be considered to better understanding of the present invention, which should not be interpreted as a limitation.

*Example 1: (1S, 2R, 4S, 5R)-2-bromo-1,4-di(tert-butyldimethylsilyloxy)-3-oxocyclohexan-1,5-carbolactone (**VI**).* A solution of *(1S, 4S, 5R)*-1,4-di(*tert*-butyldimethylsilyloxy)-3-oxocyclohexan-1,5-carbolactone (300 mg, 0.75 mmol), under inert atmosphere and at room temperature, in dry diethyl ether (20 mL) was treated with freshly made dioxane dibromide (223 mg, 0.90 mmol). The red reaction mixture was stirred at room temperature until decoloration (1.5 h), diluted with diethyl ether and washed successively with aqueous sodium bisulfate (sat.), sodium bicarbonate (sat.) and water. The organic extract was dried with $Na_2SO_4$ (anh.), filtered and concentrated under reduced pressure to afford α-bromo ketone **VI** as a white solid (356 mg, 99%). Mp: 97-100 ˚C. $[\alpha]_D^{20}$ -116.1˚ (*c*1.2, in $CHCl_3$). $^1H$ NMR (300 MHz, $CDCl_3$) δ 4.65 (dd, 1H, *J* = 4. and 3.0 Hz), 4.27 (dd, 1H, *J* = 2.1 and 0.9 Hz), 4.09 (br d, 1H, *J* = 3.0 Hz), 3.22 (d, 1H, *J* = 9.6 Hz), 2.44 (dddd, 1H, *J* = 9.6, 4.8, 2.1 and 0.9 Hz), 0.94 (s, 9H), 0.90 (s, 9H), 0.23 (s, 3H), 0.18 (s, 3H), 0.15 (s, 3H) and 0.13 (s, 3H) ppm. $^{13}C$ NMR (75 MHz, $CDCl_3$) δ 198.9 (C), 172.1 (C), 76.1 (C), 74.2 (CH), 71.3 (CH), 53.3 (CH), 33.6 ($CH_2$), 25.6 ($C(CH_3)_3$), 25.5 ($C(CH_3)_3$), 18.4 ($C(CH_3)_3$), 18.0 ($C(CH_3)_3$), -3.2 ($SiCH_3$), -3.3 ($SiCH_3$), -5.1 ($SiCH_3$) and -5.4 ($SiCH_3$) ppm. IR (KBr) 1803 (C=O) and 1731 (C=O) $cm^{-1}$. MS (CI) *m/z* (%) 479 and 481 ($MH^+$). HRMS calcd for $C_{19}H_{36}O_5Br^{81}Si_2$ ($MH^+$): 481.1264; found, 481.1275.

The starting material of Example 1 *(1S, 4S, 5R)*-1,4-di(*tert*-butyldimethylsilyloxy)-3-oxocyclohexan-1,5-carbolactone was obtained from (1*S*, 3*R*, 4*R*, 5*R*)-3-benciloxy-1,4-dihydroxycyclohexan-1,5-carbolactone following the three steps shown below:

Step 1: (1*S*, 3*R*, 4*R*, 5*R*)-3-benciloxy-1,4-di(tert-butyldimethylsilyloxy)cyclohexan-1,5-carbolactone. To a stirred solution of (1*S*, 3*R*, 4*R*, 5*R*)-3-benciloxy-1,4-dihydroxicyclohexan-1,5-carbolactone (1.00 g, 3.79 mmol), obtained according to Hanessian, S.; Pan, J.; Carnell, A.; Bouchard, H.; Lesage, L. Total Synthesis of (-)-Reserpine Using the Chiron Approach J. Org. Chem. 1997, 62, 465, in dry DCM (13 ml) and pyridine (1.1 mL, 13.27 mmol), under inert atmosphere at 0 ˚C, was added *tert*butyldimethylsilyl trifluorosulfonate (2.6 ml, 11.37 mmol). The resultant solution was stirred at room temperature for 12 h and then diluted with DCM and water. The aqueous layer was acidified with HCl (10%) and the organic phase was separated. The aqueous phase was extracted twice with DCM. All the combined

organic extracts were dried (anh. $Na_2SO_4$), filtered and evaporated. The obtained residue was purified by flash chromatography eluting with 10% ethyl acetate-hexanes to yield (1*S*, 3*R*, 4*R*, 5*R*)-3-benciloxy-1,4-di(tert-butyldimethylsilyloxy)cyclohexan-1,5-carbolactone (1.81 g, 97%) as a colourless oil. $[\alpha]^{20}_D$ -14° (*c*1.4, in $CHCl_3$); $^1H$ NMR (250 MHz, $CDCl_3$) δ 7.31 (m, 5H), 4.45 (t, 1H, *J* 5.6), 4.42 (s, 2H), 4.11 (t, 1H, *J* 4.6), 3.42 (ddd, 1H, *J* 11.7, 6.2 and 4.1), 2.43 (d, 1H, *J* 11.4), 2.13-1.98 (m, 2H), 1.76 (t, 1H, *J* 11.9), 0.70 (s, 9H), 0.69 (s, 9H), -0.07 (s, 3H), -0.09 (s, 3H), -0.15 (s, 3H) and -0.16 (s, 3H) ppm. $^{13}C$ NMR (63 MHz, $CDCl_3$) δ 176.3, 137.8, 128.3 (2x), 127.7, 127.6 (2x), 75.9, 74.1, 73.7, 71.1, 65.7, 38.1, 37.5, 25.7 (3x), 25.6 (3x), 18.1, 18.0, -2.9 (2x), -4.5 and -5.0 ppm.

Step 2: (1*S*, 3*R*, 4*R*, 5*R*)-1,4-di(*tert*-butyldimethylsilyloxy)-3-hydroxycyclohexan-1,5-carbolactone.cursiva A suspension of (1*S*, 3*R*, 4*R*, 5*R*)-3-benciloxy-1,4-di(tert-butyldimethylsilyloxy)cyclohexan-1,5-carbolactone (270 mg, 0.55 mmol) and 20% palladium hydroxide-on-carbon (50 mg) in methanol (15 ml) was shaken under hydrogen atmosphere at room temperature for 48 h. The mixture was filtered over Celite and the residue was washed with methanol. The filtrate and washings were evaporated under reduced pressure to yield a white solid which was purified by flash chromatography eluting with 10% ethyl acetate-hexanes to yield (1*S*, 3*R*, 4*R*, 5*R*)-1,4-di(*tert*-butyldimethylsilyloxy)-3-hydroxycyclohexan-1,5-carbolactone (218 mg, 99%) as white needless. Mp 107-108 °C; $[\alpha]^{20}_D$ -1° (*c*1.1, in $CHCl_3$); $^1H$ NMR (250 MHz, $CDCl_3$) δ 4.49 (t, 1H, *J* 5.5), 3.95 (t, 1H, *J* 4.8),3.75-3.61 (m, 1H), 2.24 (d, 1H, *J* 11.4), 2.08 (m, 2H), 1.87 (d, 1H, *J* 11.6), 1.53 (t, 1H, *J* 12.6), 0.73 (s, 9H), 0.67 (s, 9H), -0.06 (s, 3H), -0.08 (s, 3H), -0.10 (s, 3H) and -0.11 (s, 3H) ppm; $^{13}C$ NMR (63 MHz, $CDCl_3$) δ 175.9, 75.6, 73.6, 67.0, 66.1, 41.4, 37.8, 25.7 (3x), 25.5 (3x), 18.0 (2x), -2.9 (2x), -4.6 and -4.9 ppm.

Step 3: (1*S*, 4*S*, 5*R*)-1,4-di(*tert*-butyldimethylsilyloxy)-3-oxocyclohexan-1,5-carbolactone. To a stirred suspension of (1*S*, 3*R*, 4*R*, 5*R*)-1,4-di(*tert*-butyldimethylsilyloxy)-3-hydroxycyclohexan-1,5-carbolactone (1.24 g, 3.09 mmol) and activated powder molecular sieves 4Å (1.24 g) in dry DCM (31 ml) was added pyridinium dichromate (1.40 g, 3.71 mmol). The resultant suspension was stirred vigorously at room temperature. After 3 h more activated powder molecular sieves 4Å (750 mg) were added and the resultant suspension was stirred for additional 2 h. The reaction mixture was filtered over a plug of Celite and silica gel and the residue was washed with diethyl ether. The filtrate and the washings were concentrated under reduced pressure. The brown solid obtained was redisolved in hot hexane and treated with activated carbon.

The black suspension was filtered over Celite and the residue was washed with hot hexane. The filtrate and the washings were concentrated and recrystallised to afford (1*S*, 4*S*, 5*R*)-1,4-di(*tert*-butyldimethylsilyloxy)-3-oxocyclohexan-1,5-carbolactone (1.17 g, 95%) as white needless. Mp 50-51 °C (hexanes); $[\alpha]^{20}_D$ -24° (*c*1.1, in $CHCl_3$); $^1H$ NMR (250 MHz, $CDCl_3$) δ 4.51 (dd, 1H, *J* 5.9 and 4.1), 3.81 (br d, 1H, *J* 4.1), 2.74 (d, 1H, *J* 17.6), 2.60 (ddd, 1H, *J* 17.6, 2.7 and 0.9), 2.50 (d, 1H, *J* 12.3), 2.44-2.35 (ddd, 1H, *J* 12.3, 5.9 and 0.9), 0.67 (s, 9H), 0.66 (s, 9H), -0.04 (s, 3H), -0.09 (s, 3H), -0.11(s, 3H), and -0.15 (s, 3H) ppm; $^{13}C$ NMR (63 MHz, $CDCl_3$) δ 203.2, 175.2, 74.1, 73.1, 71.0, 50.9, 37.0, 25.5 (3x), 25.5 (3x), 18.0, 17.9, -3.1, -3.4, -4.9 and -5.3 ppm.

*Example 2: (1R, 2R, 4S, 5R)-2-allyl-1,4-di(tert-butyldimethylsilyloxy)-3-oxocyclohexan-1,5-carbolactone (**III-1**).* A solution of the α-bromo ketone **VI** (285 mg, 0.60 mmol) in dry toluene (17 mL), under inert atmosphere, was treated allyltributyltin (0.26 mL, 0.84 mmol) and AIBN (15 mg, 0.09 mmol). The resultant reaction mixture was deoxygenated by bubbling argon through it for 30 min. and then heated at 80 °C for 14 h. After cooling at room temperature, the solvent was evaporated and the crude product was purified by flash chromatography eluting with ethyl acetate-hexane (5:95) to yield the α-allyl ketone **III-1** (265 mg, 99%) as beige solid. Mp: 91-94 °C. $[\alpha]_D^{20}$ -24.2° (*c*1.1, en $CHCl_3$). $^1H$ NMR (250 MHz, $CDCl_3$) δ 5.75 (m, 1H), 5.05 (m, 2H), 4.59 (dd, 1H, *J* = 6.3 and 4.3 Hz), 3.92 (d, 1H, *J* = 4.3 Hz), 2.89 (d, 1H, *J* = 12.5 Hz), 2.78 (m, 1H), 2.61 (m, 1H), 2.48 (m, 1H), 2.35 (m, 1H), 0.91 (s, 9H), 0.88 (s, 9H), 0.20 (s, 3H), 0.13 (s, 3H), 0.12 (s, 3H) and 0.09 (s, 3H) ppm. $^{13}C$ NMR (63 MHz, $CDCl_3$) δ 204.3 (C), 175.8 (C), 134.4 (CH), 117.4 ($CH_2$), 75.5 (C), 74.1 (CH), 71.5 (CH), 59.1 (CH), 32.2 ($CH_2$), 31.9 ($CH_2$), 25.6 ($C(CH_3)_3$), 25.5 ($C(CH_3)_3$), 18.3 ($C(CH_3)_3$), 18.0 ($C(CH_3)_3$), -3.2 ($SiCH_3$), -3.3 ($SiCH_3$), -4.8 ($SiCH_3$) and -5.4 ($SiCH_3$) ppm. IR (KBr): 1797 (C=O) and 1733 (C=O) $cm^{-1}$. MS (CI) *m*/*z* (%) 441 ($MH^+$). HRMS calcd for $C_{22}H_{41}O_5Si_2$ ($MH^+$): 441.2493; found, 441.2494. Anal. calcd for $C_{22}H_{40}O_5Si_2.H_2O$: C, 57.60; H, 9.23. Found: C, 57.80; H, 9.17.

*Example 3: (2R)-2-allyl-3-dehydroquinic acid [**(2R)-Ia-1**].* A solution of the silyl ether **III-1** (250 mg, 0.57 mmol) in aqueous trifluoroacetic acid (5.7 mL, 50%) was heated at 90 °C for 3 h. After cooling at room temperature, the solvents were removed under reduced pressure. The crude residue was dissolved in water and washed with ethyl acetate (2x). The aqueous phase was lypholised and the crude product was purified by HPLC using a semipreparative column Merck LiChroCART RP-18 (10 μm, 250x10 mm) with a gradient 0-50% B (35 min) at a flow rate of 5 ml $min^{-1}$. The eluents for this column were: (A) water with 0.1 % TFA and (B) acetonitrile with 0.1 % TFA. Allyl derivative **(2*R*)-Ia-1** (60 mg, 46%) was obtained as a white solid. Mp: 144-148 °C. $[\alpha]_D^{20}$ -30.3° (*c*1.0, in $H_2O$). $^1H$ NMR (400 MHz, $D_2O$) δ 5.82 (m, 1H), 5.10 (ddd, 1H, *J* = 17.2, 3.2 and 1.6 Hz), 5.03 (ddd, 1H, *J* = 10.0, 3.2 and 1.2 Hz), 4.40 (dd, 1H, *J* = 9.6 and 1.2 Hz),

3.89 (ddd, 1H, *J* = 11.2, 9.6 and 5.2 Hz), 3.28 (ddd, 1H, *J* = 8.4, 4.8 and 1.2 Hz), 2.62 (m, 1H), 2.41 (dd, 1H, *J* = 11.2 and 13.6 Hz), 2.34 (dd, 1H, *J* = 13.6 and 5.2 Hz) and 1.98 (m, 1H) ppm. $^{13}C$ NMR (75 MHz, $D_2O$) δ 211.0 (C), 179.5 (C), 138.8 (CH), 119.4 ($CH_2$), 83.9 (CH), 80.1 (C), 74.7 (CH), 56.3 (CH), 43.7 ($CH_2$) and 30.4 ($CH_2$) ppm. IR (KBr): 3449 (O-H), 3302 (O-H) and 1733 (C=O) $cm^{-1}$. MS (ESI) *mlz* (%) 253 ($MNa^+$). HRMS calcd for $C_{10}H_{14}O_6Na$ ($MNa^+$): 253.0683; found, 253.0689.

*Example 4: (2R)-2-propyl-3-dehydroquinic acid [**(2R)-Ia-2**].* A suspension of the allyl derivative **(2*R*)-Ia-1** (15.4 mg, 0.067 mmol) and 10% palladium-on-carbon (2 mg) in methanol (0.7 mL) was stirred under hydrogen atmosphere at room temperature for 3 h. The mixture was filtered over Celite and the residue was washed with methanol. The filtrate and washings were evaporated, redissolved in water and lypholised to yield propyl derivative **(2*R*)-Ia-2** (15.5 mg, 99%) as a light yellow solid. $[\alpha]_D^{20}$ -29.0° (*c*1.7, in $H_2O$). $^1H$ NMR (250 MHz, $D_2O$) δ 4.31 (d, 1H, *J* = 9.3 Hz), 3.80 (m, 1H), 3.06 (d, 1H, *J* = 9.0 Hz), 2.37 (d, 1H, *J* = 13.5 Hz), 2.24 (m, 1H), 1.80 (m, 1H), 1.39-0.88 (m, 3H) and 0.80 (t, 3H, *J* = 7.3 Hz) ppm. $^{13}C$ NMR (63 MHz, $D_2O$) δ 209.5 (C), 177.8 (C), 81.6 (CH), 78.8 (C), 72.6 (CH), 54.3 (CH), 41.2 ($CH_2$), 26.0 ($CH_2$), 21.2 ($CH_2$) and 13.8 ($CH_3$) ppm. IR (KBr): 3431 (O-H) and 1726 (C=O) $cm^{-1}$. MS (ESI) *mlz* (%) 255 ($MNa^+$). HRMS calcd for $C_{10}H_{16}O_6Na$ ($MH^+$): 255.0839; found, 255.0848.

*Example 5 (epimerization): (1R, 2S, 4S, 5R)-2-allyl-1,4-di(tert-butyldimethylsilyloxy)-3-oxocyclohexan-1,5-carbolactone (**III2**).* A stirred solution of the (2*R*)-2-allyl ketone **III-1** (100 mg, 0.23 mmol) in dry THF (6.1 mL), under argon and at room temperature, was treated with a solution of LHMDS (345 μL, 0.35 mmol, 1.0 M in THF) and 12-crown-4 ether (4 μL, 0.023 mmol). The reaction mixture was stirred for 30 min and then diluted successively with diethyl ether and water. The organic layer was separated and the aqueous phase was extracted with diethyl ether (3x). All the combined organic extracts were dried (anh. $Na_2SO_4$), filtered and evaporated in vacuo. The obtained residue was purified by flash chromatography eluting with ethyl acetate-hexanes (10:90) to yield (2*S*)-2-allyl ketone **III-2** as a light yellow oil (75 mg, 74%). $[\alpha]_D^{20}$ -26.2° (*c*1.0, in $CHCl_3$). $^1H$ NMR (400 MHz, $CDCl_3$) δ 5.92 (m, 1H), 5.05 (dq, 1H, *J* = 17.2 and 1.6 Hz), 4.98 (m, 1H), 4.58 (dd, 1H, *J* = 6.4 and 4.0 Hz), 4.02 (br d, 1H, *J* = 4.0 Hz), 2.91 (dd, 1H, *J* = 8.4 and 3.6 Hz), 2.75 (d, 1H, *J* = 12.0 Hz), 2.62 (ddd, 1H, *J* = 12.0, 6.4 and 0.8 Hz), 2.53 (m, 1H), 2.36 (m, 1H), 0.93 (s, 9H), 0.88 (s, 9H), 0.23 (s, 3H), 0.14 (s, 3H), 0.13 (s, 3H) and 0.09 (s, 3H) ppm. $^{13}C$ NMR (63 MHz, $CDCl_3$) δ 204.9 (C), 173.9 (C), 137.0 (CH), 115.8 ($CH_2$), 75.7 (C), 74.0 (CH), 72.4 (CH), 60.0 (CH), 38.0 ($CH_2$), 27.5 ($CH_2$), 25.6 (2x($C(CH_3)_3$), 18.1 ($C(CH_3)_3$), 18.0 ($C(CH_3)_3$), -3.3 ($SiCH_3$), -3.3 ($SiCH_3$), -4.8 ($SiCH_3$) and -5.2 ($SiCH_3$) ppm. IR (film): 1803 (C=O) and 1730 (C=O) $cm^{-1}$. MS (CI) *mlz* (%) 441 ($MH^+$). HRMS calcd for $C_{22}H_{41}O_5Si_2$ ($MH^+$): 441.2493; found, 441.2490.

*Example 6: (2S)-2-allyl-3-dehydroquinic acid [**(2S)-Ia-I**].* The same experimental procedure was used as in the synthesis of acid **(2*R*)-Ia-1** (Example 3), but using **III-2** as starting material (71 mg, 0.16 mmol). Yield = 25 mg (68%). Mp: 127-130 °C. $[\alpha]_D^{20}$ +17.3° (*c*1.8, in $H_2O$). $^1H$ NMR (400 MHz, $D_2O$) δ 5.67 (m, 1H), 5.15 (td, 1H, *J* = 17.2 and 1.2 Hz), 5.09 (d, 1H, *J* = 10.4 Hz), 4.40 (d, 1H, *J* = 9.6 Hz), 3.90 (m, 1H), 2.81 (m, 1H) and 2.55-2.33 (m, 4H) ppm. $^{13}C$ NMR (100 MHz, $D_2O$) δ 213.9 (C), 178.7 (C), 136.4 (CH), 120.8 ($CH_2$), 81.5 (CH), 79.4 (C), 74.0 (CH), 61.2 (CH), 38.4 ($CH_2$) and 36.1 ($CH_2$) ppm. IR (KBr): 3442 (O-H), 3415 (O-H), 1724 (C=O) and 1714 (C=O) $cm^{-1}$. MS (ESI) *mlz* (%) 253 ($MNa^+$). HRMS calcd for $C_{10}H_{14}O_6Na$ ($MNa^+$): 253.0683; found, 253.0682.

*General alkylation and hydrolysis/deprotection method for Examples 7-16.* A flame-dried round bottom flask was charged with *(1S, 4S,* 5*R*)-1,4-di(tert-butyldimethylsilyloxy)-3-oxocyclohexan-1,5-carbolactone (1 equivalent) and then dissolved in dry THF (0.04 M). The resultant solution was treated with 1.5 equivalents of LHMDS solution (1.0 M in THF) and 0.1 equivalents of 12-crown-4 ether. After being stirred for 20 min, a solution of the corresponding iodide (1.5 equivalents) in dry THF (0.6 M) was added. After 4 h, the reaction mixture was diluted successively with diethyl ether and ammonium chloride (sat.). The organic phase was separated and the aqueous layer was extracted three times with diethyl ether. All combined organic extracts were dried (anh. $Na_2SO_4$), filtered and evaporated under reduced pressure. The obtained residue was purified by flash chromatography eluting with diethyl ether-hexanes (10:90) to afford the corresponding compound of formula **III** as a mixture of diastereoisomers. The obtained alkyl ketones **III** were dissolved in 50% aqueous trifluoroacetic acid (0.1 M) and then were heated at 70 °C for 2-3 h. After cooling at room temperature, the solvents were removed under reduced pressure. The crude residue was dissolved in water and washed with ethyl acetate (2x). The aqueous phase was lypholised and the crude product was purified by HPLC using a semipreparative column Merck LiChroCART RP-18 (10 μm, 250x10 mm) with a gradient 0-50% B (35 min) at a flow rate of 5 ml $min^{-1}$. The eluents for this column were: (A) water with 0.1% TFA and (B) acetonitrile with 0.1% TFA. **(2*R*)-Ia-(3-7)** and **(2*S*)-Ia-(3-7)** were obtained. The spectroscopic data of the synthesized compounds using this method is indicated above:

*Example 7: (2R)-2-benzyl-3-dehydroquinic acid [**(2R)-Ia-3**].* Experimental procedure using general alkylation method. Overall yield = 5%. White solid. Mp: 146-148 °C. $^1H$ NMR (400 MHz, $D_2O$) δ 7.39-7.24 (m, 5H), 4.34 (dd, 1H,

$J$ = 9.6 and 1.2 Hz), 3.90 (ddd, 1H, $J$ = 9.6, 11.6 and 5.2 Hz), 3.54 (ddd, 1H, $J$ = 8.8, 3.6 and 1.2 Hz), 3.21 (dd, 1H, $J$ = 14.4 and 8.8 Hz), 2.52 (dd, 1H, $J$ = 14.4 and 3.6 Hz), 2.43 (dd, 1H, $J$ = 14.0 and 11.6 Hz) and 2.34 (dd, 1H, $J$ = 14.0 and 5.2 Hz) ppm. $^{13}C$ NMR (75 MHz, $D_2O$) δ 208.4 (C), 177.1 (C), 140.2 (C), 129.6 (2xCH), 129.0 (2xCH), 127.0 (CH), 81.5 (CH), 78.2 (C), 72.4 (CH), 56.3 (CH), 41.3 ($CH_2$) and 29.7 ($CH_2$) ppm. IR (KBr): 3435 (O-H) and 1728 (C=O) $cm^{-1}$. MS (ESI) *mlz* (%) 303 ($MNa^+$). HRMS calcd for $C_{14}H_{16}O_6Na$ ($MH^+$): 303.0839; found, 303.0838.

*Example 8: (2S)-2-benzyl-3-dehydroquinic acid ácido [(**2S)-Ia-3**].* Experimental procedure using general alkylation method. Overall yield = 20%. White solid. Mp: 131-134 ˚C. $^1H$ NMR (400 MHz, $D_2O$) δ 7.39 (m, 2H), 7.33 (m, 1H), 7.25 (m, 2H), 4.61 (d, 1H, $J$ = 9.2 Hz), 3.93 (ddd, 1H, $J$ = 9.2, 11.6 and 5.2 Hz), 3.01 (m, 3H), 2.64 (dd, 1H, $J$ = 14.4 and 11.6 Hz) and 2.44 (dd, 1H, $J$ = 14.4 and 5.2 Hz) ppm. $^{13}C$ NMR (75 MHz, $D_2O$) δ 210.1 (C), 175.0 (C), 136.7 (C), 128.8 (2xCH), 128.6 (2xCH), 127.0 (CH), 78.4 (CH), 76.3 (C), 70.9 (CH), 60.2 (CH), 35.1 ($CH_2$) and 34.6 ($CH_2$) ppm. IR (KBr): 3520 (O-H), 3475 (O-H), 3256 (O-H), 1719 (C=O) and 1706 (C=O) $cm^{-1}$. MS (ESI) *m/z* (%) 303 ($MNa^+$). HRMS calcd for $C_{14}H_{16}O_6Na$ ($MH^+$): 303.0839; found, 303.0836.

*Example 9: (2R)-2-(4-methyl)benzyl-3-dehydroquinic acid [(**2R)-Ia-4**].* Experimental procedure using general alkylation method. Overall yield = 5%. White solid. Mp: 105-108 ˚C. $[\alpha]_D^{20}$ -47.0˚ (*c*1.6, in $H_2O$). $^1H$ NMR (400 MHz, $D_2O$) δ 7.16 (m, 4H), 4.29 (d, 1H, $J$ = 9.6 Hz), 3.89 (m, 1H), 3.47 (m, 1H), 3.16 (dd, 1H, $J$ = 14.0 and 8.4 Hz), 2.48-2.32 (m, 3H) and 2.29 (s, 3H) ppm. $^{13}C$ NMR (100 MHz, $D_2O$) δ 210.7 (C), 179.4 (C), 139.5 (C), 139.4 (C), 132.0 (2xCH), 132.0 (2xCH), 83.9 (CH), 80.5 (C), 74.8 (CH), 58.7 (CH), 43.6 ($CH_2$), 31.7 ($CH_2$) and 23.0 ($CH_3$) ppm. IR (KBr): 3433 (O-H) and 1726 (C=O) $cm^{-1}$. MS (ESI) *mlz* (%) 317 ($MNa^+$). HRMS calcd for $C_{15}H_{18}O_6Na$ ($MNa^+$): 317.0996; found, 317.0993.

*Example 10: (2S)-2-(4-methyl)benzyl-3-dehydroquinic acid [(**2S)-Ia-4**].* Experimental procedure using general alkylation method. Overall yield = 23%. White solid. Mp: 134-138 ˚C. $[\alpha]_D^{20}$ -23.2˚ (*c*1.0, in $CH_3OH$). $^1H$ NMR (400 MHz, $CD_3OD$) δ 7.03 (d, 2H, $J$ = 8.0 Hz), 6.98 (d, 2H, $J$ = 8.0 Hz), 4.34 (d, 1H, $J$ = 9.2 Hz), 3.80 (ddd, 1H, $J$ = 9.2, 14.4 and 5.2 Hz), 2.99 (ddd, 1H, $J$ = 11.2, 5.2 and 1.2 Hz), 2.86 (m, 2H), 2.45 (dd, 1H, $J$ = 14.4 and 11.6 Hz), 2.30 (ddd, 1H, $J$ = 1.2, 14.4 and 5.2 Hz) and 2.23 (s, 3H) ppm. $^{13}C$ NMR (100 MHz, $CD_3OD$) δ 209.0 (C), 175.5 (C), 137.4 (C), 135.6 (C), 130.3 (2xCH), 129.6 (2xCH), 80.2 (CH), 77.6 (C), 73.1 (CH), 61.4 (CH), 37.1 ($CH_2$), 36.1 ($CH_2$) and 21.1 ($CH_3$) ppm. IR (KBr): 3484 (O-H), 3438 (O-H), 3346 (O-H) and 1718 (C=O) $cm^{-1}$. MS (ESI) *m/z* (%) 317 ($MNa^+$). HRMS calcd for $C_{15}H_{18}O_6Na$ ($MNa^+$): 317.0996; found, 317.1004.

*Example 11: (2R)-2-(4-methoxy)benzyl-3-dehydroquinic acid [(**2R)-Ia-5**].* Experimental procedure using general alkylation method. Overall yield = 4%. White solid. $^1H$ NMR (250 MHz, $D_2O$) δ 7.16 (d, 2H, $J$ = 8.8 Hz), 6.87 (d, 2H, $J$ = 8.8 Hz), 4.26 (d, 1H, $J$ = 9.5 Hz), 3.84 (m, 1H), 3.76 (s, 3H), 3.43 (dd, 1H, $J$ = 8.3 and 4.3 Hz), 3.10 (dd, 1H, $J$ = 14.5 and 8.5 Hz) and 2.45-2.19 (m, 3H) ppm. $^{13}C$ NMR (75 MHz, $D_2O$) δ 208.7 (C), 177.6 (C), 157.8 (C), 132.9 (C), 130.8 (2xCH), 114.5 (2xCH), 81.6 (CH), 78.5 (C), 72.6 (CH), 56.6 (CH), 55.9 ($CH_3$), 41.4 ($CH_2$) and 28.9 ($CH_2$) ppm. IR (KBr): 3390 (O-H) and 1736 (C=O) $cm^{-1}$. MS (ESI) *mlz* (%) 333 ($MNa^+$). HRMS calcd. for $C_{15}H_{18}O_7Na$ ($MNa^+$): 333.0945; found, 333.0946.

*Example 12: (2S)-2-(4-Methoxy)benzyl-3-dehydroquinic acid [(**2S)-Ia-5**].* Experimental procedure using general alkylation method. Overall yield = 16%. White solid. Mp: 138-143 ˚C. $[\alpha]_D^{20}$ -36.2˚ (*c*1.1, in MeOH). $^1H$ NMR (250 MHz, $D_2O$) δ 7.08 (d, 2H, $J$ = 8.8 Hz), 6.87 (d, 2H, $J$ = 8.8 Hz), 4.51 (d, 1H, $J$ = 9.3 Hz), 3.86 (ddd, 1H, $J$ = 14.5, 9.3 and 5.3 Hz), 3.74 (s, 3H), 2.88 (m, 3H), 2.55 (dd, 1H, $J$ = 14.5 and 11.3 Hz) and 2.37 (ddd, 1H, $J$ = 14.5, 5.3 and 1.5 Hz) ppm. $^{13}C$ NMR (63 MHz, $D_2O$) δ 210.8 (C), 175.6 (C), 158.3 (C), 130.4 (2xCH), 129.8 (C), 114.8 (2xCH), 79.1 (CH), 76.9 (C), 71.6 (CH), 61.0 (CH), 55.9 ($CH_3$), 35.7 ($CH_2$) and 34.4 ($CH_2$) ppm. IR (KBr): 3367 (O-H), 1739 (C=O) and 1720 (C=O) $cm^{-1}$. MS (ESI) *mlz* (%) 333 ($MNa^+$). HRMS calcd for $C_{15}H_{18}O_7Na$ ($MNa^+$): 333.0945; found, 333.0934.

*Example 13: (2R)-2-perfluorobenzyl-3-dehydroquinic acid [(**2R)-Ia-6**].* Experimental procedure using general alkylation method. Overall yield = 2%. White solid. $^1H$ NMR (400 MHz, $D_2O$) δ 4.37 (d, 1H, $J$ = 9.6 Hz), 3.90 (ddd, 1H, $J$ = 11.6, 9.6 and 5.2 Hz), 3.64 (t, 1H, $J$ = 6.6 Hz), 3.22 (dd, 1H, $J$ = 14.8 and 7.2 Hz), 2.76 (dd, 1H, $J$ = 14.8 and 6.4 Hz), 2.38 (dd, 1H, $J$ = 11.6 and 13.6 Hz) and 2.31 (dd, 1H, $J$ = 13.6 and 5.6 Hz) ppm. $^{13}C$ NMR (75 MHz, $D_2O$) 8207.9 (C), 177.0 (C), 147.8-135.9 (5xC, m), 112.6 (C, m), 81.3 (CH), 77.3 (C), 72.2 (CH), 53.1 (CH), 41.6 ($CH_2$) and 17.4 ($CH_2$) ppm. $^{19}F$ NMR (282 MHz, $D_2O$) δ -140.3 (dd, 2F, $J$ = 21.8 and 6.1 Hz), -155.9 (t, 2F, $J$ = 21.8 Hz) and -160.1 (td, 1F, $J$ = 21.8 and 6.1 Hz) ppm. IR (KBr): 3435 (O-H) and 1730 (C=O) $cm^{-1}$. MS (ESI) *mlz* (%) 393 ($MNa^+$). HRMS calcd for $C_{14}H_{11}O_6F_5Na$ ($MNa^+$): 393.0368; found, 393.0366.

*Example 14: (2S)-2-perfluorobenzyl-3-dehydroquinic acid* ***[(2S)-Ia-6].*** Experimental procedure using general alkylation method. Overall yield = 9%. White solid. Mp: 123-125 ˚C. $[\alpha]_D^{20}$ -27.4˚ (*c*1.3, in $H_2O$). $^1H$ NMR (400 MHz,

$D_2O$) δ 4.64 (d, 1H, *J* = 9.2 Hz), 3.96 (ddd, 1H, *J* = 11.2, 9.2 and 5.2 Hz), 3.19 (dd, 1H, *J* = 14.8 and 11.6 Hz), 3.09 (dd, 1H, *J* = 14.8 and 4.4 Hz), 2.92 (ddd, 1H, *J* = 11.6, 4.4 and 2.0 Hz), 2.58 (dd, 1H, *J* = 14.4 and 11.2 Hz) and 2.47 (ddd, 1H, *J* = 14.4, 5.2 and 2.0 Hz) ppm. $^{13}C$ NMR (63 MHz, $D_2O$) δ 210.3 (C), 175.4 (C), 147.6-135.8 (5xC, m), 110.8 (C, td, *J* = 2.8 and 18.5 Hz), 78.5 (CH), 76.7 (C), 71.2 (CH), 58.2 (CH), 35.6 ($CH_2$) and 22.4 ($CH_2$) ppm. $^{19}F$ NMR (282 MHz, $D_2O$) δ -141.4 (dd, 2F, *J* = 21.7 and 6.2 Hz), -154.0 (t, 2F, *J* = 21.7 Hz) and -160.5 (td, 1F, *J* = 21.7 and 6.2 Hz) ppm. IR (KBr): 3437 (O-H) and 1720 (C=O) $cm^{-1}$. MS (ESI) *m*/*z* (%) 393 ($MNa^+$). HRMS calcd for $C_{14}H_{11}O_6F_5Na$ ($MNa^+$): 393.0368; found, 393.0360.

*<u>Example 15</u>: (2R)-2-(benzo[b]thiophen-5-yl)methyl-3-dehydroquinic acid* ***[(2R)-Ia-7].*** Experimental procedure using general alkylation method. Overall yield = 5%. $[\alpha]_D^{20}$ -60.1° (*c*1.8, in $CH_3OH$). $^1H$ NMR (250 MHz, $CD_3OD$) δ 7.63 (m, 2H), 7.39 (d, 1H, *J* = 5.3 Hz), 7.17 (dd, 1H, *J* = 5.5 and 0.5 Hz), 7.13 (dd, 1H, *J* = 8.3 and 1.8 Hz), 4.00 (d, 1H, *J* = 9.3 Hz), 3.77 (ddd, 1H, *J* = 11.0, 9.3 and 5.5 Hz), 3.37-3.24 (m, 2H), 2.44 (m, 1H), 2.22 (dd, 1H, *J* = 13.5 and 11.0 Hz) and 2.14 (dd, 1H, *J* = 13.5 and 5.5 Hz) ppm. $^{13}C$ NMR (63 MHz, $CD_3OD$) δ 207.0 (C), 176.7 (C), 141.3 (C), 138.9 (C), 138.0 (C), 127.5 (CH), 127.0 (CH), 125.0 (CH), 124.7 (CH), 122.9 (CH), 82.9 (CH), 78.7 (C), 73.5 (CH), 57.9 (CH), 42.8 ($CH_2$) and 30.9 ($CH_2$) ppm. IR (KBr): 3496 (O-H), 3421 (O-H) and 1736 (C=O) $cm^{-1}$. MS (ESI) *m*/*z* (%) 359 ($MNa^+$). HRMS calcd. for $C_{16}H_{16}O_6SNa$ ($MNa^+$): 359.0560; found, 359.0564.

*<u>Example 16</u>: (2S)-2-(benzo[b]thiophen-5-yl)methyl-3-dehydroquinic acid* ***[(2S)-Ia-7].*** Experimental procedure using general alkylation method. Overall yield = 19%. White solid. Mp: 152-155 °C. $[\alpha]_D^{20}$ -37.6° (*c*1.1, in $CH_3OH$). $^1H$ NMR (250 MHz, $CD_3OD$) δ 7.66 (d, 1H, *J* = 8.3 Hz), 7.49 (s, 1H), 7.41 (d, 1H, *J* = 5.5 Hz), 7.17 (d, 1H, *J* = 5.5 Hz), 7.02 (dd, 1H, *J* = 8.3 and 1.3 Hz), 4.34 (d, 1H, *J* = 9.0 Hz), 3.76 (ddd, 1H, *J* = 14.5, 9.0 and 5.3 Hz), 2.96 (m, 3H), 2.43 (dd, 1H, *J* = 14.5 and 11.5 Hz) and 2.25 (ddd, 1H, *J* = 14.5, 5.3 and 1.0 Hz) ppm. $^{13}C$ NMR (63 MHz, $CD_3OD$) δ 209.0 (C), 175.4 (C), 141.5 (C), 139.6 (C), 134.8 (C), 127.9 (CH), 126.2 (CH), 124.7 (CH), 124.5 (CH), 123.5 (CH), 80.2 (CH), 77.6 (C), 73.0 (CH), 61.6 (CH), 37.1 ($CH_2$) and 36.4 ($CH_2$) ppm. IR (KBr): 3483 (O-H), 3431 (O-H), 3379 (O-H) and 1730 (C=O), 1711 and 1703 (C=O) $cm^{-1}$. MS (ESI) *m*/*z* (%) 359 ($MNa^+$). HRMS calcd. for $C_{16}H_{16}O_6SNa$ ($MNa^+$): 359.0560; found, 359.0559.

*<u>Example 17</u>: (1R, 4S, SR)-3-(benzo[b]thiophen-2-yl)methoxy-1,4-di(tert-butyldimethylsilyloxy)cyclohex-2-en-1,5-carbolactone* ***(IV-1)*** *and (1R, 4S, 5R)-3-(benzo[b]thiophen-2-yl)methoxy-2-(benzo[b]thiophen-2-yl)methyl-1,4-di (tert-butyldimethylsilyloxy)cyclohex-2-en-1,5-carbolactone* ***(IV-2).*** A flame-dried round bottom flask was charged with *(1R, 4S, 5R)*-1,4-di(*tert*-butyldimethylsilyloxy)-3-oxocyclohexan-1,5-carbolactone (250 mg, 0.63 mmol) and then dissolved in dry DMF (17 mL). The resultant solution was treated with LHMDS (1.3 mL, 1.26 mmol, 1.0 M in THF) and was stirred at room temperature for 20 min. Then, a solution of 2-(bromomethyl)benzo[*b*]thiophene (215 mg, 0.95 mmol) in dry DMF (1.6 mL) was added. After 30 min., the reaction mixture was diluted succesively with diethyl ether and water. The organic phase was separated and the aqueous layer was extracted three times with diethyl ether. All combined organic extracts were dried (anh. $Na_2SO_4$), filtered and evaporated under reduced pressure. The obtained residue was purified by flash chromatography eluting with diethyl ether-hexanes (5:95) to afford *O*-alkyl derivative **IV-1** (63 mg, 18%) and dialkyl derivative **IV-2** (128 mg, 29%), both as light yellow oils.

Data for **IV-1:** $[\alpha]_D^{20}$ -103.3° (*c*2.1, in $CHCl_3$). $^1H$ NMR (250 MHz, $CDCl_3$) δ 7.86-7.79 (m, 1H), 7.78-7.72 (m, 1H), 7.35 (m, 2H), 7.25 (m, 1H), 5.06 (s, 1H), 5.01 (d, 1H, *J* = 12.0 Hz), 4.95 (d, 1H, *J* = 12.0 Hz), 4.49 (dd, 1H, *J* = 5.5 and 3.5 Hz), 4.19 (d, 1H, *J* = 3.5 Hz), 2.42 (d, 1H, *J* = 10.8 Hz), 2.34 (ddd, 1H, *J* = 10.8, 5.5 and 1.0 Hz), 0.93 (s, 9H), 0.89 (s, 9H), 0.17 (s, 3H), 0.13 (s, 3H), 0.11 (s, 3H) and 0.10 (s, 3H) ppm. $^{13}C$ NMR (63 MHz, $CDCl_3$) δ 176.1 (C), 153.2 (C), 140.3 (C), 139.1 (C), 138.9 (C), 124.5 (CH), 124.3 (CH), 123.6 (CH), 123.3 (CH), 122.4 (CH), 105.3 (CH), 75.3 (CH), 73.7 (C), 67.4 (CH), 65.2 ($CH_2$), 38.0 ($CH_2$), 25.7 ($C(CH_3)_3$), 25.6 ($C(CH_3)_3$), 18.1 ($C(CH_3)_3$), 18.0 ($C(CH_3)_3$), -3.1 (2x$SiCH_3$), -4.4 ($SiCH_3$) and -5.1 ($SiCH_3$) ppm. IR (film): 1803 (C=O) $cm^{-1}$. MS (CI) *m*/*z* (%) 547 ($MH^+$). HRMS calcd for $C_{28}H_{43}O_5SSi_2$ ($MH^+$): 547.2370; found, 547.2372.

Data for **IV-2:** $[\alpha]_D^{20}$ -148.6° (*c*1.0, in $CHCl_3$). $^1H$ NMR (250 MHz, $CDCl_3$) δ 7.82-7.67 (m, 3H), 7.61 (m, 1H), 7.38-7.20 (m, 4H), 7.06 (s, 1H), 7.00 (s, 1H), 5.07 (s, 2H), 4.60 (dd, 1H, *J* = 5.5 and 3.3 Hz), 4.50 (d, 1H, *J* = 3.3 Hz), 3.98 (d, 1H, *J* = 15.5 Hz), 3.83 (d, 1H, *J* = 15.5 Hz), 2.60 (d, 1H, *J* = 10.8 Hz), 2.48 (dd, 1H, *J* = 10.8 and 5.8 Hz), 0.99 (s, 9H), 0.80 (s, 9H), 0.24 (s, 3H), 0.22 (s, 3H), 0.20 (s, 3H) and 0.10 (s, 3H) ppm. $^{13}C$ NMR (63 MHz, $CDCl_3$) δ 175.2 (C), 148.7 (C), 144.0 (C), 140.1 (C), 139.7 (C), 139.4 (C), 139.1 (C), 128.9 (2xC), 124.4 (CH), 124.2 (CH), 123.8 (CH), 123.6 (CH), 123.1 (CH), 122.8 (CH), 122.6 (CH), 122.3 (CH), 121.9 (CH), 121.2 (CH), 74.7 (C), 74.6 (CH), 68.5 ($CH_2$), 67.4 (CH), 37.5 ($CH_2$), 25.7 ($CH_2$ + $C(CH_3)_3$), 25.5 ($C(CH_3)_3$), 18.1 ($C(CH_3)_3$), 18.0 ($C(CH_3)_3$), -3.3 ($SiCH_3$), -3.4 ($SiCH_3$),

-4.4 ($SiCH_3$) and -4.5 ($SiCH_3$) ppm. IR (film): 1799 (C=O) $cm^{-1}$. MS (CI) *m/z* (%) 693 ($MH^+$). HRMS calcd for $C_{37}H_{49}O_5S_2Si_2$ ($MH^+$): 693.2560; found, 693.2563.

*Example 18: (1R, 4S, 5R)-3-(benzo[b]thiophen-2-yl)methoxy-1,4-dihydroxycyclohex-2-en-1,5-carbolactone **(IV-3).*** To a stirred solution of silylether **IV-1** (42 mg, 0.077 mmol) in dry THF (1.1 mL), under argon at 0 °C, was added tetrabutylammonium fluoride (0.20 mL, 0.20 mmol, *ca* 1.0 M in THF). After 1 h, the ice-bath was removed and the reaction mixture was stirred for another hour. The solvent was evaporated and the obtained residue was dissolved in a mixture of ethyl acetate and water. The aqueous phase was acidified with dilute HCl and the organic layer was separated. The aqueous phase was extracted with ethyl acetate (x2). Al the combined organic extracts were dried (anh. $Na_2SO_4$), filtered and concentrated under reduced pressure. The obtained residue was purified by flash chromatography eluting with (60:40) diethyl ether-hexanes to yield diol **IV-3** (24 mg, 99%) as a colourless oil. $[\alpha]_D^{20}$ -151.2° (*c*1.1, in MeOH). $^1H$ NMR (250 MHz, $CD_3OD$) δ 7.77 (m, 1H), 7.70 (m, 1H), 7.32-7.21 (m, 3H), 5.13 (s, 1H), 5.02 (br s, 2H), 4.57 (m, 1H), 4.07 (d, 1H, *J* = 3.3 Hz) and 2.27 (m, 2H) ppm. $^{13}C$ NMR (63 MHz, $CD_3OD$) δ 179.2 (C), 155.3 (C), 141.6 (C), 140.8 (C), 140.8 (C), 125.7 (CH), 125.5 (CH), 124.8 (CH), 124.6 (CH), 123.3 (CH), 105.4 (CH), 77.0 (CH), 73.0 (C), 67.6 (CH), 66.3 ($CH_2$) and 38.3 ($CH_2$) ppm. IR (KBr): 3390 (O-H) and 1765 (C=O) $cm^{-1}$. MS (ESI) *m/z* (%) 319 ($MH^+$). HRMS calcd for $C_{16}H_{15}O_5S$ ($MH^+$): 319.0635; found, 319.0634.

*Example 19: Sodium (1R, 4S, 5R)-3-(benzo[b]thiophen-2-yl)methoxy-1,4,5-trihydroxycyclohex-2-en-1-carboxylate **(Ib-1).*** A solution of lactone **IV-3** (28 mg, 0.088 mmol) in THF (0.8 mL) and aqueous NaOH (176 μL, 0.088 mmol, 0.5M) was stirred at room temperature for 15 min. Water was added and THF was evaporated under reduced pressure. The aqueous solution was washed with diethyl ether (x2) and liophilisated to afford *O*-alkyl derivative **Ib-1** (31 mg, 98%) as beige solid. $[\alpha]_D^{20}$ -52.0° (*c*1.3, in MeOH). $^1H$ NMR (250 MHz, $CD_3OD$) δ 7.74 (m, 1H), 7.68 (m, 1H), 7.24 (m, 3H), 5.01 (d, 1H, *J* = 12.5 Hz), 4.94 (d, 1H, *J* = 12.5 Hz), 4.82 (s, 1H), 3.87 (m, 2H) and 2.05 (m, 2H) ppm. $^{13}C$ NMR (63 MHz, $CD_3OD$) δ 182.1 (C), 157.1 (C), 141.6 (2xC), 140.9 (C), 125.5 (CH), 125.4 (CH), 124.7 (CH), 124.1 (CH), 123.3 (CH), 103.6 (CH), 74.7 (C), 72.4 (CH), 71.6 (CH), 65.9 ($OCH_2$) and 37.4 ($CH_2$) ppm. IR (KBr): 3435 (O-H), 1664 (C=O), 1610 (C=O) and 1585 (C=O) $cm^{-1}$. MS (ESI) *m/z* (%) 359 ($MH^+$). HRMS calcd for $C_{16}H_{16}O_6SNa$ ($MH^+$): 359.0560; found, 359.0560.

*Example 20: (1R, 4S, 5R)-3-(benzo[b]thiophen-2-yl)methoxy-2-(benzo[b]thiophen-2-yl)methyl-1,4-dihydroxycyclohex-2-en-1,5-carbolactone **(IV-4).*** The same experimental procedure as used for compound **IV-3** (example 18), was applied to silyl ether **IV-2** (75 mg, 0.11 mmol) in 1.6 mL of THF and 0.29 mL of tetrabutylammonium fluoride (0.29 mmol). Yield = 50 mg (98%). Light yellow oil. $[\alpha]_D^{20}$ -228.5° (*c*1.0, in acetone). $^1H$ NMR (250 MHz, acetone-$d_6$) δ 7.89 (m, 1H), 7.76 (m, 2H), 7.61 (m, 1H), 7.39-7.19 (m, 5H), 7.12 (m, 1H), 5.50-5.34 (m, 2H), 4.70 (m, 2H), 4.01 (d, 1H, *J* = 14.8 Hz), 3.82 (d, 1H, *J* = 14.8 Hz), 2.52 (dd, 1H, *J* = 11.0 and 2.8 Hz) and 2.42 (dd, 1H, *J* = 11.0 and 5.8 Hz) ppm. $^{13}C$ NMR (63 MHz, acetone-$d_6$) δ 177.7 (C), 149.8 (C), 146.4 (C), 143.1 (C), 142.2 (C), 141.9 (C), 141.5 (C), 141.3 (CH), 126.3 (CH), 126.2 (CH), 125.6 (CH), 125.5 (CH), 125.0 (CH), 124.9 (C), 124.6 (CH), 124.5 (CH), 124.2 (CH), 123.7 (CH), 123.3 (CH), 76.9 (CH), 74.3 (C), 67.4 ($CH_2$), 67.2 (CH), 39.1 ($CH_2$) and 26.6 ($CH_2$) ppm. IR (film): 3415 (O-H) and 1788 (C=O) $cm^{-1}$. MS (ESI) *m/z* (%) 487 ($MNa^+$). HRMS calcd for $C_{25}H_{20}O_5S_2Na$ ($MH^+$): 487.0644; found, 487.0644.

*Example 21: Sodium (1R, 4S, 5R)-3-(benzo[b]thiophen-2-yl)methoxy-2-(benzo[b]thiophen-2-yl)methyl-1,4-dihydroxycyclohex-2-en-1-carboxylate **(Ib-2).*** The same experimental procedure as used for the synthsis of compound **Ib-1** (example 19), was applied to silyl ether **IV-4** (52 mg, 0.11 mmol) in 1 mL of THF and 220 μL of NaOH (aq.). Yield = 54 mg (97%). Beige solid. $[\alpha]_D^{20}$ -62.7° (*c*1.5, in MeOH). $^1H$ NMR (400 MHz, DMSO-$d_6$) δ 8.62 (d, 1H, *J* = 8.0 Hz), 7.87 (m, 1H), 7.74 (m, 1H), 7.69 (m, 1H), 7.55 (m, 1H), 7.35-7.27 (m, 2H), 7.25-7.16 (m, 3H), 7.04 (s, 1H), 5.23 (br s, 1H), 5.19-5.12 (m, 3H), 4.10 (br s, 1H), 3.63 (m, 2H), 3.23 (d, 1H, *J* = 15.2 Hz), 2.12 (dd, 1H, *J* = 14.0 and 3.2 Hz) and 1.70 (dd, 1H, *J* = 14.0 and 3.2 Hz) ppm. $^{13}C$ NMR (63 MHz, DMSO-$d_6$) δ 177.3 (C), 150.1 (C), 146.3 (C), 142.1 (C), 139.8 (C), 139.2 (C), 139.0 (C), 138.9 (C), 124.2 (CH), 124.1 (CH), 123.6 (CH), 123.4 (CH), 122.7 (CH), 122.4 (CH), 122.2 (CH), 121.9 (CH), 121.8 (CH), 120.8 (CH), 120.5 (C), 74.2 (C), 69.8 (CH), 68.0 (CH), 64.2 ($CH_2$), 34.9 ($CH_2$) and 26.2 ($CH_2$) ppm. IR (KBr): 3398 (O-H) and 1601 (C=O) $cm^{-1}$. MS (ESI) *m/z* (%) 505 ($MH^+$). HRMS calcd for $C_{25}H_{22}O_6S_2Na$ ($MH^+$): 505.0750; found, 505.0751.

*Example 22: (1R, 4S, 5R)-1,4-di(tert-butyldimethylsilyloxy)-3-(5-methylbenzo[b] thiophen-2-yl)methoxycyclohex-2-en-1,5-carbolactone **(IV-5)** and (1R, 4S, 5R)-1,4-di(tert-butyldimethylsilyloxy)-2-(S-methylbenzo[b]thiophen-2-yl)methyl-3-(5-methylbenzo[b]thiophen-2-yl)methoxycyclohex-2-en-1,5-carbolactone **(IV-6).*** The experimental procedure used was the same as for compounds **IV-1** and **IV-2** (exmple 17) using the following: First, 200 mg of *(1R, 4S,*

5R)-1,4-di(*tert*-butyldimethylsilyloxy)-3-oxocyclohexan-1,5-carbolactone (0.50 mmol) in 13.3 mL of DMF and 1.0 mL of LHMDS (1.00 mmol) was used for the enolate generation. In the alkylation step, 180 mg of 2-(bromomethyl)-5-methylbenzo[b]thiophen (0.75 mmol) in 1.3 mL of DMF was used. Yield = 62 mg (22%) of **IV-5** and 99 mg (28%) of **IV-6,** both as light yellow oils. Data for **IV-5:** $[\alpha]_D^{20}$ -120.6° (*c*1.0, in $CHCl_3$). $^1H$ NMR (250 MHz, $CDCl_3$) δ 7.70 (d, 1H, *J* = 8.5 Hz), 7.54 (s, 1H), 7.17 (m, 2H), 5.05 (s, 1H), 4.98 (d, 1H, *J* = 12.0 Hz), 4.92 (d, 1H, *J* = 12.0 Hz), 4.48 (dd, 1H, *J* = 5.3 and 3.5 Hz), 4.17 (d, 1H, *J* = 3.5 Hz), 2.46 (s, 3H), 2.44-2.30 (m, 2H), 0.92 (s, 9H), 0.87 (s, 9H), 0.17 (s, 3H), 0.12 (s, 3H), 0.10 (s, 3H) and 0.08 (s, 3H) ppm. $^{13}C$ NMR (63 MHz, $CDCl_3$) δ 176.1 (C), 153.3 (C), 139.4 (C), 138.9 (C), 137.4 (C), 134.0 (C), 126.3 (CH), 123.6 (CH), 123.0 (CH), 122.0 (CH), 105.2 (CH), 75.3 (CH), 73.7 (C), 67.4 (CH), 65.2 ($OCH_2$), 38.0 ($CH_2$), 25.7 ($C(CH_3)_3$), 25.6 ($C(CH_3)_3$), 21.3 ($CH_3$), 18.1 ($C(CH_3)_3$), 18.0 ($C(CH_3)_3$), -3.1 (2x$SiCH_3$), -4.4 ($SiCH_3$) and -5.2 ($SiCH_3$) ppm. IR (Film): 1801 (C=O) $cm^{-1}$. MS (CI) *m/z* (%) 561 ($MH^+$). HRMS calcd for $C_{29}H_{45}O_5SSi_2$ ($MH^+$): 561.2526; found, 561.2530. Data for **IV-6:** $[\alpha]_D^{20}$ -75.4° (*c*1.3, in $CHCl_3$). $^1H$ NMR (250 MHz, $CDCl_3$) δ 7.65 (d, 1H, *J* = 8.0 Hz), 7.59 (d, 1H, *J* = 8.0 Hz), 7.45 (s, 1H), 7.37 (s, 1H), 7.13 (d, 1H, *J* = 8.3 Hz), 7.05 (d, 1H, *J* = 8.3 Hz), 6.95 (s, 1H), 6.87 (s, 1H), 5.02 (s, 2H), 4.57 (d, 1H, *J* = 5.8 and 3.3 Hz), 4.46 (d, 1H, *J* = 3.3 Hz), 3.93 (d, 1H, *J* = 15.5 Hz), 3.79 (d, 1H, *J* = 15.5 Hz), 2.56 (d, 1H, *J* = 10.8 Hz), 2.45 (m, 1H), 2.44 (s, 3H), 2.42 (s, 3H), 0.96 (s, 9H), 0.78 (s, 9H), 0.21 (s, 3H), 0.19 (s, 3H), 0.16 (s, 3H) and 0.07 (s, 3H) ppm. $^{13}C$ NMR (63 MHz, $CDCl_3$) δ 175.2 (C), 148.6 (C), 144.0 (C), 140.4 (C), 139.8 (C), 139.5 (C), 137.3 (C), 136.5 (C), 133.9 (C), 133.3 (C), 129.0 (C), 126.1 (CH), 124.8 (CH), 123.6 (CH), 122.7 (CH), 122.6 (CH), 121.9 (CH), 121.5 (CH), 121.0 (CH), 74.7 (C), 74.6 (CH), 68.5 ($CH_2$), 67.4 (CH), 37.5 ($CH_2$), 25.7 ($CH_2$ + $C(CH_3)_3$), 25.5 ($C(CH_3)_3$), 21.4 ($CH_3$), 21.3 ($CH_3$), 18.1 ($C(CH_3)_3$), 18.0 ($C(CH_3)_3$), -3.3 ($SiCH_3$), -3.4 ($SiCH_3$) and -4.5 (2x$SiCH_3$) ppm. IR (film): 1799 (C=O) $cm^{-1}$. MS (CI) *m/z* (%) 721 ($MH^+$). HRMS calcd for $C_{39}H_{53}O_5S_2Si_2$ ($MH^+$): 721.2873; found, 721.2878.

*Example 23: (1R, 4S, 5R)-1,4-dihydroxy-3-(5-methylbenzo[b]thiophen-2-yl)methoxycyclohex-2-en-1,5-carbolactone **(IV-7).*** The experimental procedure used was the same as for compound **IV-3** (example 18), but using silyl ether **IV-5** as starting material (72 mg, 0.13 mmol) in 1.9 mL of THF and 0.34 mL de tetrabutylammonium fluoride (0.34 mmol). Yield = 34 mg (79%). Beige solid. $[\alpha]_D^{20}$ -135.1° (*c*1.4, in $Me_2CO$). $^1H$ NMR (250 MHz, $CD_3OD$) δ 7.63 (d, 1H, *J* = 8.3 Hz), 7.50 (m, 1H), 7.21 (m, 1H), 7.10 (m, 1H), 5.14 (s, 1H), 5.00 (s ancho, 2H), 4.60 (m, 1H), 4.08 (d, 1H, *J* = 3.3 Hz), 2.38 (s, 3H) and 2.28 (m, 2H) ppm. $^{13}C$ NMR (63 MHz, $CDCl_3$) δ 179.2 (C), 155.3 (C), 141.1 (C), 140.8 (C), 138.8 (C), 135.3 (C), 127.3 (CH), 124.7 (CH), 124.4 (CH), 123.0 (CH), 105.4 (CH), 77.0 (CH), 73.0 (C), 67.6 (CH), 66.4 ($OCH_2$), 38.4 ($CH_2$) and 21.4 ($CH_3$) ppm. IR (KBr): 3444 (O-H), 3377 (O-H) and 1765 (C=O) $cm^{-1}$. MS (CI) *m/z* (%) 333 ($MH^+$). HRMS calcd for $C_{17}H_{17}O_5S$ ($MH^+$): 333.0797; found, 333.0786.

*Example 24: Sodium (1R, 4S, 5R)-1,4,5-trihydroxy-3-(5-methylbenzo[b]thiophen-2-yl)methoxycyclohex-2-en-1-carboxylate **(Ib-3).*** The experimental procedure used was the same as for compound **Ib-1** (example 19), but using carbolactone **IV-7** as starting material (21 mg, 0.063 mmol) in 0.6 mL of THF and 126 μL of NaOH (aq.). Yield = 22 mg (95%). Beige solid. $[\alpha]_D^{20}$ -41.9° (*c*1.5, in MeOH). $^1H$ NMR (250 MHz, $CD_3OD$) δ 7.63 (d, 1H, *J* = 8.3 Hz), 7.50 (br s, 1H), 7.21 (s, 1H), 7.09 (dd, 1H, *J* = 8.3 and 1.0 Hz), 5.01 (d, 1H, *J* = 12.3 Hz), 4.94 (d, 1H, *J* = 12.3 Hz), 4.84 (s, 1H), 3.88 (m, 2H), 2.38 (s, 3H) and 2.06 (m, 2H) ppm. $^{13}C$ NMR (63 MHz, $CD_3OD$) δ 182.1 (C), 157.2 (C), 141.7 (C), 141.3 (C), 138.9 (C), 135.2 (C), 127.1 (CH), 124.6 (CH), 123.9 (CH), 122.9 (CH), 103.5 (CH), 74.7 (C), 72.4 (CH), 71.6 (CH), 66.0 ($OCH_2$), 37.4 ($CH_2$) and 21.5 ($CH_3$) ppm. IR (KBr): 3435 (O-H), 1649 and 1618 (C=O) $cm^{-1}$. MS (ESI) *m/z* (%) 373 ($MH^+$). HRMS calcd for $C_{17}H_{18}O_6SNa$ ($MH^+$): 373.0716; found, 373.0729.

*Example 25: (1R, 4S, 5R)-1,4-dihydroxy-3- (5-methylbenzo[b]thiophen-2-yl)methoxy-2-(5-methylbenzo[b]thiophen-2-yl)methylcyclohex-2-en-1,5-carbolactone **(IV-8).*** The experimental procedure used was the same as for compound **IV-3** (example 18), but using silyl ether **IV-6** as starting material (119 mg, 0.17 mmol) in 2.4 mL of THF and 0.44 mL de tetrabutylammonium fluoride(0.44 mmol). Yield = 70 mg (84%). Beige solid. $[\alpha]_D^{20}$ -232.4° (*c*1.7, in $Me_2CO$). $^1H$ NMR (250 MHz, acetone-$d_6$) δ 7.74 (d, 1H, *J* = 8.3 Hz), 7.62 (d, 1H, *J* = 8.3 Hz), 7.52 (s, 1H), 7.35 (s, 1H), 7.24 (s, 1H), 7.17 (dd, 1H, *J* = 8.0 and 0.8 Hz), 7.06 (dd, 1H, *J* = 8.0 and 0.8 Hz), 6.99 (s, 1H), 5.43 (d, 1H, *J* = 12.5 Hz), 5.35 (d, 1H, *J* = 12.5 Hz), 4.68 (m, 2H), 3.97 (d, 1H, *J* = 14.8 Hz), 3.79 (d, 1H, *J* = 14.8 Hz), 2.51 (d, 1H, *J* = 12.5 Hz), 2.45-2.38 (m, 1H), 2.41 (s, 3H) and 2.38 (s, 3H) ppm. $^{13}C$ NMR (63 MHz, acetone-$d_6$) δ 177.8 (C), 149.8 (C), 146.5 (C), 143.1 (C), 142.5 (C), 141.7 (C), 139.2 (C), 138.7 (C), 135.7 (C), 135.1 (C), 127.9 (CH), 126.6 (CH), 125.4 (CH), 125.0 (C), 124.5 (CH), 124.4 (CH), 123.8 (CH), 123.3 (CH), 123.1 (CH), 76.9 (CH), 74.3 (C), 67.4 ($OCH_2$), 67.2 (CH), 39.1 ($CH_2$), 26.6 ($CH_2$) and 22.3 (2x$CH_3$) ppm. IR (film): 3471 (O-H), 3344 (O-H) and 1770 (C=O) $cm^{-1}$. MS (CI) *m/z* (%) 493 ($MH^+$).

HRMS calcd for $C_{27}H_{25}O_5S_2$ ($MH^+$): 493.1143; found, 493.1131.

*Example 26: Sodium (1R, 4S, 5R)-1,4,5-trihydroxy-3-(5-methylbenzo[b]thiophen-2-yl)methoxy-2-(5-methylbenzo[b]thiophen-2-yl)methylcyclohex-2-en-1-carboxylate* ***(Ib-4)***. The experimental procedure used was the same as for compound **Ib-1** (example 19), but using carbolactone **IV-8** as starting material (35 mg, 0.071 mmol) in 0.65 mL of THF and 142 μL of NaOH (aq.). Yield = 22 mg (95%). Beige solid. $[\alpha]_D^{20}$ -61.3° (*c*1.5, in MeOH). $^1$H NMR (400 MHz, DMSO-$d_6$) δ 8.63 (d, 1H, *J* = 8.0 Hz), 7.73 (d, 1H, *J* = 8.0 Hz), 7.61 (d, 1H, *J* = 8.0 Hz), 7.45 (br s, 1H), 7.29 (br s, 1H, ArH), 7.13 (dd, 1H, *J* = 8.0 and 1.6 Hz), 7.08 (br s, 1H), 7.01 (dd, 1H, *J* = 8.0 and 1.6 Hz), 6.92 (s, 1H), 5.21 (br s, 1H), 5.15 (br s, 1H), 5.14 (d, 1H, *J* = 13.2 Hz), 5.10 (d, 1H, *J* = 13.2 Hz), 4.08 (br s, 1H), 3.64 (m, 1H), 3.60 (d, 1H, *J* = 15.2 Hz), 3.19 (d, 1H, *J* = 15.2 Hz), 2.38 (s, 3H), 2.35 (s, 3H), 2.11 (dd, 1H, *J* = 14.0 and 3.2 Hz) and 1.68 (dd, 1H, *J* = 14.0 and 3.2 Hz) ppm. $^{13}$C NMR (100 MHz, DMSO-$d_6$) δ 177.2 (C), 150.1 (C), 146.4 (C), 142.1 (C), 140.1 (C), 139.4 (C), 136.4 (C), 136.1 (C), 133.3 (C), 132.5 (C), 125.7 (CH), 124.3 (CH), 123.3 (CH), 122.2 (CH), 122.0 (CH), 121.7 (CH), 121.4 (CH), 120.6 (CH), 120.5 (C), 74.3 (C), 69.8 (CH), 68.0 (CH), 64.2 ($CH_2$), 34.9 ($CH_2$), 26.3 ($CH_2$), 21.0 ($CH_3$) and 20.9 ($CH_3$) ppm. IR (KBr): 3435 (O-H) and 1599 (C=O) cm$^{-1}$.

*Example 27: (1R, 4S, 5R)-2-allyl-1,4-di(tert-butyldimethylsilyloxy)-3-(benzo[b]thiophen-2-yl)methoxycyclohex-2-en-1,5-carbolactone* ***(IV-9).*** To a solution of KHMDS (1.8 mL, 0.91 mmol, 0.5 M in toluene) in dry DMF (3 mL), under argon and at -78 °C, a solution of (2*S*)-2-allyl ketone **III-1** (200 mg, 0.45 mmol) in DMF (3 mL) and toluene (1.9 mL), both dry, was added. The resultant solution was stirred at this temperatura for 30 min. A solution of 2-(bromomethyl) benzo[b]thiophene (206 mg, 0.91 mmol) in DMF (1.8 mL) and toluene (1.2 mL), both dry, was then added. After 1 h, water and brine were added. The aqueous phase was extracted with diethyl ether (3 x 2 mL). All the combined organic extracts were dried (anh. $MgSO_4$), filtered and concentrated. The obtained residue was purified by flash chromatography over silica gel eluting with diethyl ether/hexanes [1°) (0:100), 2°) (20:80)] to afford 118 mg (45%) of ether **IV-9.** Light yellow oil. $[\alpha]_D^{20}$ -100° (*c*1.0, in $CHCl_3$). $^1$H NMR (250 MHz, $CDCl_3$) δ 7.66 (dd, 1H, *J* = 6.5 and 2.2 Hz), 7.59 (dd, 1H, *J* = 6.5 and 2.5 Hz), 7.17 (m, 2H), 7.03 (s, 1H), 5.87-5.71 (m, 1H), 4.95-4.80 (m, 4H), 4.34 (dd, 1H, *J* = 4.7 and 3.5 Hz), 4.17 (d, 1H, *J* = 3.5 Hz), 2.96 (d, 2H, *J* = 6.2 Hz), 2.26 (m, 2H), 0.78 (s, 9H), 0.77 (s, 9H), 0.08 (s, 3H), 0.02 (s, 3H), 0.00 (s, 3H) and -0.03 (s, 3H) ppm. $^{13}$C NMR (75 MHz, $CDCl_3$) δ 176.3 (C), 148.3 (C), 140.9 (C), 139.2 (C), 136.5 (CH), 131.2 (C), 125.1 (CH), 125.0 (CH), 124.3 (CH), 123.1 (CH), 123.0 (CH), 122.9 (C), 116.1 ($CH_2$), 77.3 (C), 75.4 (CH), 69.8 ($CH_2$), 68.1 (CH), 38.0 ($CH_2$), 30.1 ($CH_2$), 26.4 ($C(CH_3)_3$), 26.2 ($C(CH_3)_3$), 18.9 ($C(CH_3)_3$), 18.7 (*C*($CH_3$)$_3$), -2.5 ($SiCH_3$), -2.6 ($SiCH_3$), -3.8 ($SiCH_3$) and -4.0 ($SiCH_3$) ppm. IR (film): 1799 (C=O) cm$^{-1}$. MS (CI) *m*/*z* (%) 587 ($MH^+$). HRMS calcd for $C_{31}H_{47}O_5SSi_2$ ($MH^+$): 587.2683; found, 587.2682.

*Example 28: (1R, 4S, 5R)-2-allyl-1,4-dihydroxy-3-(benzo[b]thiophen-2-yl)methoxycyclohex-2-en-1,5-carbolactone* ***(IV-10)*** The experimental procedure used was the same as for compound **IV-3** (example 18), but using silyl ether **IV-9** as starting material (78 mg, 0.13 mmol) in 0.9 mL of THF and 0.29 mL of tetrabutylammonium fluoride (0.29 mmol). Yield = 38 mg (83%). Beige solid. Mp: 122-125 °C. $[\alpha]_D^{20}$ -143° (*c*1.5, in MeOH). $^1$H NMR (250 MHz, $CD_3OD$) δ 7.76 (m, 1H), 7.69 (m, 1H, ArH), 7.26 (m, 2H), 7.22 (s, 1H), 5.78 (m, 1H), 5.22 (d, 1H, *J* = 12.5 Hz), 5.12 (d, 1H, *J* = 12.5 Hz), 4.97 (dq, 1H, *J* = 17.0 and 1. 7 Hz), 4.81 (m, 1H), 4.55 (m, 1H), 4.40 (d, 1H, *J* = 3.5 Hz), 3.00 (d, 2H, *J* = 6.5 Hz) and 2.29 (m, 2H) ppm. $^{13}$C NMR (63 MHz, $CD_3OD$) δ 178.8 (C), 148.1 (C), 142.3 (C), 141.5 (C), 140.8 (C), 137.1 (CH), 126.5 (C), 125.5 (CH), 125.3 (CH), 124.7 (CH), 123.8 (CH), 123.2 (CH), 115.5 ($CH_2$), 76.8 (CH), 73.8 (C), 67.2 ($CH_2$), 66.4 ($CH_2$), 38.3 ($CH_2$) and 29.5 ($CH_2$) ppm. IR (KBr): 3482 (O-H), 3369 (O-H) and 1780 (C=O) cm$^{-1}$. MS (ESI) *m*/*z* (%) 381 ($MNa^+$). HRMS calcd for $C_{19}H_{18}O_5SNa$ ($MNa^+$): 381.0751; found, 381.0758.

*Example 29: Sodium (1R, 4S, 5R)-2-allyl-3-(benzo[b]thiophen-2-yl)methoxy-1,4,5-trihydroxycyclohex-2-en-1-carboxylate* ***(Ib-5).*** The experimental procedure used was the same as for compound **Ib-1** (example 19), but using carbolactone **IV-10** as starting material (30 mg, 0.084 mmol) in 0.75 mL of THF and 160 μL of NaOH (aq.). Yield = 33 mg (99%). Beige solid. $[\alpha]_D^{20}$ -49° (*c*1.1, in $H_2O$). $^1$H NMR (250 MHz, $D_2O$) δ 7.80 (m, 2H), 7.34 (m, 2H), 7.30 (s, 1H), 5.79 (m, 1H), 5.09 (d, 1H, *J* = 11.8 Hz), 4.98 (m, 3H), 4.32 (d, 1H, *J* = 6.7 Hz), 3.93 (m, 1H), 2.91 (dd, 1H, *J* = 15.0 and 6.5 Hz), 2.66 (dd, 1H, *J* = 15.0 and 6.5 Hz) and 2.05 (m, 2H) ppm. $^{13}$C NMR (63 MHz, $D_2O$) δ 180.5 (C), 150.9 (C), 140.8 (C), 140.7 (C), 139.8 (C), 137.4 (CH), 125.4 (CH), 125.2 (CH), 124.6 (CH), 123.9 (C), 123.1 (CH), 115.9 ($CH_2$), 77.1 (C), 70.7 (CH), 70.6 (CH), 39.4 ($CH_2$) and 31.5 ($CH_2$) ppm. IR (KBr): 3427 (O-H) and 1597 (C=O) cm$^{-1}$. MS (ESI) *m*/*z* (%) 399 ($MH^+$). HRMS calcd for $C_{19}H_{20}O_6SNa$ ($MH^+$): 399.0873; found, 399.0887.

*Example 30: (1R, 2R, 4S, 5R)-1,4-di(tert-butyldimethylsilyloxy)-2-(2-methyl)allyl-3-oxocyclohexan-1,5-carbol-*

*actone **(III-3)**.* A solution of the α-bromo ketone **VI** (500 mg, 1.04 mmol) in dry toluene (21 mL), under inert atmosphere, was treated 2-methylallyltributyltin (504 mg, 1.46 mmol) and AIBN (26 mg, 0.16 mmol). The resultant reaction mixture was deoxygenated by bubbling argon through it for 30 min. and then heated at 80 ˚C for 14 h. After cooling at room temperature, the solvent was evaporated and the crude product was purified by flash chromatography eluting with ethyl acetate-hexane (5:95) affording 2-methylallyl ketone **III-3** (457 mg, 96%) as white solid. Mp: 93-95 ˚C. $[\alpha]_D^{20}$ -29.1˚ (*c*1.1, in $CHCl_3$). $^1H$ NMR (250 MHz, $CDCl_3$) δ 4.81 (br s, 1H), 4.70 (br s, 1H), 4.59 (dd, 1H, *J* = 6.2 and 4.3 Hz), 3.92 (d, 1H, *J* = 4.3 Hz), 2.88 (d, 1H, *J* = 12.3 Hz), 2.75 (m, 2H), 2.49 (m, 1H), 2.37 (m, 1H), 1.75 (s, 3H), 0.91 (s, 9H), 0.89 (s, 9H), 0.22 (s, 3H), 0.14 (s, 3H), 0.12 (s, 3H) and 0.09 (s, 3H) ppm. $^{13}C$ NMR (63 MHz, $CDCl_3$) δ 203.0 (C), 175.8 (C), 141.3 (C), 114.0 ($CH_2$), 75.5 (C), 74.1 (CH), 71.7 (CH), 57.3 (CH), 35.9 ($CH_2$), 32.0 ($CH_2$), 25.6 (2x$C(CH_3)_3$), 21.6 ($CH_3$), 18.2 ($C(CH_3)_3$), 18.0 ($C(CH_3)_3$), -3.2 ($SiCH_3$), -3.3 ($SiCH_3$), -4.7 ($SiCH_3$) and -5.4 ($SiCH_3$) ppm. IR (KBr): 1801 (C=O) and 1731 (C=O) $cm^{-1}$. MS (CI) *m*/*z* (%) 455 ($MH^+$). HRMS calcd for $C_{23}H_{43}O_5Si_2$ ($MH^+$): 455.2649; found, 455.2648.

*<u>Example 31</u>: (4R, 6R, 7S)-4,7-di(tert-butyldimethylsilyloxy)-4,5,6,7-tetrahydrobenzo[b]thiophen-4,6-carbolactone **(V-1)**.* A solution of allylketone **III-1** (1.6 g, 3.64 mmol) in dicloromethane (125 mL) was placed into a gas-washing bottle and cooled down to -78 ˚C in a dry ice bath. The mixture was then flushed with argon for 5 min, oxygen for 5 min and then with ozone for 12 min. The end of the reaction was indicated by the orange coloration of the aqueous KI (5%), which is located at the exit. Afterwards, the reaction mixture was flushed for 10 min with nitrogen to remove excess ozone. The gas-washing bottle was taken from the cooling bath and the content was slowly warmed to room temperature. The solvent was evaporated under reduced pressure to afford an oil, which it was used without further purification in the subsequent cyclization reaction. The obtained residue was dissolved in dry toluene (30 mL), under argon, was treated with Lawesson's reagent (1.4 g, 3.46 mmol) and heated at 100 ˚C for 5 hours. After cooling to room temperature, the reaction mixture was poured into cool hexane. The obtained precipitate was filtered and washed with more hexane. The filtrate and the washings were treated with activated carbon and filtered. The solvent were concentrated and the obtained residue was crystallized from (99: 1) ethanol-diethyl ether to afford tiophene **V-1** (922 mg, 58%) as white needles. Mp: 133-137 ˚C (EtOH-$Et_2O$). $[\alpha]_D^{20}$ -96.6˚ (*c*1.0, in $CHCl_3$). $^1H$ NMR (250 MHz, $CDCl_3$) δ 7.17 (d, 1H, *J* = 5.2 Hz), 6.99 (d, 1H, J = 5.2 Hz), 4.82 (d, 1H, *J* = 3.2 Hz), 4.60 (dd, 1H, *J* = 5.5 and 3.2 Hz), 2.52 (d, 1H, *J* = 10.7 Hz), 2.44 (dd, 1H, *J* = 10.7 and 5.5 Hz), 0.90 (s, 9H), 0.84 (s, 9H), 0.18 (s, 3H), 0.15 (s, 3H), 0.12 (s, 3H) and 0.07 (s, 3H) ppm. $^{13}C$ NMR (63 MHz, $CDCl_3$) δ 175.2 (C), 141.6 (C), 134.6 (C), 125.9 (CH), 123.2 (CH), 77.3 (CH), 75.7 (C), 66.2 (CH), 37.9 ($CH_2$), 25.6 (2x$C(CH_3)_3$), 18.2 (*C*$(CH_3)_3$), 18.0 ($C(CH_3)_3$), -3.1 ($SiCH_3$), -3.2 ($SiCH_3$) and -4.7 (2x$SiCH_3$) ppm. IR (KBr): 1797 (C=O) $cm^{-1}$. MS (ESI) *m*/*z* (%) 441 ($MH^+$). HRMS calcd for $C_{21}H_{37}O_4SSi_2$ ($MH^+$): 441.1946; found, 441.1964.

*<u>Example 32</u>: (4R, 6R, 7S)-4,7-dihydroxy-4,5,6,7-tetrahydrobenzo[b]thiophen-4,6-carbolactone **(V-2)**.* To a stirred solution of the silylether **V-1** (70 mg, 0.16 mmol) in dry THF (2.3 mL), under argon at 0 ˚C, was added tetrabutylammonium fluoride (0.41 mL, 0.41 mmol, *ca* 1.0 M en THF). After stirring for 15 min at 0 ˚C, the ice bath was removed and the reaction mixture was stirred for 1 h. The solvent was concentrated and the obtained residue was disolved in ethyl acetate and water. The aqueous phase was acidified with HCl (10%). The organic phase was separated and the aqueous phase was extracted with ethyl acetate (x2). The combined organic extracts were dried (anh. $Na_2SO_4$), filtered and concentrated under reduced pressure to yield **V-2** (29 mg, 85%) as a colourless oil. $[\alpha]_D^{20}$ -112.2˚ (*c*1.0, in MeOH). $^1H$ NMR (250 MHz, $CD_3OD$) δ 7.44 (d, 1H, *J* = 5.2 Hz), 7.11 (d, 1H, *J* = 5.2 Hz), 4.84 (m, 3H), 4.64 (br s, 1H) and 2.52 (m, 2H) ppm. $^{13}C$ NMR (63 MHz, $CD_3OD$) δ 178.4 (C), 142.2 (C), 136.4 (C), 127.7 (CH), 123.5 (CH), 79.2 (CH), 75.1 (C), 66.6 (CH) and 38.5 ($CH_2$) ppm. IR (KBr): 3523, 3305 (O-H), 1779 and 1753 (C=O) $cm^{-1}$. MS (ESI) *m*/*z* (%) 235 ($MNa^+$). HRMS calcd for $C_9H_8O_4SNa$ ($MH^+$): 235.0033; found, 235.0036.

*<u>Example 33</u>: (4R, 6R, 7S)-4,6,7-trihydroxy-4,5,6,7-tetrahydrobenzo[b]thiophen-4-carboxylic acid **(Ic-1)**.* A solution of the lactone **V-2** (25 mg, 0.12 mmol) in THF (0.6 mL) and aqueous LiOH (0.6 mL, 0.30 mmol, 0.5M) was stirred at room temperature for 10 min. Water was added and THF was removed under reduced pressure. The resultant aqueous solution was washed with diethyl ether (x2) and the aqueous extract was treated with Amberlite IR-120 until pH 6. The resin was filtered and washed with miliQ water. The filtrate and the washings were lyophilised to afford acid **Ic-1** (25 mg, 92%) as beige solid. Mp: 128-131 ˚C. $[\alpha]_D^{20}$ -9.2˚ (*c*1.5, in MeOH). $^1H$ NMR (250 MHz, $D_2O$) δ 7.33 (d, 1H, *J* = 5.0 Hz), 6.77 (d, 1H, *J* = 5.0 Hz), 4.63 (d, 1H, *J* = 8.3 Hz), 3.97 (m, 1H), 2.33 (dd, 1H, *J* = 13.5 and 12.0 Hz), and 2.16 (dd, 1H, *J* = 13.5 and 3.8 Hz) ppm. $^{13}C$ NMR (63 MHz, $D_2O$) δ 178.2 (C), 143.1 (C), 135.6 (C), 128.1 (CH), 125.2 (CH), 73.6 (C), 72.8 (CH), 70.6 (CH), and 41.5 ($CH_2$) ppm. IR (KBr): 3437 (O-H) and 1717 (C=O) $cm^{-1}$. MS (ESI) *m*/*z* (%) 213

(MH+). HRMS calcd for $C_9H_9O_4S$ (MH+): 213.0216; found, 213.0213.

*Example 34: (4R, 6R, 7S)-4,7-di(tert-butyldimethylsilyloxy)-2-methyl-4,5,6,7-tetrahydrobenzo[b]thiophen-4,6-carbolactone* ***(V-3).*** A solution of 2-methylallyl ketone **III-3** (0.47 g, 1.03 mmol) in dicloromethane (30 mL) was placed into a gas-washing bottle and cooled down to -78 ˚C in a dry ice bath. The mixture was then flushed with argon for 5 min, oxygen for 5 min and then with ozone for 12 min. The end of the reaction was indicated by the orange coloration of the aqueous KI (5%), which is located at the exit. Afterwards, the reaction mixture was flushed for 10 min with nitrogen to remove excess ozone. The gas-washing bottle was taken from the cooling bath and the content was slowly warming up to room temperature. The solvent was evaporated under reduced pressure to afford an oil which was used without further purification in the subsequent cyclization reaction. A small amount was purified by flash chromatography eluting with ethyl acetate-hexanes (5:95) and characterized as the ozonide intermediate. $[\alpha]_D^{20}$ +9.7˚ (*c*1.2, en $CHCl_3$). $^1H$ NMR (250 MHz, $CDCl_3$) δ 4.51 (br d, 1H, *J* = 5.8 Hz), 4.11 (br s, 1H), 2.69 (m, 1H), 2.66 (d, 1H, *J* = 12.0 Hz), 2.36 (dd, 1H, *J* = 13.0 and 8.8 Hz), 2.18 (dd, 1H, *J* = 12.0 and 5.8 Hz), 2.10 (dd, 1H, *J* = 13.0 and 5.5 Hz), 1.71 (s, 3H), 0.90 (s, 9H), 0.87 (s, 9H), 0.17 (s, 3H), 0.13 (s, 3H), 0.12 (s, 3H) and 0.08 (s, 3H) ppm. $^{13}C$ NMR (63 MHz, $CDCl_3$) δ 178.6 (C), 110.2 (C), 108.6 (C), 77.9 (CH), 73.1 (C), 66.8 (CH), 48.1 (CH), 39.1 ($CH_2$), 33.6 ($CH_2$), 25.7 ($2xC(CH_3)_3$), 18.3 ($C(CH_3)_3$), 18.2 ($C(CH_3)_3$), 14.7 ($CH_3$), -3.2 ($SiCH_3$), -3.3 ($SiCH_3$), -4.7 ($SiCH_3$) and -5.6 ($SiCH_3$) ppm. IR (KBr): 1807 (C=O) $cm^{-1}$. MS (CI) *m/z* (%) 473 (MH+).

The obtained residue from the ozonolysis reaction was dissolved in dry toluene (30 mL) and under argon. The resultant solution was treated with Lawesson's reagent (0.33 g, 0.81 mmol) and heated at 90 ˚C for 3 hours. After cooling to room temperature, the reaction mixture was poured into cool hexane. The obtained precipitate was filtered and washed with more hexane. The filtrate and the washings were treated with activated carbon and filtered. The solvent were concentrated and the obtained residue was purified by flash chromatography over silica gel eluting with diethyl ether-hexanes (10:90) to afford tiophene **V-3** (293 mg, 62% from **III-3**) as a colourless oil. $[\alpha]_D^{20}$ -94.2˚ (*c*1.0, in $CHCl_3$). $^1H$ NMR (500 MHz, $CDCl_3$) δ 6.70 (br q, 1H, *J* = 1.0 Hz), 4.81 (d, 1H, *J* = 3.5 Hz), 4.64 (dd, 1H, *J* = 6.0 and 3.5 Hz), 2.59 (d, 1H, *J* = 11.0 Hz), 2.48 (dd, 1H, *J* = 11.0 and 6.0 Hz), 2.44 (d, 3H, *J* = 1.0 Hz), 0.98 (s, 9H), 0.92 (s, 9H), 0.25 (s, 3H), 0.21 (s, 3H), 0.18 (s, 3H) and 0.14 (s, 3H) ppm. $^{13}C$ NMR (125 MHz, $CDCl_3$) δ 175.4 (C), 141.5 (C), 140.9 (C), 132.0 (C), 121.1 (CH), 77.4 (CH), 75.6 (C), 66.2 (CH), 37.9 ($CH_2$), 25.7 ($C(CH_3)_3$), 25.7 ($C(CH_3)_3$), 18.2 ($C(CH_3)_3$), 18.0 ($C(CH_3)_3$), 15.5 ($CH_3$), - 3.1 ($SiCH_3$), -3.2 ($SiCH_3$) and -4.7 ($2xSiCH_3$) ppm. IR (KBr): 1810 (C=O) $cm^{-1}$. MS (ESI) *m/z* (%) 455 (MH+). HRMS calcd for $C_{22}H_{39}O_4SSi_2$ (MH+): 455.2102; found, 455.2106.

*Example 35: (4R, 6R, 7S)-4,7-dihydroxy-2-methyl-4,5,6,7-tetrahydrobenzo[b]thiophen-4,6-carbolactone* ***(V-4).*** The experimental procedure used was the same as for compound **V-2** (example 32), but using **V-3** using as starting material (175 mg, 0.38 mmol) in 5.5 mL of THF and 1 mL of tetrabutylammonium fluoride. Purification by columm chromatography eluting with diethyl ether-hexanes (70:30). Yield = 61 mg (70%). Colourless oil. $[\alpha]_D^{20}$ -100.6˚ (*c*1.0, in MeOH). $^1H$ NMR (250 MHz, $CD_3OD$) δ 6.72 (s, 1H), 4.71 (m, 2H), 3.26 (s, 1H), 2.52-2.38 (m, 6H) ppm. $^{13}C$ NMR (63 MHz, $CD_3OD$) δ 178.4 (C), 142.7 (C), 142.2 (C), 133.9 (CH), 121.7 (CH), 79.2 (CH), 75.0 (C), 66.7 (CH), 38.5 ($CH_2$) and 15.4 ($CH_3$) ppm. IR (NaCl): 3444 (O-H) and 1770 (C=O) $cm^{-1}$. MS (ESI) *m/z* (%) 249 (MNa+). HRMS calcd for $C_{10}H_{10}O_4SNa$ (MNa+): 249.0198; found, 249.0192.

*Example 36: (4R, 6R, 7S)-4,6,7-trihydroxy-2-methyl-4,5,6,7-tetrahydrobenzo[b]thiophen-4-carboxylic acid* ***(Ic-2).*** The experimental procedure used was the same as for compound **Ic-1** (example 33), but using lactone **V-4** as starting material (58 mg, 0.26 mmol) in 1.3 mL of THF and 0.64 mL of LiOH (aq.). Yield = 50 mg (79%). Beige solid. Mp: 92-95 ˚C. $[\alpha]_D^{20}$ 18.9˚ (*c*1.5, in $H_2O$). $^1H$ NMR (250 MHz, $D_2O$) δ 6.39 (s, 1H), 4.52 (d, 1H, *J* = 8.0 Hz), 3.90 (ddd, 1H, *J* = 11.5, 8.0 and 3.5 Hz), 2.26 (m, 4H) and 2.07 (dd, 1H, *J* = 13.5 and 3.5 Hz) ppm. $^{13}C$ NMR (63 MHz, $D_2O$) δ 178.4 (C), 142.5 (C), 140.0 (C), 135.5 (C), 122.7 (CH), 73.6 (C), 72.4 (CH), 70.4 (CH), 41.1 ($CH_2$) and 14.9 ($CH_3$) ppm. IR (KBr): 3434 (O-H) and 1729 (C=O) $cm^{-1}$. MS (ESI) *m/z* (%) 267 (MNa+). HRMS calcd for $C_{10}H_{12}O_5SNa$ (MNa+): 267.0298; found, 267.0301.

*Example 37: (4R, 6R, 7S)-4,7-di(tert-butyldimethylsilyloxy)-2-iodo-4,5,6,7-tetrahydrobenzo[b]thiophen-4,6-carbolactone* ***(VII).*** A stirred solution of thiophene **V-1** (475 mg, 1.08 mmol) in dry dichloromethane (10 mL) and under argon was treated with glacial acetic acid (0.37 mL, 6.48 mmol) and *N*-iodosuccinimide (364 mg, 1.62 mmol). The reaction mixture was stirred at room temperature for 24 h. The solvents were removed under reduced pressure and the obtained residue was portioned in diethyl ether and 10% sodium tiosulfate. The aqueous layer was separated and the organic layer was washed with aqueous sodium bicarbonate and brine. The organic extract was dried (anh. $Na_2SO_4$),

filtered and concentrated to afford iodide **VII** (569 mg, 93%) as white solid. Mp: 52-55 °C. $[\alpha]_D^{20}$ -44.9° (*c*1.0, in $CHCl_3$). $^1H$ NMR (250 MHz, $CDCl_3$) δ 7.17 (s, 1H), 4.82 (d, 1H, *J* = 3.3 Hz), 4.65 (dd, 1H, *J* = 5.8 and 3.3 Hz), 2.5 (d, 1H, *J* = 11.0 Hz), 2.46 (dd, 1H, *J* = 11.0 and 5.8 Hz), 0.98 (s, 9H), 0.91 (s, 9H), 0.25 (s, 3H), 0.21 (s, 3H), 0.18 (s, 3H) and 0.14 (s, 3H) ppm. $^{13}C$ NMR (63 MHz, $CDCl_3$) δ 174.8 (C), 143.3 (C), 140.6 (C), 132.7 (CH), 77.2 (CH), 75.2 (C), 74.5 (C), 65.9 (CH), 37.6 ($CH_2$), 25.6 (2x$C(CH_3)_3$), 18.2 ($C(CH_3)_3$), 18.0 ($C(CH_3)_3$), -3.1 ($SiCH_3$), -3.2 ($SiCH_3$), -4.7 ($SiCH_3$) and -4.7 ($SiCH_3$) ppm. IR (KBr): 1801 (C=O) $cm^{-1}$. MS (ESI) *m/z* (%) 567 ($MH^+$). HRMS calcd for $C_{21}H_{36}O_4SSi_2I$ ($MH^+$): 567.0986; found, 567.0919.

<u>Example 38</u>: *(4R, 6R, 7S)-4,7-di(tert-butyldimethylsilyloxy)-2-vinyl-4,5,6,7-tetrahydrobenzo[b]thiophen-4,6-carbolactone* ***(V-5).*** A Shlenck tube was charged with iodide **VII** (150 mg, 0.26 mmol), $Pd(PPh_3)_4$ (31 mg, 0.03 mmol) and dry dioxane (3 mL). Anhydrous $K_2CO_3$ (0.72 mL, 0.79 mmol, 1.1 M) and vinyl-4,4,5,5-tetramethyl-1,3,2-dioxaborolane (61 μL, 0.40 mmol) was then added and the resultant solution was deoxygenated and heated at 100 °C for 2 h. After cooling to room temperature, the reaction mixture was filtered through a plug of Celite and the precipitate was washed with hexane. The filtrate and the washings were concentrated and the obtained residue was purified by columm chromatography over silica gel, previously neutralized with triethylamine-hexanes (5:95), using diethyl ether-hexanes (5:95) as eluent. It was obtained 83 mg oftiophene V-5 (69%) as colourless oil. $[\alpha]_D^{20}$ +59.4° (*c*1.1, in $CHCl_3$). $^1H$ NMR (250 MHz, $CDCl_3$) δ 6.90 (s, 1H, ArH), 6.73 (dd, 1H, *J* = 17.3 and 10.8 Hz), 5.53 (d, 1H, *J* = 17.3 Hz), 5.15 (d, 1H, *J* = 10.8 Hz), 4.83 (d, 1H, *J* = 3.3 Hz), 4.66 (dd, 1H, *J* = 5.5 and 3.3 Hz), 2.59 (d, 1H, *J* = 11.0 Hz), 2.50 (dd, 1H, *J* = 11.0 and 5.8 Hz), 0.98 (s, 9H), 0.93 (s, 9H), 0.26 (s, 3H), 0.23 (s, 3H), 0.20 (s, 3H) and 0.15 (s, 3H) ppm. $^{13}C$ NMR (63 MHz, $CDCl_3$) δ 175.2 (C), 143.6 (C), 141.9 (C), 133.3 (C), 129.7 (CH), 121.5 (CH), 114.2 ($CH_2$), 77.2 (CH), 75.4 (C), 66.3 (CH), 37.7 ($CH_2$), 25.6 (2x$C(CH_3)_3$), 18.2 ($C(CH_3)_3$), 18.0 ($C(CH_3)_3$), -3.1 ($SiCH_3$), -3.2 ($SiCH_3$) and -4.7 (2x$SiCH_3$) ppm. IR (film): 1801 (C=O) $cm^{-1}$. MS (CI) *mlz* (%) 467 ($MH^+$). HRMS calcd for $C_{23}H_{39}O_4SSi_2$ ($MH^+$): 467.2108; found, 467.2102.

<u>Example 39</u>: *(4R, 6R, 7S)-4,7-dihydroxy-2-vinyl-4,5,6,7-tetrahydrobenzo[b]thiophen-4,6-carbolactone* ***(V-6).*** The experimental procedure used was the same as for compound V-2 (example 32), but using silyl ether **V-5** as starting material (50 mg, 0.11 mmol) in 1.5 mL of THF and 0.3 mL of TBAF. Purification by columm chromatography eluting with diethyl ether-hexanes (70:30). Yield = 17 mg (68%). Colourless oil. $[\alpha]_D^{20}$ -50.9° (*c*1.1, in MeOH). $^1H$ NMR (250 MHz, $CD_3OD$) δ 6.94 (s, 1H), 6.76 (ddd, 1H, *J* = 17.5, 11.0 and 0.5 Hz), 5.51 (d, 1H, *J* = 17.5 Hz), 5.11 (dd, 1H, *J* = 11.0 Hz), 4.74 (m, 2H) and 2.45 (m, 2H) ppm. $^{13}C$ NMR (63 MHz, $CD_3OD$) δ 178.2 (C), 145.4 (C), 142.7 (C), 135.3 (C), 131.1 (CH), 122.1 (CH), 114.5 ($CH_2$), 79.0 (CH), 74.8 (C), 66.7 (CH) and 38.4 ($CH_2$) ppm. MS (CI) *mlz* (%) 239 ($MH^+$). HRMS calcd for $C_{11}H_{11}O_4S$ ($MH^+$): 239.0378; found, 239.0378.

<u>Example 40</u>: *(4R, 6R, 7S)-4,6,7-trihydroxy-2-vinyl-4,5,6,7-tetrahydrobenzo[b]thiophen-4-carboxylic acid* ***(Ic-3).*** The experimental procedure used was the same as for compound **Ic-1** (example 33), but using lactone **V-6** as starting material (30 mg, 0.13 mmol) in 0.7 mL of THF and 0.3 mL of LiOH (aq.). Yield = 32 mg (99%). Beige solid. $^1H$ NMR (250 MHz, $CDCl_3$) δ 6.81-6.70 (m, 2H), 5.49 (d, 1H, *J* = 17.5 Hz), 5.46 (d, 1H, *J* = 10.8 Hz), 4.53 (d, 1H, *J* = 8.0 Hz), 4.03 (m, 1H), 2.39 (dd, 1H, *J* = 13.3 and 12.0 Hz) and 2.16 (dd, 1H, *J* = 13.3 and 3.5 Hz) ppm. $^{13}C$ NMR (63 MHz, $CDCl_3$) δ 177.4 (C), 144.6 (C), 143.4 (C), 137.8 (C), 131.4 (CH), 125.1 (CH), 113.5 ($CH_2$), 74.1 (CH), 74.0 (C), 71.6 (CH) and 42.7 ($CH_2$) ppm. IR (KBr): 3400 (O-H) and 1718 (C=O) $cm^{-1}$. MS (ESI) *mlz* (%) 279 ($MNa^+$). HRMS calcd for $C_{11}H_{12}O_5SNa$ ($MNa^+$): 279.0298; found, 279.0290.

<u>Example 41</u>: *(4R, 6R, 7S)-4,7-di(tert-butyldimethylsilyloxy)-2-[(E)-prop-1-enyl)]-4,5,6,7-tetrahydrobenzo[b]thiophen-4,6-carbolactone* ***(V-8).*** A Shlenck tube was charged with iodide **VII** (100 mg, 0.18 mmol), $Pd(PPh_3)_4$ (20 mg, 0.02 mmol) and dry dioxane (1.8 mL). Anhydrous $K_2CO_3$ (0.48 mL, 0.53 mmol, 1.1 M) and (*E*)-prop-1-enylboronic acid (23 mg, 0.26 mmol) was then added and the resultant solution was deoxygenated and heated at 100 °C for 2 h. After cooling to room temperature, the reaction mixture was filtered through a plug of Celite and the precipitate was washed with hexane. The filtrate and the washings were concentrated and the obtained residue was purified by columm chromatography over silica gel, previously neutralized with triethylamine-hexanes (5:95), using diethyl ether-hexanes (5:95) as eluent. It was obtained 78 mg of tiophene **V-8** (92%) as colourless oil. $[\alpha]_D^{20}$ -61.9° (*c*1.1, in $CHCl_3$). $^1H$ NMR (250 MHz, $CDCl_3$) δ 6.71 (s, 1H), 6.38 (d, 1H, *J* = 15.5 Hz), 5.97 (m, 1H), 4.74 (d, 1H, *J* = 3.0 Hz), 4.57 (dd, 1H, *J* = 6.0 and 3.0 Hz), 2.52 (d, 1H, *J* = 10.8 Hz), 2.42 (dd, 1H, *J* = 10.8 and 6.0 Hz), 1.76 (d, 3H, *J* = 6.5 Hz), 0.91 (s, 9H), 0.85 (s, 9H), 0.19 (s, 3H), 0.15 (s, 3H), 0.12 (s, 3H) and 0.07 (s, 3H) ppm. $^{13}C$ NMR (63 MHz, $CDCl_3$) δ 174.9 (C), 143.4 (C), 141.5 (C), 131.6 (C), 126.5 (CH), 123.9 (CH), 119.4 (CH), 76.2 (CH), 75.2 (C), 66.0 (CH), 37.5 ($CH_2$), 25.4 (2x$C(CH_3)_3$), 18.0 ($CH_3$), 17.8 (2x$C(CH_3)_3$), -3.3 ($SiCH_3$), -3.5 ($SiCH_3$) and -4.9 (2x$SiCH_3$) ppm. IR (film): 1801 (C=O) $cm^{-1}$. MS (CI)

*m*/*z* (%) 481 ($MH^+$). HRMS calcd for $C_{24}H_{41}O_4SSi_2$ ($MH^+$): 481.2264; found, 481.2267.

*Example 42: (4R, 6R, 7S)-4,7-dihydroxy-2-[(E)-prop-1-enyl]-4,5,6,7-tetrahydrobenzo[b]thiophen-4,6-carbolactone* ***(V-8)*****.** The experimental procedure used was the same as for compound **V-2** (example 32), but using silyl ether **V-7** as starting material (85 mg, 0.18 mmol) in 2.5 mL of THF and 0.44 mL of TBAF. Purification by columm chromatography using diethyl ether-hexano (70:30). Yield = 39 mg (87%). Mp: 150-154 °C. $[\alpha]_D^{20}$ -45.6° (c1.1, in $CH_3OH$). $^1H$ NMR (250 MHz, $CDCl_3$) δ 6.86 (br s, 1H), 6.51 (dq, 1H, *J* = 15.5 and 1.5 Hz), 6.09 (m, 1H), 4.81-4.75 (m, 2H), 2.49 (m, 2H) and 1.83 (dd, 3H, *J* = 6.5 and 1.5 Hz) ppm. $^{13}C$ NMR (63 MHz, $CDCl_3$) δ 178.3 (C), 145.5 (C), 142.5 (C), 133.7 (C), 127.7 (CH), 125.4 (CH), 120.3 (CH), 79.1 (CH), 74.8 (C), 66.7 (CH), 38.4 ($CH_2$) and 18.4 ($CH_3$) ppm. IR (KBr): 3305 (O-H) and 1783 and 1753 (C=O) $cm^{-1}$. MS (CI) *m*/*z* (%) 253 ($MH^+$). HRMS calcd for $C_{12}H_{13}O_4S$ ($MH^+$): 253.0534; found, 253.0535.

*Example 43: (4R, 6R, 7S)-4, 6, 7-trihydroxy-2-[(E)-prop-1-enyl]-4,5,6,7-tetrahydrobenzo[b]thiophen-4-carboxylic acid* ***(Ic-4).*** The experimental procedure used was the same as for compound **Ic-1** (example 33), but using lactone **V-8** as starting material (30 mg, 0.12 mmol) in 0.6 mL of THF and 0.29 mL of LiOH (aq.). Yield = 31 mg (96%). White solid. $[\alpha]_D^{20}$ -12.4° (*c*1.2, in $CH_3OH$). $^1H$ NMR (250 MHz, $CD_3OD$) δ 6.61 (s, 1H), 6.43 (d, 1H, *J* = 15.5 Hz), 6.00 (m, 1H), 4.5 (d, 1H, *J* = 7.0 Hz), 4.02 (m, 1H), 2.38 (m, 1H), 2.18 (m, 1H) and 1.80 (d, 1H, *J* = 6.3 Hz) ppm. $^{13}C$ NMR (63 MHz, $CD_3OD$) δ 144.5 (C), 141.2 (C), 138.4 (C), 126.5 (C), 125.8 (CH), 123.3 (CH), 73.9 (C), 73.5 (C), 71.9 (CH), 42.0 ($CH_2$) and 18.4 ($CH_3$) ppm. IR (KBr): 3399 (O-H) and 1720 (C=O) $cm^{-1}$. MS (ESI) *m*/*z* (%) 293 ($MNa^+$). HRMS calcd for $C_{12}H_{14}O_5SNa$ ($MH^+$): 293.0454; found, 293.0453.

*Example 44: (4R, 6R, 7S)-4,7-di(tert-butyldimethylsilyloxy)-2-(2-methyl)vinyl-4,5,6,7-tetrahydrobenzo[b]thiophen-4,6-carbolactone* ***(V-9).*** A Shlenck tube was charged with iodide **VII** (150 mg, 0.26 mmol), $Pd(PPh_3)_4$ (31 mg, 0.03 mmol) and dry dioxane (2.6 mL). Anhydrous $K_2CO_3$ (0.7 mL, 0.79 mmol, 1.1 M) and 4,4,5,5-tetramethyl-2-(2-methyl)vinyl-1,3,2-dioxaborolane (75 μL, 0.40 mmol) was then added and the resultant solution was deoxygenated and heated at 100 °C for 1.5 h. After cooling to room temperature, the reaction mixture was filtered through a plug of Celite and the precipitate was washed with hexane. The filtrate and the washings were concentrated and the obtained residue was purified by columm chromatography over silica gel, previously neutralized with triethylamine-hexanes (5:95), using diethyl ether-hexanes (10:90) as eluent. It was obtained 80 mg oftiophene **V-9** (63%) as colourless oil. $[\alpha]_D^{20}$ -53.7° (*c*1.5, in $CHCl_3$). $^1H$ NMR (300 MHz, $CDCl_3$) δ 6.96 (s, 1H), 5.33 (s, 1H), 4.96 (s, 1H), 4.83 (d, 1H, *J* = 3.3 Hz), 4.66 (dd, 1H, *J* = 6.0 and 3.3 Hz), 2.59 (d, 1H, *J* = 10.8 Hz), 2.51 (dd, 1H, *J* = 10.8 and 6.0 Hz), 2.10 (s, 3H), 0.99 (s, 9H), 0.93 (s, 9H), 0.26 (s, 3H), 0.23 (s, 3H), 0.20 (s, 3H) and 0.15 (s, 3H) ppm. $^{13}C$ NMR (75 MHz, $CDCl_3$) δ 175.2 (C), 146.3 (C), 141.8 (C), 136.9 (C), 133.2 (C), 119.4 ($CH_2$), 112.0 (CH), 77.3 (CH), 75.4 (C), 66.3 (CH), 37.8 ($CH_2$), 25.7 ($C(CH_3)_3$), 25.6 ($C(CH_3)_3$), 21.5 ($CH_3$), 18.2 ($C(CH_3)_3$), 18.0 ($C(CH_3)_3$), -3.0 ($SiCH_3$), -3.2 ($SiCH_3$), -4.6 ($SiCH_3$) and -4.7 ($SiCH_3$) ppm. IR (film): 1803 (C=O) $cm^{-1}$. MS (ESI) *m*/*z* (%) 447 ($MNa^+$-$^t$Bu).

*Example 45: (4R, 6R, 7S)-4,7-dihydroxy-2-(2-methyl)vinyl-4,5,6,7-tetrahydrobenzo[b]thiophen-4,6-carbolactone* ***(V-10).*** The experimental procedure used was the same as for compound **V-2** (example 32), but using silyl ether **V-9** as starting material (110 mg, 0.23 mmol) in 3.2 mL of THF and 0.5 mL of TBAF. Purification by columm chromatography over silica gel eluting with diethyl ether-hexanes [1) 75:25, 2) 100:0]. Yield = 41 mg (71%). White foam. $[\alpha]_D^{20}$ -37.0° (*c*1.4, in $CH_3OH$). $^1H$ NMR (250 MHz, $CD_3OD$) δ 7.01 (br s, 1H), 5.31 (br s, 1H), 4.93 (br s, 1H), 4.75 (m, 2H), 2.47 (m, 2H) and 2.07 (br s, 3H) ppm. $^{13}C$ NMR (63 MHz, $CD_3OD$) δ 178.2 (C), 147.9 (C), 142.6 (C), 138.6 (C), 135.1 (CH), 120.0 (CH), 112.4 ($CH_2$), 79.0 (CH), 74.8 (C), 66.6 (CH), 38.4 ($CH_2$) and 21.7 ($CH_3$) ppm. IR (KBr): 3458 (O-H) and 1784 (C=O) $cm^{-1}$.

*Example 46: (4R, 6R, 7S)-4,6,7-trihydroxy-2-(2-methyl)vinyl-4,5,6,7-tetrahydrobenzo[b]thiophen-4-carboxylic acid* ***(Ic-5).*** The experimental procedure used was the same as for compound **Ic-1** (example 33), but using lactone **V-10** as starting material (40 mg, 0.16 mmol) in 1.5 mL of THF and 0.8 mL ofLiOH (aq.). Purification by HPLC using a semipreparative columm Merck ZORBAX ODS (212x25 mm), eluting with (20:80) acetonitrile-water and at a flow of 7 mL $min^{-1}$. Yield = 42 mg (98%). Beige solid. $[\alpha]_D^{20}$ -1.9 (*c*1.4, in $CH_3OH$). $^1H$ NMR (250 MHz, $D_2O$) δ 6.72 (br s, 1H), 5.29 (br s, 1H), 4.92 (br s, 1H), 4.58 (d, 1H, *J* = 8.0 Hz), 3.95 (m, 1H), 2.29 (dd, 1H, *J* = 12.0 and 13.3 Hz), 2.13 (dd, 1H, *J* = 3.8 and 13.3 Hz) and 1.97 (br s, 3H) ppm. $^{13}C$ NMR (100 MHz, $D_2O$) δ 177.9 (C), 146.8 (C), 141.4 (C), 137.6 (C), 136.0 (C), 121.5 (CH), 111.9 ($CH_2$), 73.4 (C), 72.2 (CH), 70.1 (CH), 41.0 ($CH_2$) and 20.6 ($CH_3$) ppm. IR (KBr): 3438

(O-H) and 1729 (C=O) $cm^{-1}$. MS (ESI) *mlz* (%) 269 ($M-H^+$). HRMS calcd for $C_{12}H_{13}O_5S$ ($M-H^+$): 269.0478; found, 269.0469.

*Example 47: (4R, 6R, 7S)-4,7-di(tert-butyldimethylsilyloxy)-2-[(E)-2-cyclopropyl]vinyl-4,5,6,7-tetrahydrobenzo[b]thiophen-4,6-carbolactone **(V-11).*** A Shlenck tube was charged with iodide **VII** (100 mg, 0.18 mmol), $Pd(PPh_3)_4$ (20 mg, 0.02 mmol) and dry dioxane (1.8 mL). Anhydrous $K_2CO_3$ (0.5 mL, 0.53 mmol, 1.1 M) and (*E*)-2-(2-cyclopropyl) vinyl-4,4,5,5-tetramethyl-1,3,2-dioxaborolane (55 μL, 0.26 mmol) was then added and the resultant solution was deoxygenated and heated at 95 °C for 4 h. After cooling to room temperature, the reaction mixture was filtered through a plug of Celite and the precipitate was washed with hexane. The filtrate and the washings were concentrated and the obtained residue was was purified by columm chromatography, previously neutralized with 5% triethylamine-hexanes, eluting with diethyl ether-hexanes (5:95) to yield tiophene **IXf** (80 mg, 89%) as a light yellow oil. $[\alpha]_D^{20}$ -42.6° (*c*1.3, in $CHCl_3$). $^1H$ NMR (250 MHz, $CDCl_3$) δ 6.75 (s, 1H), 6.51 (d, 1H, *J* = 15.5 Hz), 5.55 (dd, 1H, *J* = 15.5 and 8.8 Hz), 4.80 (d, 1H, *J* = 3.3 Hz), 4.64 (dd, 1H, *J* = 5.5 and 3.3 Hz), 2.58 (d, 1H, *J* = 10.8 Hz), 2.48 (dd, 1H, *J* = 10.8 and 5.5 Hz), 1.57-1.40 (m, 1H), 0.98 (s, 9H), 0.92 (s, 9H), 0.82-0.76 (m, 2H), 0.54-0.46 (m, 2H), 0.25 (s, 3H), 0.21 (s, 3H), 0.18 (s, 3H) and 0.14 (s, 3H) ppm. $^{13}C$ NMR (63 MHz, $CDCl_3$) δ 175.2 (C), 143.7 (C), 141.7 (C), 135.8 (CH), 131.4 (C), 120.6 (CH), 119.3 (CH), 77.3 (CH), 75.4 (C), 66.3 (CH), 37.8 ($CH_2$), 25.7 (2x$C(CH_3)_3$), 18.2 ($\mathit{C}(CH_3)_3$), 18.0 ($\mathit{C}(CH_3)_3$), 14.3 (CH), 7.4 (2x$CH_2$), -3.0 ($SiCH_3$), -3.2 ($SiCH_3$), -4.6 ($SiCH_3$) and -4.7 ($SiCH_3$) ppm. IR (film): 1801 (C=O) $cm^{-1}$. MS (CI) *mlz* (%) 507 ($MH^+$). HRMS calcd for $C_{26}H_{43}O_4SSi_2$ ($MH^+$): 507.2421; found, 507.2420.

*Example 48: (4R, 6R, 7S)-2-[(E)-2-cyclopropil]vinyl-4,7-dihydroxy-4,5,6,7-tetrahydrobenzo[b]thiophen-4,6-carbolactone **(V-12).*** The experimental procedure used was the same as for compound **V-2** (example 32), but using silyl ether **V-11** as starting material (70 mg, 0.14 mmol) in 2 mL of THF and 0.36 mL of TBAF. Purification by columm chromatography over silica gel eluting with diethyl ether-hexanes (70:30). Yield = 35 mg (92%). White solid. Mp: 154-156 °C. $[\alpha]_D^{20}$ -27.0° (*c*1.05, in $CH_3OH$). $^1H$ NMR (250 MHz, $CD_3OD$) δ 6.83 (s, 1H), 6.56 (d, 1H, *J* = 15.5 Hz), 5.61 (dd, 1H, *J* = 15.5 and 9.0 Hz), 4.80-4.74 (m, 2H), 2.55-2.43 (m, 2H), 1.59-1.45 (m, 1H), 0.85-0.77 (m, 2H) and 0.51-0.45 (m, 2H) ppm. $^{13}C$ NMR (63 MHz, $CDCl_3$) δ 178.3 (C), 145.7 (C), 142.5 (C), 137.1 (CH), 133.2 (C), 121.7 (CH), 119.8 (CH), 79.1 (CH), 74.9 (C), 66.7 (CH), 38.4 ($CH_2$), 15.2 (CH) and 7.8 (2x$CH_2$) ppm. IR (KBr): 3507 (O-H) and 1788 and 1759 (C=O) $cm^{-1}$. MS (CI) *mlz* (%) 279 ($MH^+$). HRMS calcd for $C_{14}H_{15}O_4S$ ($MH^+$): 279.0691; found, 279.0691.

*Example 49: (4R, 6R, 7S)-2-[(E)-2-cyclopropyl]vinyl-4,6,7-trihydroxy-4,5,6,7-tetrahydrobenzo[b]thiophen-4-carboxylic acid **(Ic-6).*** The experimental procedure used was the same as for compound **Ic-1** (example 33), but using lactone **V-12** as starting material (28 mg, 0.10 mmol) in 0.9 mL of THF and 0.5 mL ofLiOH (aq.). Purification by HPLC using a semipreparative columm Merck ZORBAX ODS (212x25 mm), eluting with (30:70) acetonitrile-water and at a flow of 7 mL $min^{-1}$. Yield = 29 mg (97%). Mp: 103-105 °C. $[\alpha]_D^{20}$ -4.4 (*c*1.2, in $CH_3OH$). $^1H$ NMR (500 MHz, $D_2O$) δ 6.58 (s, 1H), 6.54 (d, 1H, *J* = 16.0 Hz), 5.66 (dd, 1H, *J* = 16.0 and 9.5 Hz), 4.62 (d, 1H, *J* = 8.0 Hz), 4.00 (ddd, 1H, *J* = 8.0, 4.0 and 12.0 Hz), 2.34 (dd, 1H, *J* = 13.5 and 12.0 Hz), 2.18 (dd, 1H, *J* = 13.5 and 4.0 Hz), 1.53-1.46 (m, 1H), 0.77 (m, 2H) and 0.45 (m, 2H) ppm. $^{13}C$ NMR (125 MHz, $D_2O$) δ 180.7 (C), 147.3 (C), 142.6 (C), 139.8 (CH), 138.6 (C), 123.9 (CH), 123.0 (CH), 76.2 (C), 75.1 (CH), 73.0 (CH), 43.8 ($CH_2$), 16.7 (CH) and 9.8 (2x$CH_2$) ppm. IR (KBr): 3390 (O-H) and 1722 (C=O) $cm^{-1}$. MS (ESI) *m/z* (%) 319 ($MNa^+$). HRMS calcd for $C_{14}H_{16}O_5SNa$ ($MNa^+$): 319.0611; found, 319.0618.

*Example 50: (4R, 6R, 7S)-4,7-di(tert-butyldimethylsilyloxy)-2-phenyl-4,5,6,7-tetrahydrobenzo[b]thiophen-4,6-carbolactone **(V-13).*** A Shlenck tube was charged with iodide **VII** (130 mg, 0.23 mmol), $Pd(PPh_3)_4$ (26 mg, 0.02 mmol) and dry dioxane (2.5 mL). Anhydrous $K_2CO_3$ (0.63 mL, 0.69 mmol, 1.1 M) and phenylboronic acid (35 mg, 0.28 mmol) was then added and the resultant solution was deoxygenated and heated at 100 °C for 2 h. After cooling to room temperature, the reaction mixture was filtered through a plug of Celite and the precipitate was washed with hexane. The filtrate and the washings were concentrated and the obtained residue was purified by columm chromatography over silica gel, previously neutralized with triethylamine-hexanes (5:95), using diethyl ether-hexanes (5:95) as eluent. It was obtained 89 mg (75%) of thiophene **V-13** as white foam. Mp: 58-61 °C. $[\alpha]_D^{20}$ -47.8° (*c*1.1, in $CHCl_3$). $^1H$ NMR (300 MHz, $CDCl_3$) δ 7.58 (d, 2H, *J* = 6.3 Hz), 7.40 (m, 2H), 7.32 (m, 1H), 7.26 (s, 1H), 4.93 (d, 1H, *J* = 2.4 Hz), 4.72 (dd, 1H, *J* = 2.4 and 4.5 Hz), 2.67 (d, 1H, *J* = 8.4 Hz), 2.57 (dd, 1H, *J* = 8.4 and 4.5 Hz), 1.04 (s, 9H), 0.97 (s, 9H), 0.32 (s, 3H), 0.28 (s, 3H), 0.25 (s, 3H) and 0.21 (s, 3H) ppm. $^{13}C$ NMR (75 MHz, $CDCl_3$) δ 175.1 (C), 145.0 (C), 142.5 (C), 133.9 (2xC), 128.8 (2xCH), 127.9 (CH), 125.9 (2xCH), 118.9 (CH), 77.2 (CH), 75.5 (C), 66.3 (CH), 37.8 ($CH_2$), 25.7 (2xC$(\mathit{C}H_3)_3$), 18.2 ($\mathit{C}(CH_3)_3$), 18.0 ($\mathit{C}(CH_3)_3$), -3.0 ($CH_3$), -3.2 ($CH_3$) and -4.6 (2x$CH_3$) ppm. IR (KBr): 1803 (C=O) $cm^{-1}$. MS

(CI) *m/z* (%) 517 ($MH^+$). HRMS calcd for $C_{27}H_{41}O_4SSi_2$ ($MH^+$): 517.2264; found, 517.2245.

*Example 51: (4R, 6R, 7S)-4,6,7-trihydroxy-2-phenyl-4,5,6,7-tetrahydrobenzo[b]thiophen-4-carboxylic acid **(Ic-7)**.* To a stirred solution of the silyl ether **V-13** (81 mg, 0.16 mmol) in dry THF (2.2 mL), under argon at 0 °C, was added tetrabutylammonium fluoride (0.41 mL, 0.41 mmol, *ca* 1.0 M en THF). After stirring for 15 min at 0 °C the ice bath was removed and the reaction mixture was stirred for 1 h. The solvent was concentrated and the obtained residue was disolved in ethyl acetate and water. The mixture was acidified with dilute HCl until pH 1. The organic layer was separated and the aqueous phase was extracted with ethyl acetate (x2). The combined organic extracts were dried (anh. $Na_2SO_4$), filtered and concentrated under reduced pressure. The obtained residue was purified by flash chromatography over silica gel eluting with diethyl ether-hexanes (70:30) to yield (4*R*, 6*R*, 7*S*)-4,7-dihydroxy-2-phenyl-4,5,6,7-tetrahydrobenzo[*b*]thiophen-4,6-carbolactone (**V-14,** 40 mg, 89%) as an colourless oil. Carbolactone **V-14** (40 mg, 0.14 mmol) was hydrolized as for compound **Ic-1** using 0.55 mL of LiOH (aq.) and 0.7 mL of THF. Yield = 38 mg (89%). White solid. Mp: 168-173 °C. $[\alpha]_D^{20}$ -20.2° (*c*1.0, in $CH_3OH$). $^1H$ NMR (400 MHz, $CD_3OD$) δ 7.62 (m, 2H), 7.42 (m, 2H), 7.32 (m, 1H), 7.17 (s, 1H), 4.66 (d, 1H, *J* = 8.0 Hz), 4.13 (m, 1H), 2.50 (dd, 1H, *J* = 13.6 and 11.6 Hz), and 2.27 (dd, 1H, *J* = 13.6 and 3.2 Hz) ppm. $^{13}C$ NMR (75 MHz, $CDCl_3$) δ 177.7 (C), 145.8 (C), 143.8 (C), 138.6 (C), 135.6 (C), 130.0 (2xCH), 128.8 (CH), 126.5 (2xCH), 122.0 (CH), 74.3 (C), 74.1 (CH), 71.7 (CH) and 42.6 ($CH_2$) ppm. IR (KBr): 3419 (O-H) and 1716 (C=O) $cm^{-1}$. MS (ESI) *m/z* (%) 329 ($MNa^+$). HRMS calcd for $C_{15}H_{14}O_5SNa$ ($MNa^+$): 329.0454; found, 329.0445.

*Example 52: (4R, 6R, 7S)-4,7-di(tert-butyldimethylsilyloxy)-2-(2-cyclopropyl)ethyl-4,5,6,7-tetrahydrobenzo[b] thiophen-4,6-carbolactone **(V-15)**.* A suspension of thiophene **V-11** (78 mg, 0.15 mmol) and 10% palladium-on-carbon (16 mg) in methanol (1.5 mL) was stirred under hydrogen atmosphere at room temperature for 48 h. The mixture was filtered over Celite and the residue was washed with methanol. The filtrate and washings were evaporated to yield thiophene **V-15** (75 mg, 96%) as a colourless oil. $[\alpha]_D^{20}$ -68.0° (*c*1.1, in $CHCl_3$). $^1H$ NMR (250 MHz, $CDCl_3$) δ 6.73 (s, 1H), 4.81 (d, 1H, *J* = 3.3 Hz), 4.64 (dd, 1H, *J* = 5.8 and 3.3 Hz), 2.86 (t, 2H, *J* = 5.8 Hz), 2.59 (d, 1H, *J* = 11.0 Hz), 2.48 (dd, 1H, *J* = 11.0 and 6.0 Hz), 1.55 (m, 2H), 0.98 (s, 9H), 0.92 (s, 9H), 0.73 (m, 1H), 0.44 (m, 2H), 0.25 (s, 3H), 0.21 (s, 3H), 0.18 (s, 3H), 0.14 (s, 3H) and 0.07 (m, 2H) ppm. $^{13}C$ NMR (63 MHz, $CDCl_3$) δ 175.5 (C), 146.7 (C), 141.2 (C), 131.8 (C), 120.0 (CH), 77.4 (CH), 75.6 (C), 66.3 (CH), 37.9 ($CH_2$), 36.7 ($CH_2$), 30.4 ($CH_2$), 25.7 (2x$C(CH_3)_3$), 18.2 ($C(CH_3)_3$), 18.1 ($C(CH_3)_3$), 10.6 (CH), 4.5 (2x$CH_2$), -3.1 ($SiCH_3$), -3.2 ($SiCH_3$) and -4.7 (2x$SiCH_3$) ppm. IR (film): 1801 (C=O) $cm^{-1}$. MS (CI) *m/z* (%) 509 ($MH^+$). HRMS calcd for $C_{26}H_{45}O_4SSi_2$ ($MH^+$): 509.2577; found, 509.2566.

*Example 53: (4R, 6R, 7S)-2-(2-cyclopropyl)ethyl-4,7-dihydroxy-4,5,6,7-tetrahydrobenzo[b]thiophen-4,6-carbolactone **(V-16)**.* The experimental procedure used was the same as for compound **V-2** (example 32), but using silyl ether **V-15** as starting material (38 mg, 0.075 mmol) in 1.1 mL of THF and 0.19 mL of TBAF. Purification by columm chromatography using diethyl ether-hexanes (70:30) as eluent. Yield = 16.2 mg (77%). $[\alpha]_D^{20}$ -54.7° (*c*1.6, in MeOH). $^1H$ NMR (250 MHz, $CD_3OD$) δ 6.76 (s, 1H), 4.74 (dd, 1H, *J* = 3.3 and 1.0 Hz), 4.70 (d, 1H, *J* = 3.3 Hz), 2.84 (t, 2H, *J* = 7.5 Hz), 2.43 (m, 2H), 1.49 (q, 2H, *J* = 7.5 Hz), 0.70 (m, 1H), 0.39 (m, 2H) and 0.03 (m, 2H) ppm. $^{13}C$ NMR (63 MHz, $CD_3OD$) δ 178.4 (C), 148.4 (C), 142.0 (C), 133.7 (C), 120.7 (CH), 79.2 (CH), 75.0 (C), 66.7 (CH), 38.5 ($CH_2$), 38.2 ($CH_2$), 31.3 ($CH_2$), 11.4 (CH) and 5.0 (2x$CH_2$) ppm.

*Example 54: (4R, 6R, 7S)-2-(2-cyclopropyl)ethyl-4,6,7-trihydroxy-4,5,6,7-tetrahydrobenzo[b]thiophen-4-carboxylic acid **(Ic-8)**.* The experimental procedure used was the same as for compound **Ic-1** (example 33), but using lactone **V-16** as starting material (16 mg, 0.057 mmol) in 1 mL of THF and 0.17 mL of LiOH (aq.). Yield = 10.4 mg (61%). $[\alpha]_D^{20}$ -24.9° (*c*1.0, in MeOH). $^1H$ NMR (250 MHz, $D_2O$) δ 6.41 (s, 1H), 4.54 (d, 1H, *J* = 7.8 Hz), 3.91 (m, 1H), 2.74 (t, 2H, *J* = 7.5 Hz), 2.23 (dd, 1H, *J* = 13.5 and 12.3 Hz), 2.03 (dd, 1H, *J* = 13.5 and 3.5 Hz), 1.41 (q, 2H, *J* = 7.5 Hz ), 0.63 (m, 1H), 0.28 (m, 2H) and -0.07 (m, 2H) ppm. $^{13}C$ NMR (63 MHz, $D_2O$) δ 181.1 (C), 148.0 (C), 138.8 (C), 137.9 (C), 122.4 (CH), 74.6 (C), 72.6 (CH), 71.3 (CH), 41.6 ($CH_2$), 36.6 ($CH_2$), 30.3 ($CH_2$), 10.5 (CH) and 4.3 (2x$CH_2$) ppm. MS (ESI) *m/z* (%) 271 ($M-H^+$). HRMS calcd for $C_{12}H_{15}O_5S$ ($M-H^+$): 271.0635; found, 271.0625.

*Example 55: (4R, 6R, 7R)-1-benzyl-4,7-di(tert-butyldimethylsilyloxy)-4,5,6,7-tetrahydro-1H-indol-4,6-carbolactone **(V-17)**.* A solution of the obtained residue (100 mg) by ozonolysis of allyl derivative **III-1** in acetic acid (1 mL), was treated with benzylamine (27 μL, 0.25 mmol) and was stirred at room temperature for 30 min. The reaction mixture was diluted with diethyl ether and sodium bicarbonate (sat.). The organic layer was separated and the aqueous phase was extracted with diethyl ether (2x). All the combined organic extracts were dried (anh. $Na_2SO_4$), filtered and concentrated under reduced pressure. The obtained residue was purified by columm chromatography eluting with diethyl ether-hexanes (10:90) to give indol **V-17** (64 mg, 55%) as colourless oil. $[\alpha]_D^{20}$ -106.8° (*c*1.0, in $CHCl_3$). $^1$H NMR (250 MHz, $CDCl_3$) δ 7.11 (m, 3H, 3xArH), 6.80 (d, 2H, *J* = 7.5 Hz, 2xArH), 6.32 (d, 1H, *J* = 2.8 Hz, ArH), 6.04 (d, 1H, *J* = 2.8 Hz, ArH), 4.92 (d, 1H, *J* = 16.0 Hz, C*H*H), 4.84 (d, 1H, *J* = 16.0 Hz, CH*H*), 4.57 (d, 1H, *J* = 3.3 Hz, H-4), 4.48 (dd, 1H, *J* = 6.0 and 3.3 Hz, H-5), 2.42 (d, 1H, *J* = 10.8 Hz, H-$6_{eq}$), 2.31 (dd, 1H, *J* = 10.8 and 6.0 Hz, H-$6_{ax}$), 0.82 (s, 9H, $C(CH_3)_3$), 0.69 (s, 9H, $C(CH_3)_3$), 0.09 (s, 3H, $SiCH_3$), 0.00 (s, 3H, $SiCH_3$), -0.04 (s, 3H, $SiCH_3$) and -0.13 (s, 3H, $SiCH_3$) ppm. $^{13}$C NMR (63 MHz, $CDCl_3$) δ 176.1 (C), 137.2 (C), 128.8 (2xCH), 127.6 (CH), 126.5 (2xCH), 124.7 (C), 124.2 (C), 122.3 (CH), 103.6 (CH), 76.8 (CH), 74.2 (C), 64.4 (CH), 50.3 ($NCH_2$), 38.4 ($CH_2$), 25.7 ($C(CH_3)_3$), 25.6 ($C(CH_3)_3$), 18.2 ($C(CH_3)_3$), 17.9 ($C(CH_3)_3$), -2.9 ($SiCH_3$), -3.0 ($SiCH_3$), -4.1 ($SiCH_3$) and -4.6 ($SiCH_3$) ppm. IR (film): 1799 (C=O) $cm^{-1}$. MS (ESI) *m*/*z* (%) 514 ($MH^+$). HRMS calcd for $C_{28}H_{44}O_4Si_2N$ ($MH^+$): 514.2819; found, 514.2803.

<u>*Example 57*</u>*: (4R, 6R, 7R)-1-benzyl-4,7-dihydroxy-4,5,6,7-tetrahydro-1H-indol-4,6-carbolactone* ***(V-18).*** The experimental procedure used was the same as for compound **V-2** (example 32), but using silyl ether **V-17** as starting material (80 mg, 0.16 mmol) in 1.5 mL of THF and 0.41 mL of TBAF. Purification by columm chromatography using ethyl acetate-hexanes (60:40) as eluent. Yield = 40 mg (91%). $[\alpha]_D^{20}$ -91.6° (*c*1.0, in MeOH). $^1$H NMR (250 MHz, $CD_3OD$) δ 7.29 (m, 3H), 7.13 (m, 2H), 6.70 (d, 1H, *J* = 2.8 Hz), 6.18 (d, 1H, *J* = 2.8 Hz), 5.20 (d, 1H, *J* = 15.8 Hz), 5.13 (d, 1H, *J* = 15.8 Hz), 4.69 (m, 1H), 4.63 (br s, 1H), 4.54 (d, 1H, *J* = 3.3 Hz) and 2.46 (m, 2H) ppm. $^{13}$C NMR (63 MHz, $CD_3OD$) δ 179.5 (C), 139.2 (C), 129.8 (2xCH), 128.7 (CH), 128.3 (2xCH), 126.2 (C), 124.4 (C), 123.9 (CH), 103.59 (CH), 79.3 (CH), 73.8 (C), 64.4 (CH), 51.7 ($CH_2$) and 39.3 ($CH_2$) ppm. IR (film): 3395 (O-H) and 1784 (C=O) $cm^{-1}$. MS (ESI) *m*/*z* (%) 286 ($MH^+$). HRMS calcd for $C_{16}H_{16}O_4N$ ($MH^+$): 286.1069; found, 286.1074.

<u>*Example 58*</u>*: (4R, 6R, 7S)-4,6,7-trihydroxy-2-isopropyl-4,5,6,7-tetrahydrobenzo[b]thiophen-4-carboxylic acid* ***(Ic-8).*** A suspension of thiophene **Ic-5** (20 mg, 0.074 mmol) and 10% palladium-on-carbon (4 mg) in methanol (0.7 mL) was stirred under hydrogen atmosphere at room temperature for 48 h. The mixture was filtered over Celite and the residue was washed with methanol. The filtrate and washings were concentrated under reduced pressure. The obtainded residue was purified by HPLC using a semipreparative columm Merck ZORBAX ODS (212x25 mm), eluting with (20:80) acetonitrile-water and at a flow of 7 mL $min^{-1}$ to afford thiophene **Ic-8** (15 mg, 74%) as a white solid. $[\alpha]_D^{20}$ -6.4° (*c*1.0, in MeOH). Mp: 125-127 °C. $^1$H NMR (250 MHz, $D_2O$) δ 6.51 (s, 1H), 4.57 (d, 1H, *J* = 8.3 Hz), 3.94 (m, 1H), 3.01 (quint, 1H, *J* = 6.8 Hz), 2.30 (dd, 1H, *J* = 11.8 and 13.5 Hz), 2.14 (dd, 1H, *J* = 3.3 and 13.5 Hz) and 1.16 (d, 6H, *J* = 6.8 Hz) ppm. $^{13}$C NMR (63 MHz, $D_2O$) δ 177.9 (C), 156.2 (C), 139.6 (C), 134.8 (C), 119.5 (CH), 73.5 (C), 72.4 (CH), 70.3 (CH), 41.2 ($CH_2$), 30.1 (CH) and 24.2 ($CH_3$) and 24.1 ($CH_3$) ppm. IR (KBr): 3398 (O-H) and 1724 (C=O) $cm^{-1}$. MS (ESI) *m*/*z* (%) 271 ($M-H^+$). HRMS calcd for $C_{12}H_{15}O_5S$ ($M-H^+$): 271.0635; found, 271.0639.

<u>*Example 59*</u>*: (4R, 6R, 7S)-2-ethyl-4,6,7-trihydroxy-4,5,6,7-tetrahydrobenzo[b]thiophen-4-carboxylic acid* ***(Ic-9).*** The experimental procedure used was the same as for compound **Ic-8** using vinyl thiophene **Ic-3** (18 mg, 0.070 mmol) and 10% palladium-on-carbon (4 mg) in metanol (0.7 mL). Yield = 13.9 mg (77%). $[\alpha]_D^{20}$ -34.6° (*c*1.0, in MeOH). $^1$H NMR (250 MHz, $D_2O$) δ 6.40 (br s, 1H), 4.55 (d, 1H, *J* 7.3 Hz), 3.91 (m, 1H), 2.66 (q, 2H, *J* 7.3 Hz), 2.20 (m, 1H), 2.04 (m, 1H) and 1.12 (t, 3H, *J* 7.3 Hz) ppm. $^{13}$C NMR (63 MHz, $D_2O$) δ 180.8 (C), 149.6 (C), 138.4 (C), 137.6 (C), 121.2 (CH), 74.3 (C), 72.3 (CH), 71.0 (CH), 41.3 ($CH_2$), 23.4 ($CH_2$) and 15.4 ($CH_3$) ppm.

<u>*Example 60*</u>*: Ethyl (1R, 4S, 5R)-2-allyl-3-(benzo[b]thiophen-2-yl)methoxy-1,4,5-trihydroxycyclohex-2-en-1-carboxylate* ***(Ib-9).*** A solution of lactone **IV-10** (16 mg, 0.04 mmol) and sodium ethoxide (4 mg, 0.06 mmol) in ethanol (0.4 mL) was stirred at room temperature for 1 hora. Ethyl acetate and water was then added. The organic layer was separated and the aqueous phase was extracted with ethyl acetate (x2). All the combined organic extracts were dried (anh. $MgSO_4$), filtered and concentrated under reduced pressure. The obtained residue was purified by flash chromatography over silica gel eluting with ethyl acetate-hexanes (50:50). 9 mg (50%) of esther **Ib-9** were obtained as white solid. $^1$H NMR (400 MHz, $CDCl_3$) δ 7.78 (dd, 1H, *J* = 6.8 and 1.6 Hz), 7.70 (dd, 1H, *J* = 7.6 and 1.6 Hz), 7.32 (m, 2H), 7.23 (s, 1H), 5.72-5.62 (m, 1H), 5.27 (dd, 1H, *J* = 12.4 and 0.8 Hz), 5.11 (d, 1H, *J* = 12.4 Hz), 4.98 (dd, 1H, *J* = 17.2 and 2.0 Hz), 4.89 (dd, 1H, *J* = 10.0 and 1.6 Hz), 4.28 (d, 1H, *J* = 7.2 Hz), 4.17-4.00 (m, 3H), 3.79 (br s, 1H), 2.91 (dd, 1H, *J* = 14.8 and 6.8 Hz), 2.65 (ddd, 1H, *J* = 14.8, 6.4 and 1.2 Hz), 2.08 (dd, 1H, *J* = 13.2 and 11.6 Hz), 2.00 (dd, 1H, *J* = 13.2 and 4.0 Hz) and 1.12 (t, 3H, *J* = 6.8 Hz) ppm. $^{13}$C NMR (100 MHz, $CDCl_3$) δ 175.1 (C), 150.6 (C), 140.3 (C), 140.2 (C), 139.2 (C), 136.3 (CH), 124.5 (CH), 124.3 (CH), 123.7 (CH), 123.5 (CH), 122.3 (CH), 120.5 (C), 115.5 ($CH_2$), 75.4 (C), 72.0 (CH), 70.4 (CH), 67.0 ($CH_2$), 62.4 ($CH_2$), 39.6 ($CH_2$), 31.2 ($CH_2$) and 13.8 ($CH_3$) ppm.

<u>*Example 61*</u>*: Effect of compounds of formula* ***I*** *of the invention on the catalytic activity of the type II dehydroquinase from various sources.* The inhibitory potency of compounds **I** of the invention against type II dehydroquinases

from *Mycobacterium tuberculosis, Streptomyces coelicolor* and *Helicobacter pylori* were assayed as it is described in the following articles: 1) Sánchez-Sixto, C.; Prazeres, V. F. V.; Castedo, L.; Lamb, H.; Hawkins, A. R.; González-Bello, C. J. Med. Chem. 2005, 48, 4871; 2) Prazeres, V. F. V.; Sánchez-Sixto, C.; Castedo, L.; Lamb, H.; Hawkins, A. R.; Riboldi-Tunnicliffe; Coggins, J. R.; Lapthorn, A. J.; González-Bello, C. ChemMedChem 2007, 2, 194; and 3) Sánchez-Sixto, C.; Prazeres, V. F. V.; Castedo, L.; S. W. Suh, Lamb, H.; Hawkins, A. R.; Cañada, F. J.; Jiménez-Barbero, J.; González-Bello, C. ChemMedChem 2008, 3, 756. The inhibition data ($K_i$) are summarized in Table 1.

**Table 1.** Inhibition constants $K_i$ (μM) of compounds **I** against type **II** dehydroquinases from *Mycobacterium tuberculosis*, *Helicobacter pylori* and *Streptomyces coelicolor.*[a]

| Compound | | *M. tuberculosis* | *H. pylori* | *S. coelicolor* |
|---|---|---|---|---|
| 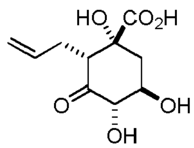 | **(2*R*)-Ia-1** | 0.76 | 9.4 | 10.2 |
| 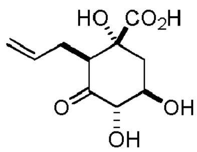 | **(2*S*)-Ia-1** | 1.85 | 21.5 | 6.7 |
| 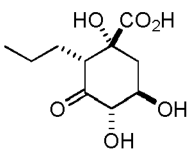 | **(2*R*)-Ia-2** | 1.70 | 20 | 76 |
| 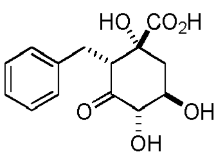 | **(2*R*)-Ia-3** | 0.10 | 1.40 | 1.55 |
| 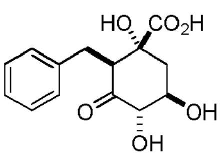 | **(2*S*)-Ia-3** | 0.09 | 1.18 | 1.05 |
| 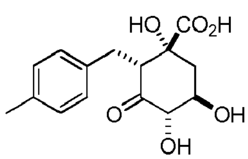 | **(2*R*)-Ia-4** | 0.089 | 0.25 | 0.022 |
| 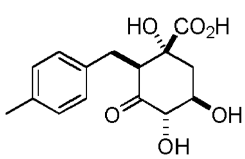 | **(2*S*)-Ia-4** | 0.09 | 0.97 | 0.15 |

(continued)

| Compound | | M. tuberculosis | H. pylori | S. coelicolor |
|---|---|---|---|---|
| 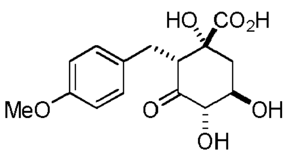 | **(2*R*)-Ia-5** | 0.025 | 0.17 | 0.034 |
| 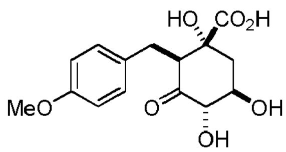 | **(2*S*)-Ia-5** | 0.10 | 1.40 | 0.34 |
| 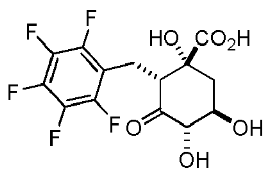 | **(2*R*)-Ia-6** | 0.047 | 2.6 | 0.242 |
| 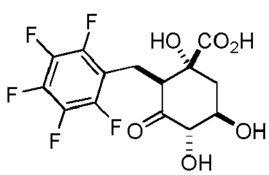 | **(2*S*)-Ia-6** | 0.074 | 0.97 | 0.0065 |
| 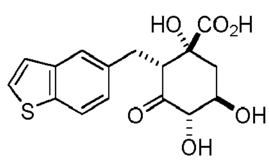 | **(2*R*)-Ia-7** | 0.029 | 0.16 | 0.004 |
| 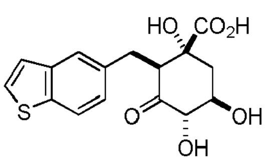 | **(2*S*)-Ia-7** | 0.055 | 0.90 | 0.038 |
| 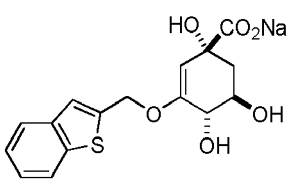 | **Ib-1** | 0.0032 | 0.16 | 0.00048 |

(continued)

| Compound | | *M. tuberculosis* | *H. pylori* | *S. coelicolor* |
|---|---|---|---|---|
| 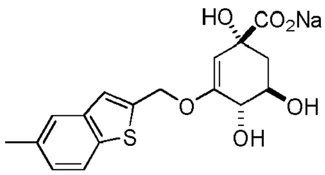 | **Ib-2** | 0.00425 | 0.14 | 0.0028 |
| 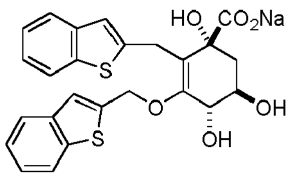 | **Ib-3** | 0.040 | 0.097 | 0.0024 |
| 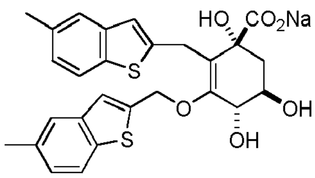 | **Ib-4** | 0.19 | 0.07 | 0.013 |
| 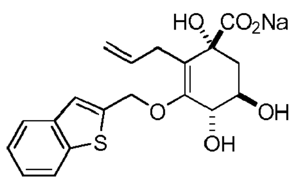 | **Ib-5** | 1.24 | 1.10 | 0.015 |
| 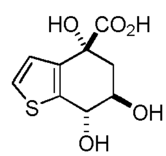 | **Ic-1** | 3.0 | 34 | 9.7 |
| 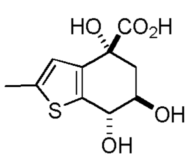 | **Ic-2** | 0.40 | 1.60 | 3.15 |
| 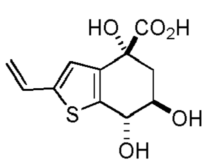 | **Ic-3** | 0.052 | 1.25 | 0.81 |

(continued)

| Compound | | *M. tuberculosis* | *H. pylori* | *S. coelicolor* |
|---|---|---|---|---|
| 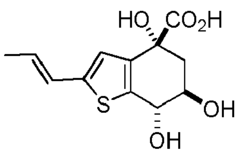 | **Ic-4** | 0.097 | 0.35 | 0.093 |
| 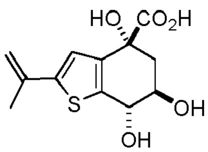 | **Ic-5** | 0.86 | 38 | 1.88 |
| 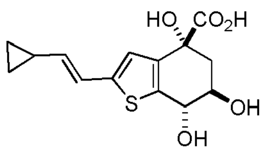 | **Ic-6** | 0.11 | 1.08 | 0.0003 |
| 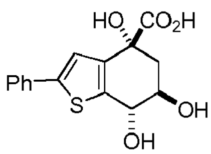 | **Ic-7** | 0.097 | >150 | 0.085 |
| 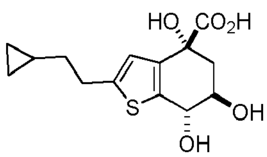 | **Ic-8** | 0.090 | 0.60 | 0.00085 |
| 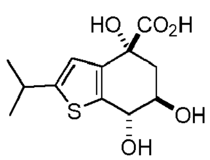 | **Ic-9** | 0.185 | 3.4 | 0.80 |

[a] Assay conditions: pH = 7.0, 25 °C, 50 mM Tris.HCl (*H. pylori* and *S. coelicolor*) or 50 mM Tris.HOAc (*M. tuberculosis*).

As shown in Table 1, the present invention provides inhibitors with inhibition constants as low as 3.2 nanomolar **(Ib-1)** or 25 nanomolar **((2R)-Ia-5)** against *Mycobacterium tuberculosis*, the bacteria which causes tuberculosis. This represents 1600 times more affinity with respect of the natural substrate ($K_m$ = 40 μM), and 8000 times more effective than 2,3-dehydroquinic acid ($K_i$= 200 μM). In the case of *H. pylori* affinities are as good as 70 nM **(Ib-4).** This represents about 6300 times more affinity with respect of the natural substrate ($K_m$ = 444 μM), and 4850 times more effective than the 2,3-dehydroquinic acid ($K_i$= 340 μM). In the case of *S. coelicolor* affinities are as good as 0.3 nanomolar **(Ic-6)** or 0.85 nanomolar **(Ic-8).**

## Claims

**1.** A compound of formula **I,** its diastereoisomers, its enantiomers or its pharmaceutically acceptable salts or solvates

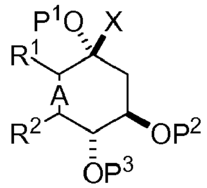

**I**

wherein,

A represents a single or double bond;
X is selected from the group consisting of -(C=O)OR$^a$ and -(C=O)NR$^b$R$^c$, wherein each of R$^a$, R$^b$ and R$^c$ is independently selected from the group consisting of hydrogen, substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl; or R$^b$ and R$^c$ together form a 5 or 6 membered heterocyclyc ring together with the nitrogen atom to which they are attached;
each P$^1$, P$^2$ and P$^3$ is independently selected from the group consisting of hydrogen, substituted or unsubstituted alkyl, substituted or unsubstituted alkenyl, substituted or unsubstituted alkynyl, substituted or unsubstituted silyl, substituted or unsubstituted arylalkyl and -(C=O)R$^a$, wherein R$^a$ is as defined above; and wherein
if A is a double bond,
then R$^2$ is selected from the group consisting of -OR$^a$, -SR$^a$ and - NR$^b$R$^c$, wherein R$^a$, R$^b$ and R$^c$ are as defined above; and R$^1$ is hydrogen or R$^{1a}$, wherein R$^{1a}$ is selected from the group consisting of substituted or unsubstituted alkyl, substituted or unsubstituted alkenyl, substituted or unsubstituted alkynyl, substituted or unsubstituted aryl, substituted or unsubstituted heterocyclyl, substituted or unsubstituted arylalkyl and substituted or unsubstituted heterocyclylalkyl, or R$^1$ and R$^2$ together form a 5-membered ring; and
if A is a single bond,
then R$^2$ is =O, =S or =NR$^b$, wherein k$^b$ is as defined above; and R$^1$ is R$^{1a}$, wherein R$^{1a}$ is a defined above.

2. A compound of formula **Ic** according to claim 1, its diastereoisomers, its enantiomers or its pharmaceutically acceptable salts or solvates

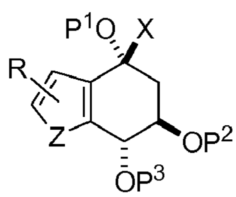

**Ic**

wherein,
X, P$^1$, P$^2$ and P$^3$ are as defined in claim 1;
Z is selected from the group consisting of O, S, NR$^b$ and $^{\oplus}$ NR$^b$R$^c$, wherein R$^b$ and R$^c$ are asdefined in claim 1; and
R is selected from the group consisting of a hydrogen, halogen, substituted or unsubstituted alkyl, substituted or unsubstituted alkenyl, substituted or unsubstituted alkynyl, substituted or unsubstituted aryl, substituted or unsubstituted heterocyclyl, substituted or unsubstituted aryalkyl and substituted or unsubstituted heterocyclylalkyl;
or
a compound of formula **Ia,** its diastereoisomers, its enantiomers or its pharmaceutically acceptable salts or solvates

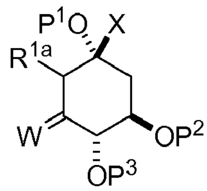

**Ia**

wherein X, $P^1$, $P^2$, $P^3$ and $R^{1a}$ are as defined in claim 1, and W is =O, =S or =$NR^b$, wherein $R^b$ is as defined in claim 1.

**3.** A compound of formula **Ib** according to claim 1, its diastereoisomers, its enantiomers or its pharmaceutically acceptable salts or solvates

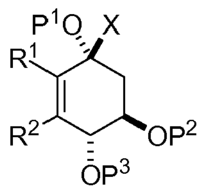

**Ib**

wherein,
X, $P^1$, $P^2$, $P^3$ and $R^1$ are as defined in claim 1; and
$R^2$ is selected from the group consisting of -$OR^a$, -$SR^a$ and -$NR^bR^c$, wherein $R^a$, $R^b$ and $R^c$ are as defined in claim 1.

**4.** A compound of formula I according to claim 1, selected from the group consisting of:

[1] (2*R*)-2-allyl-3-dehydroquinic acid,
[2] (2*S*)-2-allyl-3-dehydroquinic acid,
[3] (2*R*)-2-propyl-3-dehydroquinic acid,
[4] (2*R*)-2-benzyl-3-dehydroquinic acid,
[5] (2*S*)-2-benzyl-3-dehydroquinic acid,
[6] (2*R*)-2-(4-methyl)benzyl-3-dehydroquinic acid,
[7] (2*S*)-2-(4-methyl)benzyl-3-dehydroquinic acid,
[8] (2*R*)-2-(4-methoxy)benzyl-3-dehydroquinic acid,
[9] (2*S*)-2-(4-methoxy)benzyl-3-dehydroquinic acid,
[10] (2*R*)-2-perfluorobenzyl-3-dehydroquinic acid,
[11] (2*S*)-2-perfluorobenzyl-3-dehydroquinic acid,
[12] (2*R*)-2-(benzo[*b*]thiophen-5-yl)methyl-3-dehydroquinic acid,
[13] (2*S*)-2-(benzo[*b*]thiophen-5-yl)methyl-3-dehydroquinic acid,
[14] Sodium (1*R*, 4*S*, 5*R*)-3-(benzo[*b*]thiophen-2-yl)methoxy-1,4,5-trihydroxycyclohex-2-en-1-carboxylate,
[15] Sodium (1*R*, 4*S*, 5*R*)-3-(benzo[*b*]thiophen-2-yl)methoxy-2-(benzo[*b*]thiophen-2-yl)methyl-1,4,5-trihydroxycyclohex-2-en-1-carboxylate,
[16] Sodium (1*R*, 4*S*, S*R*)-1,4,5-trihydroxy-3-(5-methylbenzo[*b*]thiophen-2-yl)methoxycyclohex-2-en-1-carboxylate,
[17] Sodium (1*R*, 4*S*, 5*R*)-1,4,5-trihydroxy-3-(5-methylbenzo[*b*]thiophen-2-yl)methoxy-2-(5-methylbenzo[*b*]thiophen-2-yl)methylcyclohex-2-en-1-carboxylate,
[18] Sodium (1*R*, 4*S*, 5*R*)-1,4,5-trihydroxy-3-(5-methylbenzo[*b*]thiophen-2-yl)methoxy-2-(5-methylbenzo [*b*]thiophen-2-yl)methylcyclohex-2-en-1-carboxylate,
[19] Sodium (1*R*, 4*S*, 5*R*)-2-allyl-3-(benzo[*b*]thiophen-2-yl)methoxy-1,4,5-trihydroxycylohex-2-en-1-carboxylate,
[20] (4*R*, 6*R*, 7*S*)-4,6,7-trihydroxy-4,5,6,7-tetrahydrobenzo[*b*]thiophen-4-carboxylic acid,

[21] (4*R*, 6*R*, 7*S*)-4,6,7-trihydroxy-2-methyl-4,5,6,7-tetrahydrobenzo[*b*]thiophen-4-carboxylic acid,
[22] (4*R*, 6*R*, 7*S*)-4,6,7-trihydroxy-2-vinyl-4,5,6,7-tetrahydrobenzo[*b*]thiophen-4-carboxylic acid,
[23] (4*R*, 6*R*, 7*S*)-4,6,7-trihydroxy-2-[(*E*)-prop-1-enyl]-4,5,6,7-tetrahydrobenzo[b]thiophen-4-carboxylic acid,
[24] (4*R*, 6*R*, 7*S*)-4,6,7-trihydroxy-2-(1-methyl)vinyl-4,5,6,7-tetrahydrobenzo[b]thiophen-4-carboxylic acid,
[25] (4*R*, 6*R*, 7*S*)-2-[(*E*)-2-cyclopropyl]vinyl-4,6,7-trihydroxy-4,5,6,7-tetrahydrobenzo[b]thiophen-4-carboxylic acid,
[26] (4*R*, 6*R*, 7*S*)-4,6,7-trihydroxy-2-phenyl-4,5,6,7-tetrahydrobenzo[*b*]thiophen-4-carboxylic acid,
[27] (4*R*, 6*R*, 7*S*)-2-(2-cyclopropyl)ethyl-4,6,7-trihydroxy-4,5,6,7-tetrahydrobenzo[b]thiophen-4-carboxylic acid,
[28] (4*R*, 6*R*, 7*S*)-4,6,7-trihydroxy-2-isopropyl-4,5,6,7-tetrahydrobenzo[*b*]thiophen-4-carboxylic acid,
[29] (4*R*, 6*R*, 7*S*)-2-ethyl-4,6,7-trihydroxy-4,5,6,7-tetrahydrobenzo[*b*]thiophen-4-carboxylic acid,
[30] Ethyl (1*R*, 4*S*, 5*R*)-2-allyl-3-(benzo[*b*]thiophen-2-yl)methoxy-1,4,5-trihydroxycyclohex-2-en-1-carboxylate,

or its enantiomers or its pharmaceutically acceptable salts or solvates.

**5.** Process for the preparation of compounds of formula **Ia** as defined in claim 2, comprising the ring opening of a lactone of formula **III** in acidic medium,

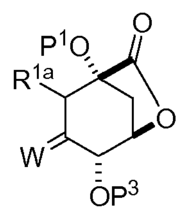

**III**

wherein, W, $P^1$, $P^3$ and $R^{1a}$ are as defined in claim 2.

**6.** Process for the preparation of a compound of formula **Ib** as defined in claim 3, comprising the ring opening of a lactone of formula **IV** in acidic or basic medium

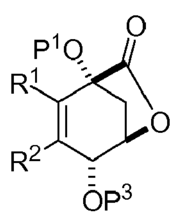

**IV**

wherein, $P^1$, $P^3$, $R^1$ and $R^2$ are as defined in claim 3.

**7.** Process according to claim 6, wherein the compound of formula **IV** is prepared by a process comprising an *O*-, *S*- or *N*-alkylation of a compound of formula **III** or of a compound of formula **II**, or a dialkylation of a compound of formula **II,** wherein $P^1$, $P^3$, $R^1$ and $R^2$ are as defined in claim 6, and W and $R^{1a}$ is as defined in claim 5

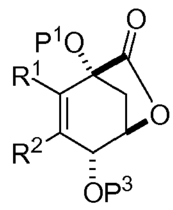

IV

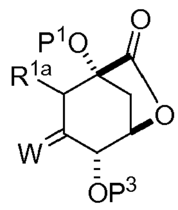

III

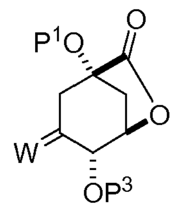

II.

8. Process for the preparation of a compound of formula **Ic,** as defined in claim 2, comprising the ring opening of a lactone of formula **V** in acidic or basic medium,

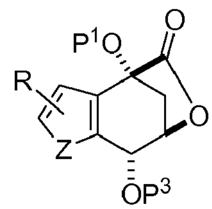

V

wherein, $P^1$, $P^3$, R and Z are as defined in claim 2.

9. A compound of formula **III,** its diastereoisomers, its enantiomers or its pharmaceutically acceptable salts or solvates

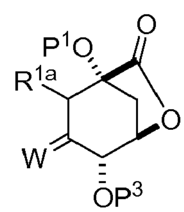

III

wherein W, $P^1$, $P^3$ and $R^{1a}$ are as defined in claim 5.

10. A compound of formula **IV,** its diastereoisomers, its enantiomers or its pharmaceutically acceptable salts or solvates

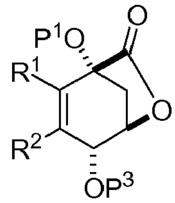

IV

wherein $P^1$, $P^3$, $R^1$ and $R^2$ are as defined in claim 6.

**11.** A compound of formula **V,** its diastereoisomers, its enantiomers or its pharmaceutically acceptable salts or solvates

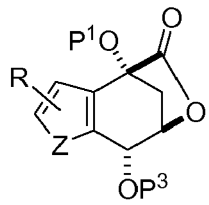

**V**

wherein $P^1$, $P^3$, R and Z are as defined in claim 8.

**12.** A pharmaceutical composition comprising a compound of formula **I** as defined in any of claims 1 to 4 and a pharmaceutically acceptable carier.

**13.** A compound of formula **I** as defined in anyone of claims 1 to 4, for use as a medicament.

**14.** A compound of formula **I** as defined in anyone of claims 1 to 4, for use as an antibiotic and/or antimicrobial.

**15.** Compound according to claim 14 for use in the treatment or prophylaxis of a disease selected from the group consisting of tuberculosis, stomach cancer, gastritis, stomach ulcers, and duodenal ulcers heartburn.

Europäisches Patentamt
European Patent Office
Office européen des brevets

# EUROPEAN SEARCH REPORT

Application Number
EP 08 38 2085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HASLAM, EDWIN ET AL: "Shikimate pathway. I. Preparation of stereospecifically labeled 2-deuterio derivatives of 3-dehydroquinic acid" JOURNAL OF THE CHEMICAL SOCIETY, SECTION C: ORGANIC, 1971, pages 1489-1495, XP008106187 ISSN: 0022-4952 * compound XVIII * ----- | 1,3 | INV. C07D333/54 C07D333/56 C07C62/26 C07C62/38 A61K31/381 A61K31/19 A61P31/00 |
| D,X | TOSCANO, MIGUEL D. ET AL: "Nanomolar inhibition of type II dehydroquinase based on the enolate reaction mechanism" CHEMMEDCHEM, vol. 2, no. 1, 2007, pages 101-112, XP002527333 ISSN: 1860-7179 * compounds 1, 3, 15, 16 * ----- | 1,3,10 | |
| X | BALTAS, MICHEL ET AL: "Addition of amines to methyl 3-dehydroquinate and 3-dehydroshikimate" BIOORGANIC & MEDICINAL CHEMISTRY LETTERS, vol. 3, no. 7, 1993, pages 1447-1452, XP002527334 ISSN: 0960-894X * compounds 1-3 * ----- | 1,3 | TECHNICAL FIELDS SEARCHED (IPC) C07D C07C A61P A61K |
| X | BARTLETT, PAUL A. ET AL: "Divergence between the enzyme-catalyzed and noncatalyzed synthesis of 3-dehydroquinate" JOURNAL OF ORGANIC CHEMISTRY, vol. 59, no. 8, 1994, pages 2082-2085, XP002527335 ISSN: 0022-3263 * compounds 15, 16 * ----- -/-- | 1,3 | |

The present search report has been drawn up for all claims

| Place of search | Date of completion of the search | Examiner |
|---|---|---|
| The Hague | 12 May 2009 | Duval, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 08 38 2085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | EP 1 647 544 A (UNIV SANTIAGO COMPOSTELA [ES]) 19 April 2006 (2006-04-19)<br>* claim 1 *<br>----- | 1 | |
| A | SANCHEZ-SIXTO C ET AL: "Structure-based design, synthesis, and biological evaluation of inhibitors of Mycobacterium tuberculosis type II dehydroquinase"<br>JOURNAL OF MEDICINAL CHEMISTRY 20050728 US,<br>vol. 48, no. 15,<br>28 July 2005 (2005-07-28), pages 4871-4881, XP002527352<br>ISSN: 0022-2623<br>* the whole document *<br>----- | 1 | |

TECHNICAL FIELDS SEARCHED (IPC)

The present search report has been drawn up for all claims

| Place of search | Date of completion of the search | Examiner |
|---|---|---|
| The Hague | 12 May 2009 | Duval, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 08 38 2085

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2009

| Patent document cited in search report | Publication date | Patent family member(s) | Publication date |
|---|---|---|---|
| EP 1647544 A | 19-04-2006 | ES 2223284 A1 | 16-02-2005 |
| | | WO 2005009330 A2 | 03-02-2005 |
| | | US 2007185214 A1 | 09-08-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005009330 A


### Non-patent literature cited in the description


- **Haslam, E.** The shikimate pathway. Wiley, 1974
- Enzymology and molecular biology of the shikimate pathway. **Abell, C.** Comprehensive Natural Products Chemistry. Pergamon, Elsevier Science Ltd, 1998, 573
- **Roberts, F.** *Nature,* 1998, vol. 393, 801
- **Roberts, C. W.** *J. Infect. Dis.,* 2002, vol. 185 (1), 25
- **McConkey, G. A. ; Pinney, J. W. ; Westhead, D. R. ; Plueckhahn, K. ; Fitzpatrick, T. B. ; Macheroux, P. ; Kappes, B.** *Trends in Parasitology,* 2004, vol. 20, 60
- **Hawkins, A. R.** *Curr. Genet.,* 1987, vol. 11, 491
- **Kleanthous, C. ; Davis, K. ; Kelly, S. M. ; Cooper, A. ; Harding, S. E. ; Price, N. C. ; Hawkins, A. R. ; Coggins, J. R.** *Biochem. J.,* 1992, vol. 282, 687
- **Gourley, D. G. ; Coggins, J. R. ; Isaacs, N. W. ; Moore, J. D. ; Charles, I. G. ; Hawkins, A. R.** *J. Mol. Biol.,* 1994, vol. 241, 488
- **Krell, T. ; Pitt, A. R. ; Coggins, J. R.** *FEBS Lett.,* 1995, vol. 360, 93
- **González-Bello, C. et al.** *Org. Biomol. Chem.,* 2003, vol. 1, 2075-2083
- **González-Bello, C. et al.** *Medicinal Research Reviews,* 2007, vol. 27 (2), 177-208
- **González-Bello, C. et al.** *ChemMedChem,* 2008, vol. 3, 756-770
- **González-Bello, C. et al.** *ChemMedChem,* 2007, vol. 2, 194-207
- **Greene, T. W. ; Wuts, P. G. M.** Protective Groups in Organic Synthesis. Wiley-Interscience, 1999
- **March, J.** Advanced Organic Chemistry; Reactions Mechanism and Structure. Wiley-Interscience, 464-473
- **Sánchez-Sixto, C. ; Prazeres, V. F. V. ; Castedo, L. ; Lamb, H. ; Hawkins, A. R. ; González-Bello, C.** *J. Med. Chem.,* 2005, vol. 48, 4871
- **Hanessian, S. ; Pan, J. ; Carnell, A. ; Bouchard, H. ; Lesage, L.** Total Synthesis of (-)-Reserpine Using the Chiron Approach. *J. Org. Chem.,* 1997, vol. 62, 465
- **March, J.** Advanced Organic Chemistry; Reactions Mechanism and Structure. Wiley-Interscience, 893-895
- **March, J.** Advanced Organic Chemistry; Reactions Mechanism and Structure. Wiley-Interscience, 896-898
- **Kraus, G. A. ; Andersh, B. ; Su, Q. ; Shi, J.** Bridgehead radicals in organic chemistry. An efficient construction of the ABDE ring system of the lycoctonine alkaloids. *Tetrahedron Letters,* 1993, vol. 34 (11), 1741-4
- **Murray, L. M. ; O'Brien, P. ; Taylor, R. J. K.** Stereoselective Reactions of a (-)-Quinic Acid-Derived Enone: Application to the Synthesis of the Core of Scyphostatin. *Organic Letters,* 2003, vol. 5 (11), 1943-1946
- **March, J.** Advanced Organic Chemistry; Reactions Mechanism and Structure. Wiley-Interscience, 750, 771-780
- **Carey, F. A. ; Sundberg, R. J.** Advanced Organic Chemistry. Part B: Reaction and Synthesis. 1-41
- **March, J.** Advanced Organic Chemistry; Reactions Mechanism and Structure. Wiley-Interscience, 365-368464-465
- Five-membered Rings with One Heteroatom and Fused Carbocyclic Derivatives. COMPREHENSIVE HETEROCYCLIC CHEMISTRY II, A review of the literature 1982-1995, The Structure, Reactions, Synthesis, and Uses of Heterocyclic Compounds. PERGAMON, 1982, vol. 2
- **R.J. Sundberg.** Pyrroles and their Benzo Derivatives: Synthesis. University of Virginia, 119-206
- **W. Friedrichsen.** Furans and their Benzo Derivates: Synthesis. Universität Kiel, 351-394
- **J. Nakayama.** Thiophenes and their Benzo Derivates: Synthesis. Saitama University, 607-678

- **Armin de Meijere ; François Diederich.** Metal-catalyzed cross-coupling reactions. Wiley-VCH, 1-3141-109
- **Armin de Meijere ; François Diederich.** Metal-catalyzed cross-coupling reactions. Wiley-VCH, 1-31217-296
- **Armin de Meijere ; François Diederich.** Metal-catalyzed cross-coupling reactions. Wiley-VCH, 1-31125-155
- **Armin de Meijere ; François Diederich.** Metal-catalyzed cross-coupling reactions. Wiley-VCH, 1-31317-386
- **Armin de Meijere ; François Diederich.** Metal-catalyzed cross-coupling reactions. Wiley-VCH, 1-31815-882
- **Prazeres, V. F. V. ; Sánchez-Sixto, C. ; Castedo, L. ; Lamb, H. ; Hawkins, A. R. ; Riboldi-Tunnicliffe ; Coggins, J. R. ; Lapthorn, A. J. ; González-Bello, C.** *ChemMedChem,* 2007, vol. 2, 194
- **Sánchez-Sixto, C. ; Prazeres, V. F. V. ; Castedo, L. ; S. W. Suh ; Lamb, H. ; Hawkins, A. R. ; Cañada, F. J. ; Jiménez-Barbero, J. ; González-Bello, C.** *ChemMedChem,* 2008, vol. 3, 756